(19) Europäisches Patentamt
European Patent Office
Office européen des brevets

(11) EP 1 268 469 B1

(12) FASCICULE DE BREVET EUROPEEN

(45) Date de publication et mention de la délivrance du brevet:
**16.06.2004 Bulletin 2004/25**

(21) Numéro de dépôt: **01919537.9**

(22) Date de dépôt: **22.03.2001**

(51) Int Cl.7: **C07D 401/14**, C07D 401/12, C07D 213/73, A61K 31/506, A61P 7/02, C07D 409/14, C07D 401/06, C07D 409/12

(86) Numéro de dépôt international:
**PCT/FR2001/000861**

(87) Numéro de publication internationale:
**WO 2001/070736 (27.09.2001 Gazette 2001/39)**

(54) **N-(HETEROCYCLYL)BENZENE-OU PYRIDINE SULFONAMIDES COMME AGENTS ANTITHROMBOTIQUES ET ANTICOAGULANTS**

N-HETEROCYCLYL-BENZEN ODER -PYRIDIN-SULFONAMIDE ZUR VERWENDUNG ALS ANTI-THROMBOGENE UND ANTIKOAGULIERENDE VERBINDUNGEN

N-(HETEROCYCLYL)BENZENE OR PYRIDINE SULPHONAMIDES AS ANTITHROMBOTIC AGENTS AND ANTICOAGULANTS

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **23.03.2000 FR 0003724**

(43) Date de publication de la demande:
**02.01.2003 Bulletin 2003/01**

(73) Titulaire: **SANOFI-SYNTHELABO**
**75013 Paris (FR)**

(72) Inventeurs:
- **ALTENBURGER, Jean-Michel**
  **78470 Saint Rémy-les-Chevreuse (FR)**
- **CREMER, Gérard**
  **91420 Morangis (FR)**
- **LASSALLE, Gilbert**
  **91470 Les Molières (FR)**
- **MATROUGUI, Mostafa**
  **91120 Palaiseau (FR)**

(74) Mandataire: **Thouret-Lemaitre, Elisabeth et al**
**Sanofi-Synthélabo,**
**Département Brevets,**
**174, avenue de France**
**75013 Paris (FR)**

(56) Documents cités:
**FR-A- 2 756 220**
**FR-A- 2 756 285**
**FR-A- 2 771 094**

EP 1 268 469 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet des dérivés N-(hétérocyclyl)benzène- ou pyridinesulfonamide, leur préparation et leur application en thérapeutique.

Les composés de la présente invention répondent à la formule [I] :

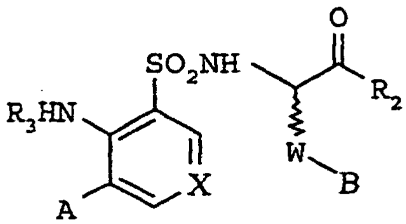

(I)

dans laquelle :

X représente soit un groupe $=CR_4-$, soit un atome d'azote,

W représente un groupe $-(CH_2)_2-$, $-(CH_2)_3-$, $-CH_2-C\equiv C-$ (liaison triple) ou $-CH_2-CH=CH-$ (liaison double en configuration cis ou trans),

$R_2$ représente

- soit un groupe pipéridinyle éventuellement substitué :
    - par un ou deux groupes choisis parmi les groupes hydroxy, $(C_1-C_4)$alkyle, hydroxy$(C_1-C_4)$alkyle, $(C_1-C_4)$alcoxy$(C_1-C_4)$alkyle, $(C_1-C_4)$alcoxy, $(C_1-C_4)$alkylthio, monofluorométhyle, difluorométhyle, trifluorométhyle, $(C_3-C_6)$cycloalkyle,
    - par un groupe =CYZ [Y et Z étant choisis indépendamment l'un de l'autre parmi les atomes d'hydrogène, d'halogène et les groupes $(C_1-C_4)$alkyle (éventuellement substitués par 1 à 3 atomes d'halogène)],
    - par un groupe :

      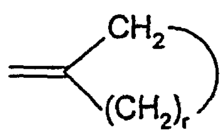

      (r = 1 à 3), ou
    - par un groupe spiro[$(C_3-C_6)$cycloalkane],
- soit un groupe 1,2,3,6-tétrahydropyridinyle éventuellement substitué par un groupe $(C_1-C_4)$alkyle (ce groupe $(C_1-C_4)$alkyle étant éventuellement substitué par 1 à 3 atomes d'halogène) ou un groupe $(C_3-C_6)$cycloalkyle,
- soit un groupe hexahydro-1*H*-azépinyle éventuellement substitué en position 4 par un groupe trifluorométhyle ou difluorométhylène,
- soit un groupe heptahydroazocin-1-yle,
- soit un groupe octahydro-1*H*-azonin-1-yle,
- soit un groupe

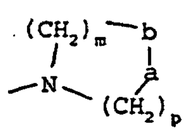

(a-b étant un groupe -CONR'-, m = 1 à 2, p = 1 à 2 et R' est un atome d'hydrogène ou un groupe $(C_1-C_4)$alkyle),

- soit un groupe

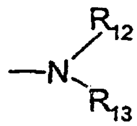

où
soit $R_{22}$ est un groupe $(C_1-C_4)$ alkyle, un groupe carboxy$(C_1-C_4)$alkyle ou un groupe $(C_1-C_4)$ alcoxycarbonyl $(C_1-C_4)$ alkyle et $R_{13}$ est un groupe $(C_1-C_4)$ alcoxy ou $(C_1-C_4)$ alkyle,
soit $R_{12}$ est un groupe $(C_1-C_4)$alkyle ou $-CH_2CF_3$ et $R_{13}$ est un groupe

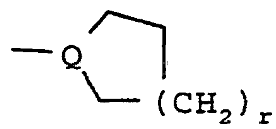

(Q étant un atome de carbone ou d'azote et r = 1 à 3),

- soit un groupe pipérazinyle éventuellement substitue par un groupe $(C_1-C_4)$ alkyle ou un groupe $(C_1-C_4)$ alkylsulfonyle,
- soit un groupe morpholinyle,

$R_4$ représente

- soit un atome d'halogène,
- soit un atome d'hydrogène,

$R_3$ représente

- soit un groupe $(C_1-C_5)$ alkyle,
- soit un groupe $-COR_1$, où $R_1$ est soit un atome d'hydrogène, soit un groupe $(C_1-C_4)$alkyle, $-(CH_2)_nOCH_3$, $-CH_2O(C_2H_4O)_nCH_3$, $-(CH_2)_nCF_3$ ou $-(CH_2)_nOH$ (n = 1 à 4),
- soit un groupe $-SO_2R_5$,
- soit un groupe $-CONHR_5$,
- soit un groupe $-SO_2N(R_5)_2$, où $R_5$ est un groupe $(C_1-C_4)$ alkyle,

A représente

- soit un groupe phényle éventuellement substitué par 1 à 3 substituants choisis parmi
  - un atome d'halogène et
  - les groupes $(C_1-C_4)$alkyle, $(C_1-C_4)$alcoxy, trifluorométhyle, trifluorométhoxy, $-CH_2OR_{10}$, $-CH_2OCOR_{10}$, $-CH_2OCONR_{10}R_{11}$, $-COOR_{10}$, $-CONR_{10}R_{11}$, nitro, $-NR_{10}R_{11}$, $-NHCOR_{10}$, et $-NH(CH_2)_qOR_{10}$, où $R_{10}$ et $R_{11}$ sont chacun indépendamment l'un de l'autre un atome d'hydrogène ou un groupe $(C_1-C_4)$alkyle et q est compris entre 0 et 6,
- soit un hétérocycle choisi parmi les groupes pyridyle, thiényle, furyle, pyrimidinyle et thiazolyle, les dits groupes pouvant être substitués comme le groupe phényle ci-dessus,
- soit un groupe $(C_5-C_8)$cycloalkyle et

B représente

- soit un groupe pyridyle éventuellement substitué par 1 ou 2 substituants choisis parmi un groupe $(C_1-C_4)$ alkyle, hydroxy ou $(C_1-C_4)$alcoxy,
- soit un groupe aminopyrazinyle,

- soit un groupe aminopyridazinyle,
- soit un groupe pyrimidinyle éventuellement substitué par un groupe amino,
- soit un groupe pipéridinyle,
- soit un groupe aminopyridinyle éventuellement substitué sur la pyridine par un groupe ($C_1$-$C_4$) alkyle, ($C_1$-$C_4$) alcoxy ou un atome d'halogène, le groupe amino pouvant éventuellement aussi être substitué par un groupe ($C_1$-$C_4$) alkyle,
- soit un groupe aminophényle, le groupe amino pouvant être éventuellement substitué par un groupe ($C_1$-$C_4$) alkyle et le groupe phényle pouvant être substitué par un groupe ($C_1$-$C_4$) alkyle ou un atome d'halogène.

Dans le cadre de l'invention, les termes ci-après ont les significations suivantes :

- un groupe ($C_1$-$C_4$)alkyle est une chaîne hydrocarbonée, saturée, linéaire ou ramifiée comportant de 1 à 4 atomes de carbone,
- un groupe ($C_x$-$C_y$)cycloalkyle est une chaîne hydrocarbonée cyclique comprenant x à y atomes de carbone,
- un groupe ($C_1$-$C_4$)alcoxy est un radical oxygène substitué par un groupe ($C_1$-$C_4$)alkyle précédemment défini,
- un atome d'halogène est un atome de chlore, de brome, d'iode ou de fluor.

Dans le cadre de l'invention, les atomes d'halogène sont préférentiellement le chlore, le fluor et le brome.

En fonction de la nature du groupe W, les composés de formule (I) conformes à l'invention peuvent être représentés par les formules ($I_1$) , ($I_2$) ($I_3$) et ($I_4$) ci-dessous :

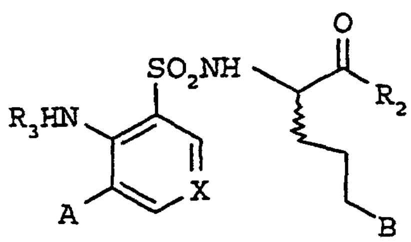

($I_1$)

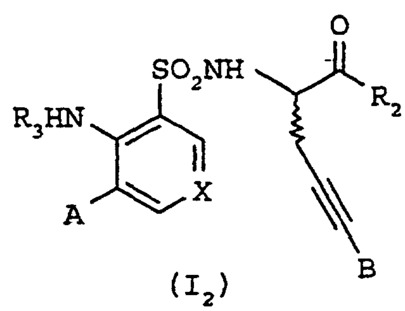

($I_2$)

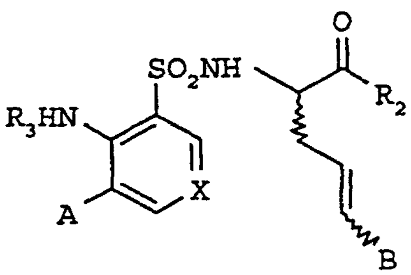

($I_3$)

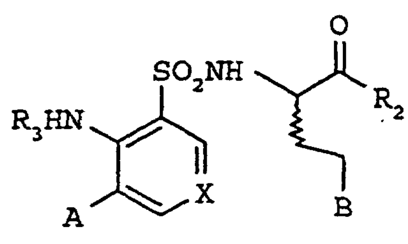

($I_4$)

Les composés préférés selon l'invention sont les composés de formule [I] dans laquelle :

X, W, $R_4$, A et B sont tels que défini précédemment,

$R_2$ représente

- soit un groupe pipéridinyle éventuellement substitué
  - par un ou deux groupes choisis parmi les groupes hydroxy, ($C_1$-$C_4$)alkyle, hydroxy($C_1$-$C_4$)alkyle, ($C_1$-$C_4$) alcoxy($C_1$-$C_4$)alkyle, ($C_1$-$C_4$)alcoxy, ($C_1$-$C_4$)alkylthio, monofluorométhyle, difluorométhyle, trifluorométhyle, ($C_3$-$C_6$)cycloalkyle,

- par un groupe =CYZ [Y et Z étant choisis indépendamment l'un de l'autre parmi les atomes d'hydrogène, d'halogène et les groupes $(C_1-C_4)$alkyle (éventuellement substitués par 1 à 3 atomes d'halogène)],

- soit un groupe 1,2,3,6-tétrahydropyridinyle éventuellement substitué par un groupe $(C_1-C_4)$alkyle (ce groupe $(C_1-C_4)$alkyle étant éventuellement substitué par 1 à 3 atomes d'halogène) ou un groupe $(C_3-C_6)$ cycloalkyle,
- soit un groupe hexahydro-1*H*-azépinyle éventuellement substitué en position 4 par un groupe trifluorométhyle ou difluorométhylène,
- soit un groupe

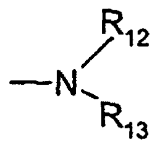

où $R_{12}$ est un groupe $(C_1-C_4)$ alkyle, un groupe carboxy $(C_1-C_4)$ alkyle ou un groupe $(C_1-C_4)$ alcoxycarbonyl $(C_1-C_4)$ alkyle et $R_{13}$ est un groupe $(C_1-C_4)$ alcoxy ou $(C_1-C_4)$ alkyle,
- soit un groupe pipérazinyle éventuellement substitué par un groupe $(C_1-C_4)$ alkyle ou un groupe $(C_1-C_4)$alkylsulfonyle,
- soit un groupe morpholinyle,

$R_3$ représente

- soit un groupe $(C_1-C_5)$ alkyle,
- soit un groupe $-COR_1$, où $R_1$ est soit un atome d'hydrogène, soit un groupe $(C_1-C_4)$alkyle, $-(CH_2)_nOCH_3$, $-CH_2O(C_2H_4O)_nCH_3$, $-(CH_2)_nCF_3$ ou $-(CH_2)_nOH$ (n = 1 à 4).

Parmi les composés préférés ci-dessus définis, on préfère particulièrement les composés de formule [I] dans laquelle :

X, $R_4$ et B sont tels que défini précédemment,

W représente un groupe $-(CH_2)_3-$ ou $-CH_2-CH=CH-$ (double liaison en configuration cis ou trans),

$R_2$ représente

- soit un groupe pipéridinyle éventuellement substitué

  - par un ou deux groupes choisis parmi les groupes hydroxy, $(C_1-C_4)$alkyle, hydroxy $(C_1-C_4)$alkyle, $(C_1-C_4)$ alcoxy $(C_1-C_4)$ alkyle, $(C_1-C_4)$ alcoxy, $(C_1-C_4)$ alkylthio, monofluorométhyle, difluorométhyle et trifluorométhyle,
  - par un groupe =CYZ [Y et Z étant choisis indépendamment l'un de l'autre parmi les atomes d'hydrogène, d'halogène et les groupes $(C_1-C_4)$ alkyle (éventuellement substitués par 1 à 3 atomes d'halogène)],

- soit un groupe 1,2,3,6-tétrahydropyridinyle éventuellement substitué par un groupe $(C_1-C_4)$ alkyle (ce groupe $(C_1-C_4)$alkyle étant éventuellement substitué par 1 à 3 atomes d'halogène),
- soit un groupe hexahydro-1*H*-azépinyle,
- soit un groupe pipérazinyle éventuellement substitué par un groupe $(C_1-C_4)$ alkylsulfonyle,
- soit un groupe morpholinyle,

$R_3$ représente un groupe $-COR_1$, où $R_1$ est un groupe $(C_1-C_4)$ alkyle, $-(CH_2)_nOCH_3$ ou $-(CH_2)_nCF_3$ (n = 1 à 4),

A représente

- soit un groupe phényle éventuellement substitué par 1 à 3 substituants choisis parmi

  - un atome d'halogène et

- les groupes ($C_1$-$C_4$)alkyle et ($C_1$-$C_4$) alcoxy,

• soit un hétérocycle choisi parmi les groupes pyridyle ou thiényle,
• soit un groupe ($C_5$-$C_8$)cycloalkyle,

La configuration préférentielle de la partie acide aminé centrale des composés conformes à la présente invention :

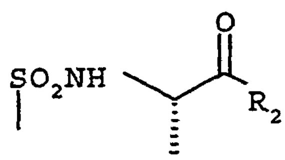

est [*S*].

Les composés de formule [I] conformes à l'invention peuvent exister sous forme de racémates ou d'énantiomères purs ou de mélanges d'énantiomères. Ils peuvent également exister sous forme d'acides ou de bases libres ou de sels d'addition à des acides pharmaceutiquement acceptables, par exemple sous forme de chlorhydrate ou de méthanesulfonate.

On peut en particulier citer les composés suivants, sous forme de racémates ou d'énantiomères purs ou de mélanges d'énantiomères, ou encore sous forme d'acides ou de bases libres, de chlorhydrate ou de tout autre sel pharmaceutiquement acceptable, qui font partie de l'invention :

- le *N*-[2-[[[(1*S*)-4-(5-amino-3-méthylpyridin-2-yl)-1-[[4-(difluorométhylène)pipéridin-1-yl]carbonyl]butyl]amino] sulfonyl]-6-thién-2-ylphényl]propanamide,
- le *N*-[2-[[[(1*S*)-4-(6-amino-4-éthylpyridin-3-yl)-1-[[4-(difluorométhylène)pipéridin-1-yl]carbonyl]butyl]amino] sulfonyl]-6-cyclopentylphényl]acétamide,
- le *N*- [3-[[[(1*S*)-4-(5-aminopyridin-2-yl)-1-[[4-(difluorométhylène)pipéridin-1-yl]carbonyl]butyl]amino] sulfonyl]-3'-fluoro[1,1'-biphényl]-2-yl]propanamide,
- le *N*-[3-[[[(1*S*)-4-(5-aminopyridin-2-yl)-1-[[4-(difluorométhylène)pipéridin-1-yl]carbonyl]butyl]amino] sulfonyl]-3'-fluoro[1,1'-biphényl]-2-yl]acétamide,
- le *N*-[2-[[[(1*S*)-4-(5-aminopyridin-2-yl)-1-[(4-éthylpipéridin-2-yl)carbonyl]butyl]amino]sulfonyl]-6-thién-2-ylphényl] propanamide,
- le *N*-[2-[[[(1*S*)-4-(6-aminopyridin-3-yl)-1-pipéridin-1-ylcarbonyl)butyl]amino]sulfonyl]-6-cyclopentylphényl] propanamide,
- le *N*-[2-[[[(1*S*)-4-(5-aminopyridin-2-yl)-1-[[4-(difluorométhylène)pipéridin-1-yl]carbonyl]butyl]amino] sulfonyl]-6-cyclopentylphényl]propanamide,
- le *N*-[3-[[[(1*S*)-4-(5-aminopyridin-2-yl)-1-[[4-(difluorométhylène)pipéridin-1-yl]carbonyl]butyl]amino] sulfonyl] [1,1'-biphényl]-2-yl]acétamide,
- le *N*-[2-[[[(1*S*)-4-(6-aminopyridin-3-yl)-1-[[4-(difluorométhylène)pipéridin-1-yl]carbonyl]butyl]amino] sulfonyl]-6-thién-2-ylphényl]acétamide,
- le *N*-[2-[[[(1*S*)-4-(6-amino-4-méthylpyridin-3-yl)-1-[[4-(difluorométhylène)pipéridin-1-yl]carbonyl]butyl]amino] sulfonyl]-6-thién-2-ylphényl]propanamide,
- le *N*-[2-[[[(1S)-4-(6-aminopyridin-3-yl)-1-[[4-(difluorométhylène)pipéridin-1-yl]carbonyl]butyl]amino] sulfonyl]-6-cyclopentylphényl]acétamide,
- le *N*-[2-[[[(1*S*)-4-(6-aminopyridin-3-yl)-1-[[4-(trifluorométhyl)-1,2,3,6-tétrahydropyridin-1-yl]carbonyl] butyl]amino] sulfonyl]-6-thién-2-ylphényl]propanamide,
- le *N*-[2-[[[(1*S*)-4-(6-aminopyridin-3-yl)-1-[[4-(difluorométhylène)pipéridin-1-yl]carbonyl]butyl]amino] sulfonyl]-6-cyclopentylphényl]propanamide,
- le *N*-[3-[[[(1*S*)-4-(5-aminopyridin-2-yl)-1-[[4-(difluorométhylène)pipéridin-1-yl]carbonyl]butyl]amino] sulfonyl][1,1'-biphényl]-2-yl]propanamide,
- le *N*-[2-[[[(1*S*)-4-(6-amino-4-méthylpyridin-3-yl)-1-[[4-difluorométhylène)pipéridin-1-yl]carbonyl]butyl]amino] sulfonyl]-6-cyclopentylphényl]propanamide,
- le *N*-[3-[[[(1*S*)-4-(6-aminopyridin-3-yl)-1-[[4-(difluorométhylène)pipéridin-1-yl]carbonyl]butyl]amino] sulfonyl]-3'-fluoro[1,1'-biphényl]-2-yl]propanamide,
- le *N*-[2-[[[(1*S*)-4-(5-aminopyridin-2-yl)-1-[(4-méthylpipéridin-1-yl)carbonyl]butyl]amino]sulfonyl]-6-thién-2-ylphényl]acétamide,

- le *N*-[2-[[[(1*S*)-4-(6-aminopyridin-3-yl)-1-[(4-éthylpipéridin-1-yl)carbonyl]butyl]amino]sulfonyl]-6-thién-2-ylphényl] propanamide,
- le *N*-[2-[[[(1*S*)-4-(6-aminopyridin-3-yl)-1-[(4-méthylpipéridin-1-yl)carbonyl]butyl]amino]sulfonyl]-6-cyclopentylphényl]acétamide,
- le *N*-[3-[[[(1*S*)-4-(5-aminopyridin-2-yl)-1-[(4-méthylpipéridin-1-yl)carbonyl]butyl]amino]sulfonyl]-3'-méthyl[1,1'-biphényl]-2-yl]acétamide,
- le *N*-[3-[[[(1*S*)-4-(6-amino-4-méthoxypyridin-3-yl-1-[[4-(difluorométhylène)pipéridin-1-yl]-carbonyl]butyl] amino] sulfonyl][1,1'-biphényl]-2-yl]propanamide,
- le *N*-[3-[[[(1*S*)-4-(5-aminopyridin-2-yl)-1-[(4-méthylpipéridin-1-yl)carbonyl]butyl]amino]sulfonyl]-3'-méthyl[1,1'-biphényl]-2-yl]propanamide,
- le *N*-[3-[[[(1*S*)-4-(5-aminopyridin-2-yl)-1-[(4-méthylpipéridin-1-yl)carbonyl]butyl]amino]sulfonyl]-3'-fluoro[1,1'-biphényl]-2-yl]acétamide,
- le *N*-[3-[[[(1*S*)-4-(5-aminopyridin-2-yl)-1-[(4-méthylpipéridin-1-yl)carbonyl]butyl]amino]sulfonyl]-3'-méthoxy[1,1'-biphényl]-2-yl]propanamide,
- le *N*-[(1*S*)-4-(5-amino-2-pyridinyl)-1-[[4-(difluorométhylène)-1-pipéridinyl]carbonyl]butyl]-2-(formylamino)-3'-méthyl-[1,1'-biphényl]-3-sulfonamide,
- le *N*-[3-[[[(1*S*,3*Z*)-4-(5-amino-2-pyridinyl)-1-[[4-(difluorométhylène)-1-pipéridinyl]carbonyl]-3-butènyl] amino] sulfonyl] [1,1-biphényl] -2-yl] -acétamide,
- le N-[3-[[[(1S,3Z)-4-(5-amino-2-pyridinyl)-1-[(4-méthyl-1-pipéridinyl) carbonyl] -3-butènyl] amino] sulfonyl] [1,1'-biphényl]-2-yl]-acétamide.

L'invention a également pour objet un médicament, caractérisé en ce qu'il contient au moins un composé de formule (I) telle que définie ci-avant.

L'invention a, en outre, pour objet une composition pharmaceutique, caractérisée en ce qu'elle contient au moins un composé de formule (I) telle que définie ci-avant, ainsi qu'au moins un excipient pharmaceutiquement acceptable.

Par référence au schéma 1, pour obtenir les composés de formule [I] conformes à la présente invention dans lesquels X représente un groupe $=CR_4-$, on fait réagir, dans une étape

Schéma 1

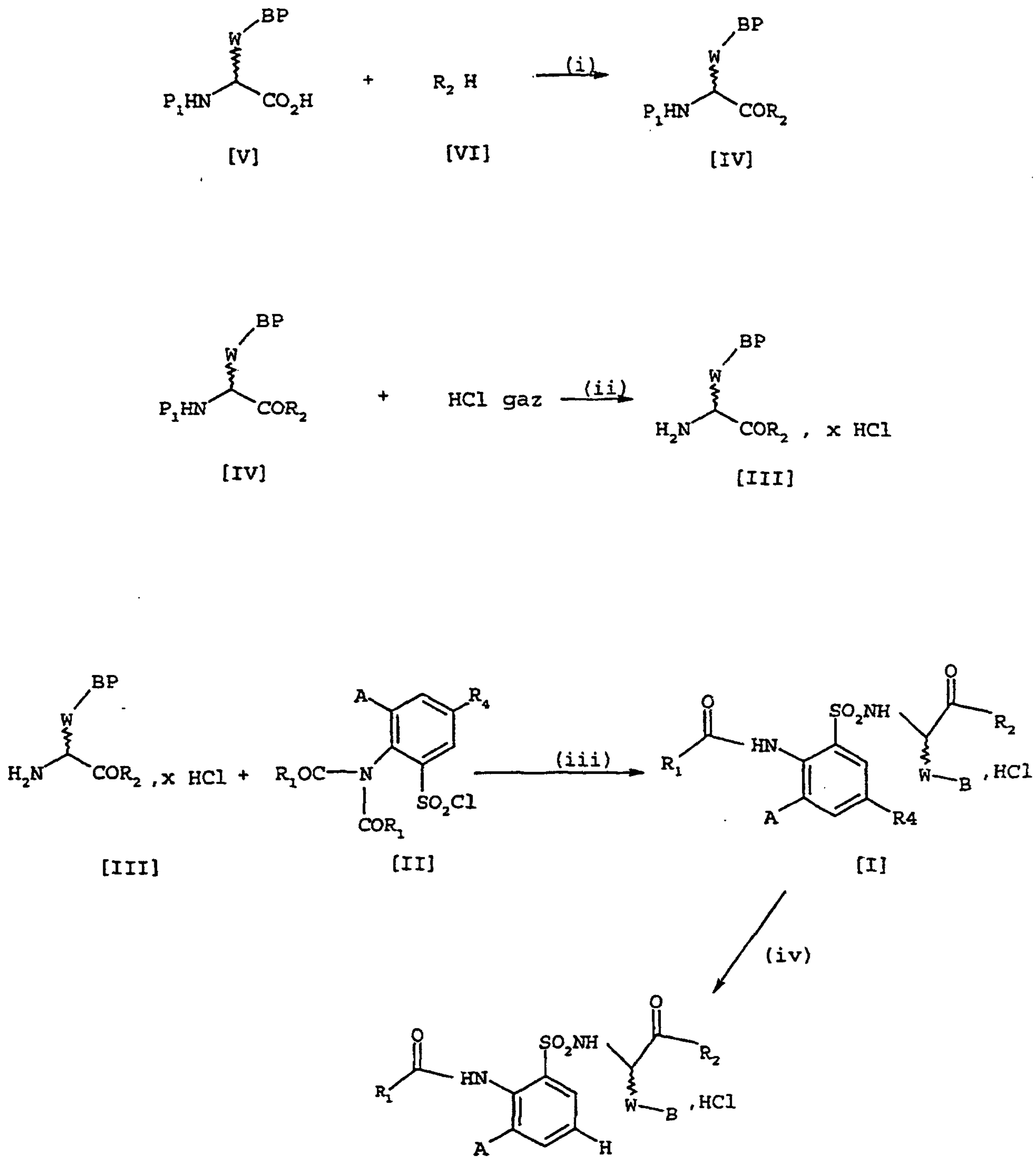


[V] [VI] [IV]

[IV] [III]

[III] [II] [I]

[Ib]

(ou [I] dans laquelle $R_4$ = -H)

(i) , un composé de formule [V] dans laquelle $P_1$ est un groupe protecteur d'une fonction amine, notamment un groupe tert-butoxycarbonyle (Boc), B et W sont tels que définis précédemment et P est soit un groupe protecteur tel que le phénylméthoxycarbonyle, soit un atome d'hydrogène, avec un composé de formule [VI], dans laquelle $R_2$ est tel que

défini précédemment. On obtient ainsi un composé de formule [IV], qui est traité au chlorure d'hydrogène dans une étape (ii) pour obtenir un composé de formule [III].

Dans une étape (iii), le composé de formule [III] est condensé, en présence de triéthylamine,avec un composé de formule [II] dans laquelle $R_1$, $R_4$ et A sont tels que définis précédemment, pour obtenir après traitement à l'ammoniac un composé de formule [I]. La modification du groupe $-NHCOR_1$ en un groupe $-NHR_3$ tel que défini précédemment en rapport avec la formule [I] est effectuée selon les techniques de chimie organique connues de l'homme du métier.

Si on désire obtenir un composé de formule [I] dans laquelle $R_4$ est un atome d'hydrogène, alors on réalise dans une étape (iv) une hydrogénolyse du composé [I] pour obtenir un composé de formule [Ib].

Selon un mode de réalisation préféré du procédé de préparation des composés de formule (I) de la présente invention,

- l'étape (i) mentionnée ci-avant peut être réalisée en présence de *N,N*-diisopropyléthylamine (DIEA) dans du dichlorométhane ou dans du diméthylformamide en ajoutant, sous azote, l'hexafluorophosphate de O-(benzotriazol-1-yl)-N,N,N',N'-tétraméthyluronium (HBTU),
- l'étape (ii) peut être effectuée dans du dichlorométhane en présence de chlorure d'hydrogène gazeux,
- l'étape (iii) peut être réalisée d'abord dans du dichlorométhane et de la triéthylamine (TEA), puis en reprenant le produit obtenu dans du tétrahydrofurane (THF), puis en faisant passer un courant d'ammoniac, suivi d'un traitement au chlorure d'hydrogène 0,1 N dans l'isopropanol, ou au bromure d'hydrogène dans l'acide acétique,
- l'étape (iv) peut être réalisée en reprenant le composé de formule [I] dans une solution 0,1 N de chlorure d'hydrogène et dans l'isopropanol.

Selon une variante du procédé conforme à la présente invention, on prépare également les composés de formule [I], dans lesquels X représente un groupe $=CR_4-$, conformément au schéma 2. Par référence au schéma 2, on peut préparer ces composés en faisant réagir, dans une étape (i), le composé de formule [III] tel qu'obtenu à l'étape (ii) du procédé de l'invention décrit ci-dessus (schéma 1) [avec P = un atome d'hydrogène] avec un composé de formule [IIa], dans laquelle $R_4$ est un atome d'halogène et $R_1$ est tel que défini précédemment. On obtient ainsi un composé de formule [Ia] que l'on condense avec un composé de formule [VII] dans laquelle A est tel que défini précédemment et $R_5$ est un groupe $(C_1-C_4)$alkyle, pour obtenir un composé de formule [I]. Si on désire obtenir un composé de formule [I] dans laquelle $R_4$ est un atome d'hydrogène, alors on réalise dans une étape (iii) une hydrogénolyse du composé [I] pour obtenir un composé de formule [Ib].

Selon un mode de réalisation préféré de cette variante du procédé de préparation des composés de formule [I] de la présente invention, dans laquelle X représente un groupe $=CR_4-$,

- l'étape (i) mentionnée ci-avant peut être réalisée d'abord dans du dichlorométhane, en présence de triéthylamine puis en reprenant ensuite le produit obtenu dans un courant d'ammoniac,

Schéma 2

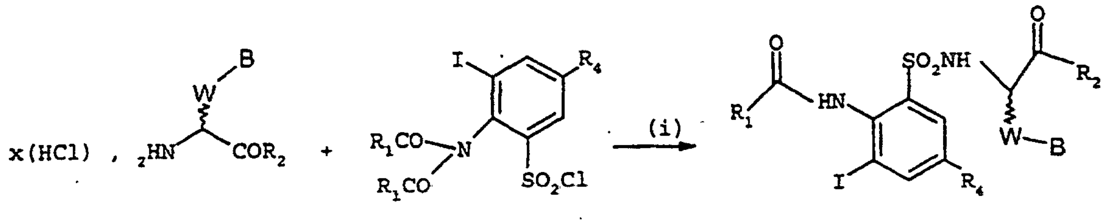


[III] avec P = -H [IIa] [Ia]

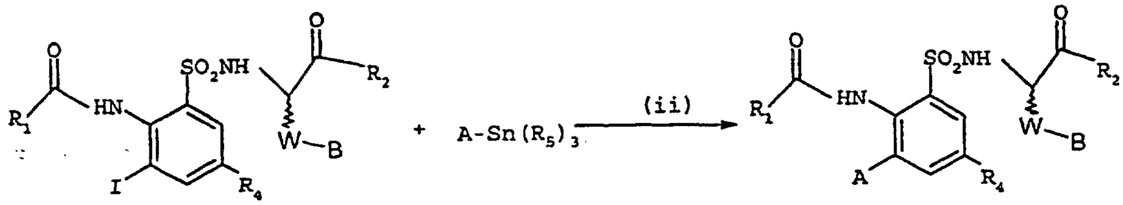


[Ia] [VII] [I]

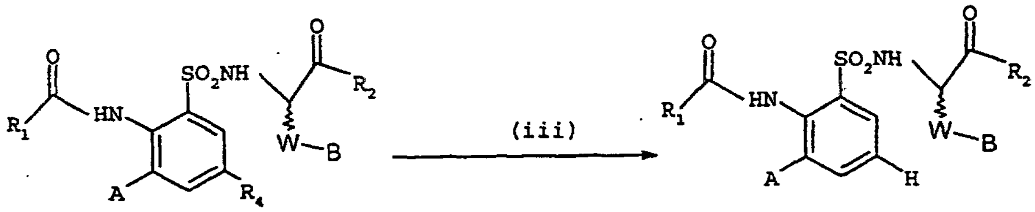


[I]

[Ib]
(ou [I] dans laquelle $R_4$ = -H)

Schéma 3

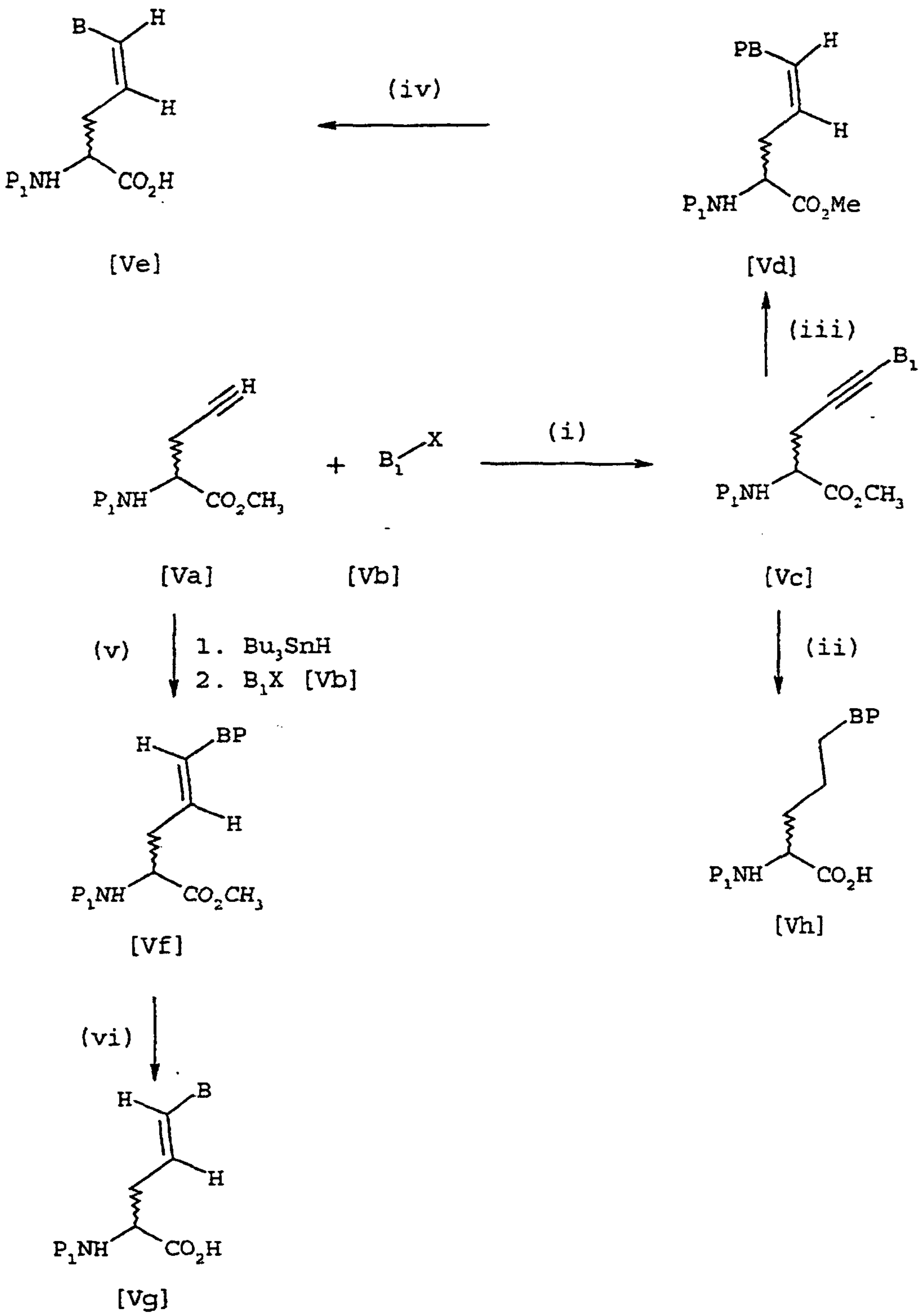


[Ve] [Vd] [Va] [Vb] [Vc] [Vf] [Vh] [Vg]

Schéma 4

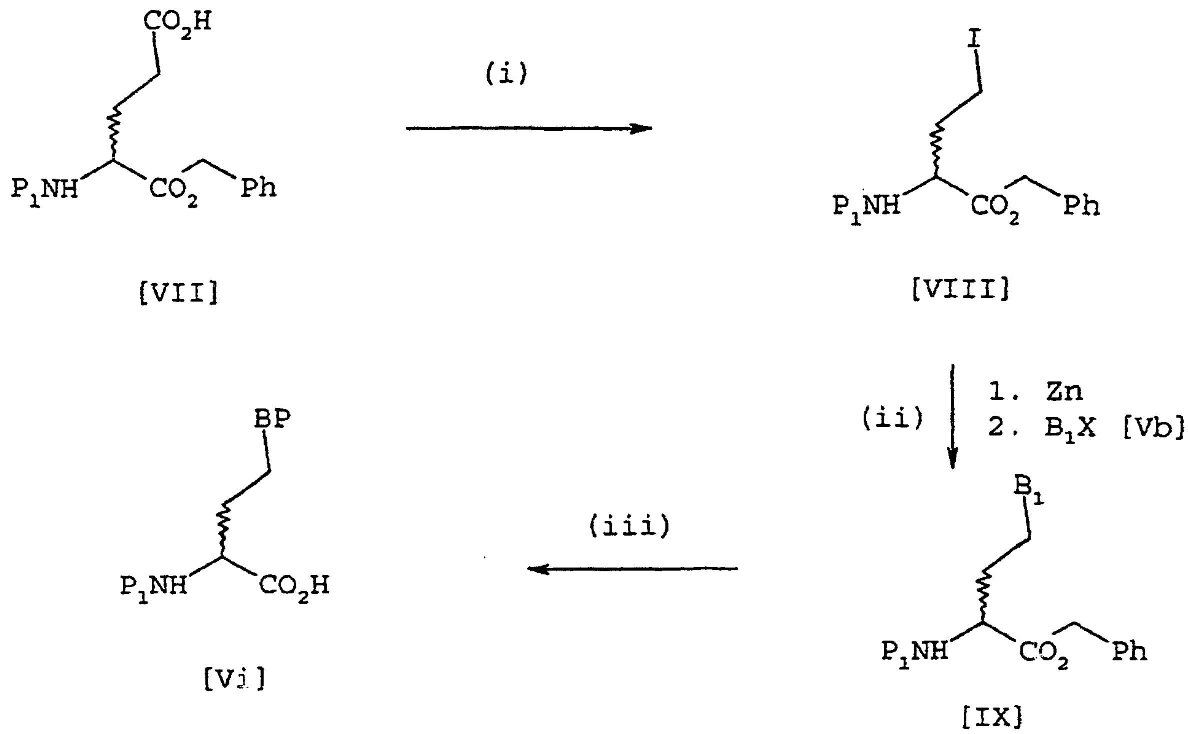


[VII]

[VIII]

[IX]

[Vi]

Schéma 5

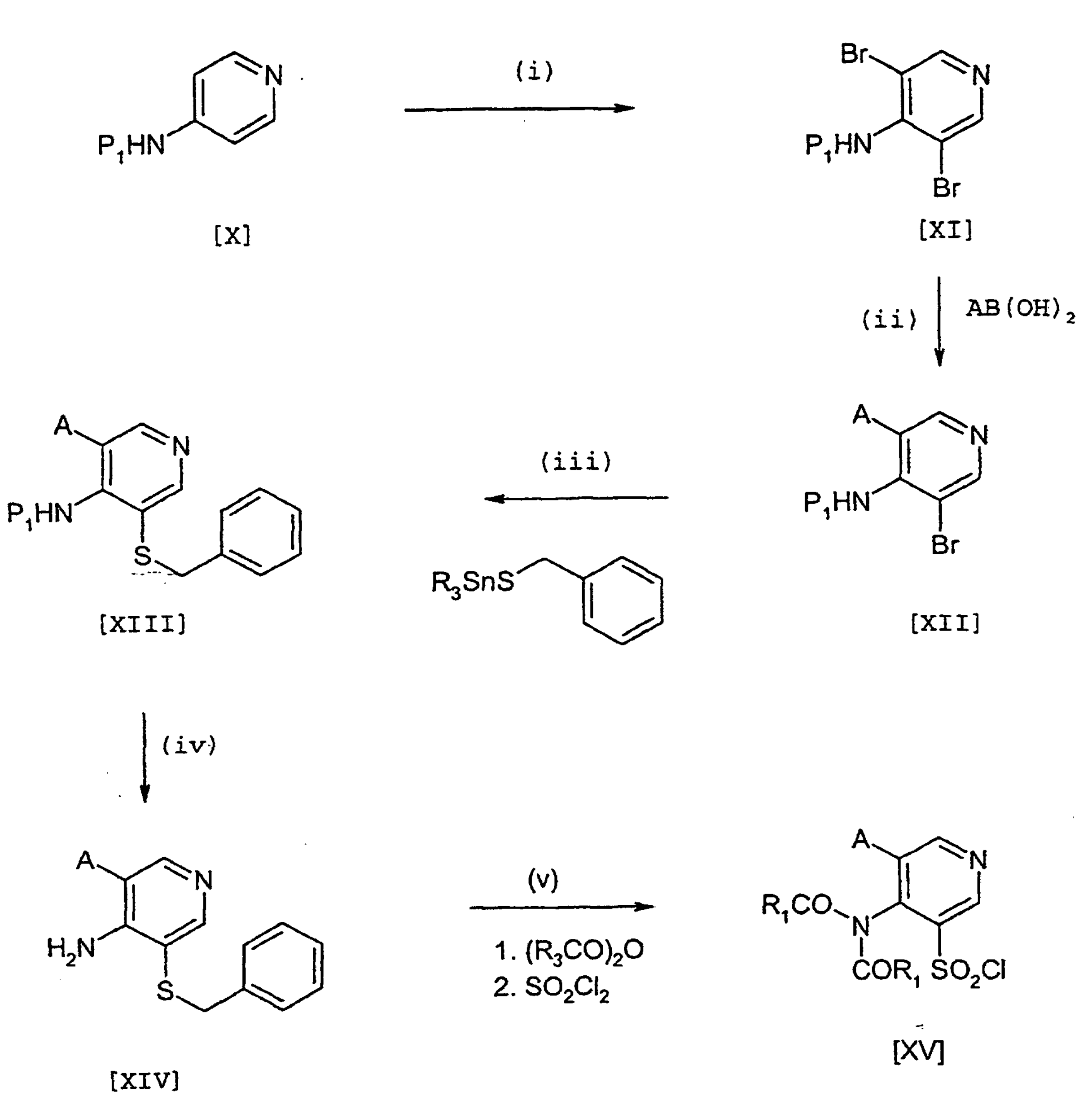


[X] [XI] [XII] [XIII] [XIV] [XV]

- l'étape (ii) peut être effectuée dans un mélange d'iodure de cuivre et de triphénylarsine ($Ph_3As$)dans du diméthylformamide (DMF) anhydre et en ajoutant du bisdibenzylidèneacétonepalladium (0) [Pd(dba)$_2$],
- l'étape (iii) peut être réalisée en présence de palladium sur charbon actif (Pd-c), et de formiate d'ammonium dans du méthanol,
- l'étape (iv) peut être réalisée en reprenant le composé de formule [I] dans une solution 0,1 N de chlorure d'hydrogène et dans l'isopropanol.

On prépare les composés de formule [V] de la présente invention, tel que représentés sur le schéma 1, selon les schémas 3 et 4.

Les schémas 3 et 4 illustrent la préparation de différents types de composés de formule [V], à savoir :

- le composé de formule [Ve], utile en tant qu'intermédiaire dans la préparation de composés de formule (I) dans laquelle W = $-CH_2-CH=CH-$ (la double liaison étant en configuration cis),
- le composé de formule [Vg], utile en tant qu'intermédiaire dans la préparation de composés de formule (I) dans

laquelle W = $-CH_2-CH=CH-$ (la double liaison étant en configuration trans),

- le composé de formule [Vh], utile en tant qu'intermédiaire dans la préparation de composés de formule (I) dans laquelle W = $-(CH_2)_3-$,
- le composé de formule [Vc], utile en tant qu'intermédiaire dans la préparation de composés de formule (I) dans laquelle W = $-CH_2-C=C-$,
- le composé de formule [Vi], utile en tant qu'intermédiaire dans la préparation de composés de formule (I) dans laquelle W = $-(CH_2)_2-$.

Pour préparer le composé de formule [Vc], on effectue par exemple les étapes suivantes (schéma 3) :

dans une étape (i), et par analogie à la synthèse décrite dans la demande brevet WO 9740052, on fait réagir un composé de formule [Va] dans laquelle $P_1$ est tel que défini précédemment, avec un composé de formule [Vb] dans laquelle $B_1$ est une base aromatique porteuse soit d'une fonction amine primaire ou secondaire protégée ou non, soit d'un précurseur de fonction amine tel qu'un groupe nitro, et X représente un atome d'halogène, pour obtenir un composé de formule [Vc].

Pour convertir le composé de formule [Vc] en composé de formule [V] directement utilisable dans le procédé représenté sur le schéma 1, on effectue une saponification du groupement ester et, dans le cas où $B_1$ est porteur d'un précurseur de fonction amine, on transforme ce précurseur en groupe amine éventuellement protégé par un groupe P, au moyen des techniques de chimie organique connues de l'homme du métier.

Pour préparer le composé de formule [Vh], on effectue par exemple les étapes suivantes (schéma 3) :

dans une étape (ii), on fait subir au composé de formule [Vc] soit une hydrogénation totale, à la fois de la triple liaison et de l'hétérocycle azoté, suivie éventuellement d'une protection orthogonale classique de l'amine secondaire non aromatique éventuellement générée, par un groupe P tel que le phénylméthoxycarbonyle, soit une hydrogénation sélective de la triple liaison, puis une saponification, pour obtenir le composé de formule [Vh].

Pour préparer le composé de formule [Ve], on effectue par exemple les étapes suivantes (schéma 3) :

dans une étape (iii), on fait subir au composé [Vc] une hydrogénation ménagée de la triple liaison, suivie éventuellement d'une protection orthogonale classique de l'amine secondaire de l'amine portée par le groupe B par un groupe P tel que tertbutyloxycarbonyle (Boc). Dans une étape (iv), on réalise une saponification du groupement ester du composé [Vd] pour conduire au composé [Ve].

Pour préparer le composé de formule [Vg], on effectue par exemple les étapes suivantes (schéma 3) :

- dans une étape (v), on fait subir au composé [Va], tout d'abord une hydrostannylation de la triple liaison, suivie d'un couplage catalysé par un complexe de palladium avec un composé [Vb] dans laquelle $B_1$ est une base aromatique porteuse soit d'une fonction amine primaire ou secondaire protégée ou non, soit d'un précurseur de fonction amine tel qu'un groupe nitro, et X représente un atome d'halogène pour conduire aux composés de formule [Vf],
- dans une étape (vi), le groupement ester du composé [Vf] est hydrolysé pour conduire au composé de formule [Vg].

Selon un mode de réalisation préféré du procédé de préparation des composés de formule [V] de la présente invention, tel qu'illustré sur le schéma 3 :

- l'étape (i) mentionnée ci-avant peut être réalisée dans du diméthylformamide, en présence d'un catalyseur à base de palladium tel que le complexe dichlorobis(triphénylphosphine) palladium/iodure cuivreux, en milieu basique par exemple avec du bicarbonate de potassium et dans du diméthylformamide anhydre,
- l'étape (ii) mentionnée ci-avant peut-être réalisée avec de l'hydrogène moléculaire ou du formiate d'ammonium, dans du méthanol, en présence d'un catalyseur à base de palladium tel que le palladium sur charbon actif, et en effectuant la saponification avec de l'hydroxyde de lithium dans un mélange méthanol/eau,
- l'étape (iii) mentionnée ci-avant peut-être réalisée en présence de palladium sur sulfate de barium dans l'acétate d'éthyle. Dans le cas où B1 porte une fonction nitro, il est souhaitable de la réduire au préalable préférentiellement par du fer dans un mélange éthanol-acide acétique,
- l'étape (iv) conduisant aux composés [Ve] peut être effectuée avec de l'hydroxyde de lithium dans un mélange méthanol/eau,
- l'étape (v) peut être effectuée avec un composé [Va] portant un groupement protecteur P1 de type trityle pour améliorer la régiosélectivité de la réaction d'hydrostannylation. Celle-ci est effectuée avec l'hydrure de tributyl étain

dans le tétrahydrofurane en présence de tétrakis triphényl phosphine palladium (o). Le couplage avec le réactif électrophile [Vb] est effectué dans du dioxane anhydre en présence de tétrakis triphénylphosphine palladium (o),
- dans le cas où le groupe $P_1$ du composé de formule [Vf] est un trityle, l'étape (vi) consiste à transformer le groupement $P_1$ de type trityle en groupement tertbutyloxycarbonyle en présence de soude aqueuse à l'aide du bis-ditertiobutylcarbonate. Dans cette opération, l'ester méthylique est hydrolysé en acide carboxylique, conduisant ainsi au composé de formule [Vg].

Par référence au schéma 4, on peut préparer les composés intermédiaires de_formule [Vi], permettant de préparer les composés de formule [I] dans laquelle W est un groupe - $(CH_2)_2$-, de la façon suivante :

- dans une étape (i), un composé de formule [VII] dérivé de l'acide glutamique, dans lequel $P_1$ est tel que défini précédemment et Ph représente un groupe phényle, est soumis à une réaction de HUNSDIECKER pour conduire au composé de formule [VIII],
- dans une étape (ii), le composé de formule [VIII] est transformé en dérivé organozincique correspondant, qui est couplé *in situ,* par une catalyse au palladium, à un composé de formule [Vb] dans laquelle $B_1$ est tel que défini précédemment et X représente un atome d'halogène, pour conduire aux composés de formule [IX],
- dans une étape (iii), le composé de formule [IX] est hydrogéné sur catalyseur au palladium pour conduire au composé de formule [Vi] portant une fonction acide carboxylique libre.

Selon un mode de réalisation préféré de ce procédé de préparation des composés de formule [Vi] de la présente invention,

- l'étape (i) mentionnée ci-avant peut être réalisée dans du tétrachlorure de carbone sous argon, en présence de di(acétyloxy)iodobenzène et d'iode moléculaire. Cette étape est effectuée ainsi sous irradiation UV,
- l'étape (ii) mentionnée ci-avant peut être réalisée dans du diméthylformamide sous argon en présence de zinc en poudre activé par addition de chlorure de triméthylsilyle et de 1,2-dibromoéthane. Le dérivé organozincique ainsi préparé est ensuite traité par l'électrophile $B_1X$, par exemple en présence de tris(dibenzylidèneacétone)dipalladium et de tri-ortho-tolylphosphine à température ambiante,
- l'étape (iii) mentionnée ci-avant peut être réalisée dans un mélange méthanol / eau en présence de catalyseur au palladium sur charbon actif à 10 % et sous 50 psi d'hydrogène.

Par référence au schéma 5, pour obtenir les composés de formule [XV], utiles en tant qu'intermédiaires pour préparer les composés de formule (I) conformes à l'invention dans laquelle X = N, on effectue les étapes suivantes :

- dans une étape (i), un composé 4-amino-pyridine de formule [X] dont la fonction amine est protégée par un groupement protecteur P1 tel que défini précédemment est converti en dérivé de formule [XI] portant 2 atomes de brome prépositionnés pour introduire les groupements recherchés,
- dans une étape (ii), le composé de formule [XI] est couplé avec un dérivé d'acide boronique de formule A $B(OH)_2$ dans laquelle A est un noyau phényle ou un hétérocycle éventuellement substitué, en présence d'un catalyseur au palladium, pour conduire à un composé de formule [XII],
- dans une étape (iii), l'atome de soufre précurseur du groupement chlorure de sulfonyle est introduit par une réaction de couplage catalysée par le palladium entre le composé de formule [XII] et un dérivé organostannique préalablement préparé à partir du benzèneméthane thiol,
- dans une étape (iv), le groupement protecteur $P_1$ est éliminé par traitement acide dans des conditions classiques pour conduire au composé de formule [XIV],
- dans une étape (v), le composé de formule [XIV] est converti en imide mixte par traitement avec un anhydride, puis le groupement benzylthiol est directement oxydé en dérivé chlorosulfonylé de formule [XV] par le chlorure de sulfuryle en présence d'acide acétique et d'eau.

Selon un mode de réalisation préféré de ce procédé de préparation des composés de formule [XV] :

- l'étape (i) mentionnée ci-avant peut être réalisée dans l'acétonitrile avec du N-bromosuccimide,
- l'étape (ii) mentionnée ci-avant peut être réalisée dans un mélange dioxane / eau en présence de carbonate de sodium et de tétrakis triphénylphosphine palladium (o),
- l'étape (iii) mentionnée ci-avant peut être réalisée dans le dioxane anhydre en présence de tétrakis triphényl phosphine palladium (o) avec le tributyl[(phénylméthyl)thio] stanne préalablement préparé,
- l'étape (iv) mentionnée ci-avant peut être réalisée de manière classique dans le méthanol en présence d'un courant de chlorure d'hydrogène,

- l'étape (v) mentionnée ci-avant peut être réalisée par chauffage dans un anhydride pur tel que l'anhydride propionique. Après évaporation de l'excès de réactif, un traitement dans un mélange d'acide acétique et d'eau par du chlorure de sulfuryle conduit directement au composé chlorosulfonyle attendu de formule [XV].

Le composé de formule [XV] ainsi obtenu peut ensuite être utilisé pour préparer les composés de formule (I) conforme à l'invention dans laquelle X = N, en suivant le protocole décrit à l'étape (iii) du schéma 1, c'est-à-dire par condensation du composé de formule [XV] avec un composé de formule [III] tel que défini ci-dessus.

Les composés de départ, tel que le composé de formule [Vb], sont disponibles dans le commerce ou décrits dans la littérature, ou bien peuvent être préparés selon des méthodes qui y sont décrites ou qui sont connues de l'homme du métier.

Les exemples suivants illustrent la préparation de certains composés conformément à l'invention. Les microanalyses et les spectres IR et RMN confirment la structure des composés obtenus.

Les numéros des composés exemplifiés renvoient à ceux des tableaux donnés plus loin qui illustrent les structures chimiques et les propriétés physiques de quelques composés selon l'invention. Le rapport (x:y) représente le rapport (acide:base).

Exemple 1 (composé n°3) :

Chlorhydrate de (*S*)-*N*-[2-[[[4-(6-aminopyridin-3-yl)-1-[(4-éthylpipéridin-3-yl)carbonyl]butyl]amino]sulfonyl]-6-thién-2-ylphényl]propanamide.

1.1. *N*-(5-bromopyridin-2-yl)-2,2,2-trifluoroacétamide.

A une solution de 75,0 g (0,433 mole) de 5-bromo pyridin-2-amine et de 41,25 ml (0,519 mole) de pyridine dans 250 ml de dichlorométhane, on ajoute à 0°C, sous azote, goutte à goutte 68,0 ml (0,477 mole) d'une solution d'anhydride trifluoroacétique dans 250 ml de dichlorométhane. On laisse revenir lentement à température ambiante et on poursuit l'agitation pendant 18 heures. Le mélange réactionnel est dilué par 300 ml de dichlorométhane puis est lavé à l'eau (2 x 400 ml) puis dans une solution saturée de chlorure de sodium (2 x 200 ml), puis est séché sur sulfate de sodium et est concentré sous pression réduite. Le résidu obtenu est purifié par chromatographie sur une colonne de gel en éluant sous pression par un mélange dichlorométhane : pentane (1 : 1). On obtient, sous forme de solide blanc, 100 g de *N*-(5-bromopyridin-2-yl)-2,2,2-trifluoroacétamide. Rdt (%) = 86 ; F (°C) = 73.

1.2. (*S*)-2-[[(1,1-Diméthyléthoxy)carbonyl]amino]-5-[6-[(trifluoroacétyl)amino]pyridin-3-yl]pent-4-ynoate de méthyle.

A un mélange de 13,0 g (48,3 mmoles) de *N*-(5-bromopyridin-2-yl)-2,2,2-trifluoroacétamide de 16,0 g (70,5 mmoles) de (*S*)-2-[[(1,1-diméthyléthoxy)carbonyl]amino]pent-4-ynoate de méthyle, de 0,46 g (2,4 mmoles) d'iodure de cuivre et de 13,35 g (96,6 mmoles) de carbonate de potassium dans 25 ml de diméthylformamide anhydre (DMF) on ajoute à température ambiante, sous argon 1,7 g (2,4 mmoles) de dichlorobis-(triphénylphosphine)-palladium. Le mélange est chauffé pendant 5 heures à 65°C. On reprend le mélange par de l'éther (800 ml), puis on le lave à l'eau (2 x 600 ml), puis dans une solution saturée de chlorure de sodium (300 ml), et on le sèche sur sulfate de sodium. Le produit obtenu est filtré puis est concentré sous pression réduite. Le résidu obtenu est purifié par chromatographie sur une colonne de gel de silice en éluant sous pression par un gradient acétate d'éthyle : cyclohexane de 0 à 20% en acétate d'éthyle. On obtient ainsi, sous forme d'une huile, 10 g de (*S*)-2-[[(1,1-diméthyléthoxy) carbonyl] amino] -5- [-6-(trifluoroacétyl)amino]pyridin-3-yl]pent-4-ynoate de méthyle. Rdt (%)= 52

1.3. Acide (S)-6-amino-α-[[(1,1-diméthyléthoxy) carbonyl]amino]pyridine-3-pentanoïque.

Un mélange de 8,04 g de (*S*)-2-[[(1,1-diméthyléthoxy) carbonyl]amino]-5-[-6-(trifluoroacétyl)amino]pyridin-3-yl]pent-4-ynoate de méthyle (20 mmoles) et de palladium sur charbon actif à 10 % (0,8 g) dans du méthanol (80 ml) et de l'acide acétique (1,32 ml ; 22 mmoles) est agité pendant 7 heures sous 50 psi d'hydrogène à température ambiante. On filtre le mélange puis on le concentre sous pression réduite. Le résidu obtenu est repris par de l'acétate d'éthyle (400 ml), puis est lavé par une solution saturée d'hydrogénocarbonate de sodium (200 ml), une solution saturée de chlorure de sodium (200 ml), filtré et est séché sur sulfate de sodium puis est concentré sous pression réduite. A une solution du résidu ainsi obtenu dans du méthanol (50 ml) et de l'eau (15 ml), on ajoute à 0°C l'hydroxyde de lithium monohydraté (1,1 g, 26 mmoles). On laisse revenir à température ambiante et on poursuit l'agitation pendant 18 heures. Le mélange réactionnel est refroidi à 0°C, est neutralisé à l'aide d'une solution 1N d'acide chlorhydrique puis est concentré sous pression réduite. On obtient, sous forme d'une huile visqueuse, 7 g d'acide (*S*)-6-amino-α-[[(1,1-diméthyléthoxy)carbonyl]amino]pyridine-3-pentanoïque (chlorure de lithium), utilisé tel quel à l'étape suivante. Rdt (%) = 88

1.4. (*S*)-[4-(6-Aminopyridin-3-yl)-1-[(4-éthylpipéridin-3-yl)carbonyl]butyl]carbamate de 1,1-diméthyl éthyle.

A un mélange de 2,0 g d'acide (*S*)-6-amino-α-[[(1,1-diméthyléthoxy)carbonyl]amino]pyridine-3-pentanoïque (5,5 mmoles), de 1,14g (7,5 mmoles) de chlorhydrate de 4-éthylpipéridine, de *N,N*-diisopropyléthylamine (DIEA) (25 ml ; 14 mmoles) dans du dichlorométhane (30 ml) et 3 ml de diméthylformamide anhydre (DMF), on ajoute sous agitation par petites quantités à 0°C, sous azote, 2,1 g (5,5 mmoles) d'hexafluorophosphate de O-(benzotriazol-1-yl)-N, N,N',N' tétraméthyluronium (HBTU). On laisse revenir à température ambiante et on poursuit l'agitation pendant 18 heures. Le mélange réactionnel est repris par 250 ml d'acétate d'éthyle et est lavé par 50 ml d'une solution d'acide chlorhydrique 0,1 N, et par 50 ml d'une solution saturée d'hydrogénocarbonate de sodium et par 50 ml d'une solution saturée de chlorure de sodium. Le produit obtenu est ensuite séché sur sulfate de sodium, puis est filtré et concentré sous pression réduite. Le résidu obtenu est purifié par chromatographie sur colonne de gel de silice en éluant sous pression par de l'acétate d'éthyle. On obtient, sous forme d'une huile visqueuse, 1,48 g de (*S*)-[4-(6-aminopyridin-3-yl)-1-[(4-éthylpipéridin-3-yl)carbonyl]butyl]carbamate de 1,1-diméthyléthyle.
Rdt (%) = 74

1.5. Chlorhydrate de (*S*)-1-[2-amino-5-(6-aminopyridin-3-yl)-1-oxopentyl]-4-éthylpipéridine (2:1).

Une solution de 1,48 g (3,6 mmoles) de (*S*)-[4-(6-aminopyridin-3-yl)-1-[(4-éthylpipéridin-3-yl)carbonyl] butyl] carbamate de 1,1-diméthyléthyle dans 40 ml de dichlorométhane est traitée pendant 1 min à 0°C par un courant de chlorure d'hydrogène. Après une heure à 0°C, on laisse revenir à température ambiante le mélange réactionnel qui est concentré ensuite sous pression réduite. On obtient, sous forme de solide blanc, 1,4 g de chlorhydrate de (*S*)-1-[2-amino-5-(6-aminopyridin-3-yl)-1-oxopentyl]-4-éthylpipéridine (2:1), utilisé tel quel à l'étape suivante.
Rdt (%) = 100 ; F (°C) = 65.

1.6. Chlorhydrate de (*S*)-*N*-[2-[[[4-(6-aminopyridin-3-yl)]-1-(4-éthylpipéridin-3-yl)carbonyl]butyl]amino] sulfonyl]-6-thién-2-ylphényl]propanamide.

A une solution de 1,5 g (4 mmoles) de chlorhydrate de (*S*)-1-[2-amino-5-(6-aminopyridin-3-yl)-1-oxopentyl]-4-éthyl pipéridine (2:1) dans 20 ml de dichlorométhane, on ajoute à 0°C 1,84 ml (13,2 mmoles) de triéthylamine (TEA) puis, par petites quantités 1,54 g (4 mmoles) de chlorure de 2-[bis(1-oxopropyl) amino]-3-thién-2-ylbenzènesulfonyle. Après 4 h à 0°C, on ajoute 200 ml d'acétate d'éthyle, puis on lave avec 100 ml d'une solution saturée d'hydrogénocarbonate de sodium et 100 ml d'une solution saturée de chlorure de sodium. On sèche le produit obtenu sur sulfate de sodium et on le concentre sous pression réduite. Le résidu ainsi obtenu est repris dans 100 ml de tétrahydrofurane (THF) puis est refroidi à 0°C, et est traité pendant 5 minutes par un courant d'ammoniac. On laisse le mélange réactionnel revenir à température ambiante puis après 4 heures on le concentre sous pression réduite. Le résidu obtenu est repris par 50 ml d'une solution 0,1N de chlorure d'hydrogène dans l'isopropanol (5 mmoles), puis est concentré sous pression réduite et est purifié par chromatographie sur colonne RP 18 en éluant par un gradient acétonitrile:eau de 5:95 à 30:70. On obtient 2 g de chlorhydrate de (*S*)-*N*-[2-[[[4-(6-aminopyridin-3-yl)]-1-(4-éthylpipéridin-3-yl)carbonyl] butyl]amino]sulfonyl]-6-thién-2-ylphényl]propanamide.
Rdt (%) = 79 ; F (°C) = 144-148 ;
$[\alpha]_D^{20}$ (°) =+120 (c = 0,2 ; méthanol).

Exemple 2 (composé n°5) :

Chlorhydrate de (*S*)-*N*-[2-[[[4-(6-aminopyridin-3-yl)-1-[(4-éthylpipéridin-3-yl)carbonyl]butyl]amino]sulfonyl]-6-pyridin-2-ylphényl]propanamide (2:1).

2.1. (*S*)-*N*-[2-[[[4-(6-aminopyridin-3-yl)-1-[(4-éthylpipéridin-3-yl)carbonyl]butyl] amino]sulfonyl]-4-bromo-6-iodophényl] propanamide.

On utilise le même mode opératoire qu'à l'exemple 1.6., à l'exception du traitement par une solution *0,1N* de chlorure d'hydrogène dans l'isopropanol et la purification par chromatographie sur colonne de RP18. Ainsi, à partir de 1,4 g (3,6 mmoles) de chlorhydrate de (*S*)-1-[2-amino-5-(6-aminopyridin-3-yl)-1-oxopentyl]-4-éthylpipéridine (2:1) et de 1,88 g (3,6 mmoles) de chlorure de 2-[bis(1-oxopropyl) amino]-5-bromo-3-iodobenzènesulfonyle, on obtient, sous forme de poudre blanche, 1,9 g de (*S*)-*N*-[2-[[[4-(6-amino pyridin-3-yl)]-1-[(4-éthylpipéridin-3-yl)carbonyl]butyl]amino sulfonyl]-4-bromo-6-iodophényl] propanamide.
Rdt (%) = 81 ; F (°C) = 193.

2.2. (*S*)-*N*-[2-[[[4-(6-Aminopyridin-3-yl)-1-[(4-éthyl pipéridin-3-yl)carbonyl]butyl]amino]sulfonyl]-4-bromo-6-pyridin-2-ylphényl]propanamide.

A un mélange de 1, 8 g (2,76 mmoles) de (*S*)-*N*-[2-[[[4-(6-amino pyridin-3-yl)]-1-[(4-éthylpipéridin-3-yl)carbonyl]butyl] amino] sulfonyl] -4-bromo-6-iodophényl] propanamide, de 1,22 g (3,32 mmoles) de 2-(tributylstannyl) pyridine, de 0,052 g d'iodure de cuivre (0,28 mmole) et de 0,17 g (0,56 mmole) de triphénylarsine dans 6 ml de diméthylformamide anhydre (DMF), on ajoute à température ambiante 0,08 g (0,14 mmole) de bis(dibenzylidèneacétone)palladium (0). On chauffe le mélange réactionnel à 80 °C pendant 7 heures puis on le reprend dans 200 ml d'acétate d'éthyle. Le mélange est ensuite lavé à deux reprises par 200 ml d'une solution d'ammoniac aqueuse à 10 %, puis par 100 ml d'eau et 100 ml d'une solution saturée de chlorure de sodium. On sèche sur sulfate de sodium et on filtre et concentre sous pression réduite le produit résultant. Le résidu ainsi obtenu est purifié par chromatographie sur une colonne de gel de silice en éluant sous pression par un mélange dichlorométhane : méthanol 94:6. On obtient, sous forme d'huile, 0,54 g de (*S*)-*N*-[2-[[[4-(6-aminopyridin-3-yl) -1-[(4-éthylpipéridin-3-yl)carbonyl]butyl]amino]sulfonyl]-4-bromo-6-pyridin-2-ylphényl]propanamide (2:1).
Rdt (%) = 30

2.3. Chlorhydrate de (*S*)-*N*-[2-[[[4-(6-aminopyridin-3-yl)-1-[(4-éthylpipéridin-3-yl)carbonyl]butyl]amino] sulfonyl]-6-pyridin-2-ylphényl]propanamide (2:1).

On chauffe pendant 2 h à reflux un mélange de 0,2 g (0,3 mmole) de (*S*)-*N*-[2-[[[4-[(6-aminopyridin-3-yl)-1-[(4-éthylpipéridin-3-yl)carbonyl]butyl]amino]sulfonyl]-4-bromo-6-pyridin-2-ylphényl]propanamide, de 0,02 g de palladium sur charbon actif à 10 % et de 0,2 g de formiate d'ammonium (3,0 mmoles) dans 10 ml de méthanol. Le mélange réactionnel est filtré, puis est repris par 100 ml de dichlorométhane, est lavé par 50 ml d'une solution saturée d'hydrogénocarbonate de sodium et 50 ml d'une solution saturée de chlorure de sodium. On sèche sur sulfate de sodium puis on filtre et concentre le produit résultant. Le résidu ainsi obtenu est repris par 40 ml d'une solution 0,1 N de chlorure d'hydrogène dans l'isopropanol, puis est concentré et purifié par chromatographie sur une colonne RP18 en éluant par un gradient acétonitrile : eau de 5 : 95 à 30 : 70. On obtient 0,135 g chlorhydrate de (*S*)-*N*-[2-[[[4-(6-aminopyridin-3-yl)-1-[(4-éthylpipéridin-3-yl)carbonyl]butyl]amino]sulfonyl]-6-pyridin-2-ylphényl] propanamide (2:1).
Rdt (%) = 75 ; F (°C) = 145-150 ;
$[\alpha]_D^{20}$ (°) = +138 (c = 0,2 ; méthanol)

<u>Exemple 3</u> (composé n°10) :

Chlorhydrate de (*S*)-*N*-[2-[[[1-[(4-éthylpipéridin-3-yl) carbonyl]-4-pipéridin-4-ylbutyl]amino]sulfonyl]-6-thién-2-ylphényl] propanamide.

3.1. (*S*)-α-[[(1,1-diméthyléthoxy)carbonyl]amino]-1-[(phénylméthoxy)carbonyl]pipéridine-4-pentanoate de méthyle.

Un mélange de 2,8 g (10,0 mmoles) de (*S*)-2-[[(1,1-diméthyléthoxy)carbonyl]amino]-5-pyridin-4-ylpent-4-ynoate de méthyle et de 0,28 g de palladium sur charbon actif à 10% dans 20 ml d'éthanol est agité pendant 3 heures à température ambiante sous 60 psi d'hydrogène. On filtre le mélange réactionnel et on le concentre sous pression réduite. Le résidu obtenu est repris par 20 ml d'acide acétique est agité pendant 14 heures, en présence de 0,05 g d'oxyde de platine sous 60 psi d'hydrogène. Le mélange réactionnel est filtré, puis est concentré sous pression réduite. Le résidu obtenu est repris par 10 ml de tétrahydrofurane (THF)et 5 ml d'eau. On refroidit à 0°C, puis on ajoute une solution de 3,4 g (40,0 mmoles) d'hydrogénocarbonate de sodium dans 40 ml d'eau et on verse goutte à goutte 1,63 ml (12,0 mmoles) de chloro-formiate de benzyle. On laisse le mélange revenir à température ambiante et on poursuit la réaction pendant 4 heures. Le mélange réactionnel est repris par 200 ml d'acétate d'éthyle et est lavé à deux reprises par 100 ml d'une solution 1N d'acide chlorhydrique, puis avec 100 ml d'une solution saturée d'hydrogénocarbonate de sodium, et avec 100 ml d'une solution saturée de chlorure de sodium. On sèche sur sulfate de sodium. Le produit résultant est filtré et est concentré. Le résidu obtenu est purifié par chromatographie sur une colonne de gel de silice en éluant sous pression par un gradient cyclohexane : acétate d'éthyle de 95 : 5 à 80 : 20. On obtient, sous forme d'huile visqueuse, 3,55 g de (*S*)-α-[[(1,1-diméthyl éthoxy)carbonyl]amino]-1-[(phényl méthoxy)carbonyl] pipéridine-4-pentanoate de méthyle.
Rdt (%) = 79 ; F (°C) = 110.

3.2. Acide (*S*)-α-[[(1,1-diméthyléthoxy)carbonyl] amino]-1-[(phénylméthoxy)carbonyl]pipéridine-4-pentanoïque.

Un mélange de 3,55 g (8,0 mmoles) de (*S*)-α-[[(1,1-diméthyléthoxy)carbonyl]amino]-1-[(phénylméthoxy)carbonyl] pipéridine-4-pentanoate de méthyle et de 0,40 g (9,6 mmoles) d'hydroxyde de lithium monohydraté dans 15 ml de méthanol et 5 ml d'eau est agité 18 h à température ambiante. On évapore le méthanol sous pression réduite, puis on refroidit le mélange à 0°C, et on l'acidifie à pH 2 à l'aide d'une solution aqueuse 1N d'acide chlorhydrique, puis on l'extrait à deux reprises par 150 ml d'acétate d'éthyle: On lave l'extrait résultant avec 100 ml d'une solution saturée de chlorure de sodium, puis on sèche sur sulfate de sodium et on filtre et concentre le résidu obtenu. On obtient,sous forme de poudre blanche, 3 g d'acide (*S*)-α-[[(1,1-diméthyléthoxy)carbonyl]amino]-1-[(phénylméthoxy)carbonyl] pipéridine-4-pentanoique, utilisé tel quel à l'étape suivante.
Rdt (%) = 86

3.3. (*S*)-4-[4-[[(1,1-diméthyléthoxy)carbonyl]amino]-5-(4-éthylpipéridin-1-yl)-5-oxopentyl]pipéridine-1-carboxylate de phénylméthyle.

A une solution de 1,73 g (4,0 mmoles) d'acide (*S*)-α-[[(1,1-diméthyléthoxy)carbonyl]amino]-1-[(phénylméthoxy) carbonyl] pipéridine-4-pentanoïque, de 0,66 g (4,4 mmoles) de chlorhydrate de 4-éthylpipéridine et de 1,8 ml (10,4 mmoles) de *N*,*N*-diisopropyléthylamine (DIEA) dans du dichlorométhane, on ajoute à 0°C, sous azote, par petites quantités 1,7 g (4,4 mmoles) d'hexafluorophosphate de O-(benzotriazol-1-yl)-N,N,N,N'tétraméthyluronium (HBTU). On laisse revenir lentement le mélange à température ambiante et on poursuit la réaction pendant 18 heures. Le mélange réactionnel est repris ensuite par 200 ml d'acétate d'éthyle et est lavé par 100 ml d'une solution 1N d'acide chlorhydrique, puis avec 100 ml d'une solution saturée d'hydrogénocarbonate de sodium et avec 100 ml d'une solution saturée de chlorure de sodium. Le produit résultant est séché sur sulfate de sodium et est filtré puis concentré. Le résidu obtenu est purifié par chromatographie sur colonne de gel de silice en éluant sous pression par un mélange acétate d'éthyle : cyclohexane 4:6. On obtient, sous forme d'huile visqueuse, 2 g de (*S*)-4-[4-[[(1,1-diméthyléthoxy)carbonyl]amino]-5-(4-éthyl pipéridin-1-yl)-5-oxopentyl]pipéridine-1-carboxylate de phénylméthyle.
Rdt (%) = 95

3.4.Chlorhydrate de (*S*)-4-[4-amino-5-(4-éthylpipéridin-1-yl)-5-oxopentyl]pipéridine-1-carboxylate de phénylméthyle.

On traite une solution de 1,54 g (3,0 mmoles) de (*S*)-4-[4-[[(1,1-diméthyléthoxy)carbonyl]amino]-5-(4-éthylpipéridin-1-yl)-5-oxopentyl]pipéridine-1-carboxylate de phénylméthyle dans 60 ml de dichlorométhane pendant 5 min à 0°C par un courant de chlorure d'hydrogène. Après 3 heures à 0°C, on concentre sous pression réduite. Le produit résultant est utilisé tel quel à l'étape suivante. On obtient ainsi, sous forme d'une huile visqueuse, 1,35 g de chlorhydrate de (*S*)-4-[4-amino-5-(4-éthylpipéridin-1-yl)-5-oxopentyl]pipéridine-1-carboxylate de phénylméthyle.
Rdt (%) = 100

3.5.Chlorhydrate de (*S*)-*N*-[2-[[[1-[(4-éthylpipéridin-3-yl)carbonyl]-4-pipéridin-4-ylbutyl]amino] sulfonyl]-6-thién-2-ylphényl]propanamide.

A une solution de 1,35 g (3,0 mmoles) de chlorhydrate de (*S*)-4-[4-amino-5-(4-éthylpipéridin-1-yl)-5-oxopentyl] pipéridine-1-carboxylate de phénylméthyle et de 0,96 ml (6,9 mmoles) de triéthylamine (TEA) dans 15 ml de dichlorométhane, on ajoute à 0°C, par petites quantités 1,27 g (3,3 mmoles) de chlorure de 2-[bis(1-oxopropyl) amino]-3-thién-2-ylbenzènesulfonyle. On laisse le mélange revenir lentement à température ambiante et on poursuit la réaction pendant 18 heures. Le mélange réactionnel est repris dans 250 ml d'acétate d'éthyle et est lavé par 100 ml d'une solution 1N d'acide chlorhydrique, et avec 100 ml d'une solution saturée d'hydrogénocarbonate de sodium et avec 100 ml d'une solution saturée de chlorure de sodium. Le produit résultant est séché sur sulfate de sodium et est filtré puis concentré sous pression réduite. Le résidu obtenu est repris par 100 ml de tétrahydrofurane (THF)et est refroidi à 0°C, puis est traité pendant 5 minutes par un courant d'ammoniac. On laisse revenir le mélange à température ambiante. Après 4 heures, le mélange réactionnel est concentré sous pression réduite. Le résidu obtenu est repris par 250 ml d'acétate d'éthyle, est lavé par 50 ml d'une solution 1N d'acide chlorhydrique, puis avec 50 ml d'une solution saturée d'hydrogénocarbonate de sodium et avec 50 ml d'une solution saturée de chlorure de sodium. Le produit résultant est séché sur sulfate de sodium et est filtré puis concentré sous pression réduite. Le résidu obtenu est repris par 1,2 ml d'acide acétique, est refroidi à 0°C, puis est traité par une solution de bromure d'hydrogène 5N dans 1,2 ml d'acide acétique, ajouté goutte à goutte. On laisse revenir le mélange à température ambiante. Après 4 heures, le mélange réactionnel est concentré sous pression réduite. Le résidu obtenu est repris par 250 ml d'acétate d'éthyle et est lavé avec 50 ml d'une solution saturée d'hydrogénocarbonate de sodium puis avec 50 ml d'une solution saturée de chlorure de sodium. Le produit résultant est séché sur sulfate de sodium et est filtré puis est concentré après addition d'une solution 0,1 N de chlorure d'hydrogène dans 40 ml d'isopropanol. Le résidu obtenu est purifié par chromatographie sur une colonne RP18 en éluant par un gradient acétonitrile : eau de 5 : 95 à 30 : 70. On obtient 0,36 g de chlorhydrate de (*S*)-*N*-[2-[[[1-[(4-éthylpipéridin-3-yl)carbonyl]-4-pipéridin-4-ylbutyl] amino]sulfonyl]-6-thién-2-ylphényl]propanamide.
Rdt (%) = 22 ; F (°C) = 134-138 ;
$[\alpha]_D^{20}$ (°) = +112 (c = 0,2 ; méthanol)

Exemple 4 (composé n° 24):

Chlorhydrate de (*S*)-*N*-[3-[[[4-(5-aminopyridin-2-yl)-1-[[4-(difluorométhylène)pipéridin-1-yl]carbonyl]butyl]amino] sulfonyl]-3'-fluoro[1,1'-biphényl]-2-yl]propanamide.

4.1.(*S*)-2-[[(1,1-diméthyléthoxy)carbonyl]amino]-5-(5-nitropyridin-2-yl)pent-4-ynoate de méthyle.

Un mélange de 2-bromo-5-nitropyridine (10,0 g ; 49,0 mmoles), de (*S*)-2-[[(1,1-diméthyléthoxy)carbonyl] amino]pent-4-ynoate de méthyle (13,34 g ; 58,8 mmoles), d'iodure de cuivre (0,465 g ; 2,5 mmoles), de carbonate de potassium (13,6 g ; 98,0 mmoles) et de dichlorobis-(triphénylphosphine)-palladium (1,7 g ; 2,5 mmoles) dans 30 ml de diméthylformamide anhydre (DMF), est chauffé 4 heures à 60°C sous argon. Le mélange réactionnel est repris par 800 ml d'acétate d'éthyle, lavé par 2x400 ml d'eau, 400 ml d'hydrogénocarbonate saturé, 400 ml de saumure, séché sur du sulfate de sodium, filtré puis concentré sous pression réduite. Le résidu obtenu est purifié sur une colonne de silice en éluant par un gradient cyclohexane : acétate d'éthyle de 0 à 20% en acétate d'éthyle. On isole, sous forme d'huile visqueuse, 11 g de (*S*)-2-[[(1,1-diméthyléthoxy)carbonyl]amino]-5-(5-nitropyridin-2-yl)pent-4-ynoate de méthyle.
Rdt (%) = 65

4.2. (*S*)-5-amino-α-[[1,1-diméthyléthoxy)carbonyl] amino]pyridine-2-pentanoate de méthyle.

Un mélange de (*S*)-2-[[(1,1-diméthyléthoxy)carbonyl] amino]-5-(5-nitropyridin-2-yl)pent-4-ynoate de méthyle (10,5 g ; 30, 0 mmoles), de formiate d'ammonium (19,0 g ; 300,0 mmoles) et de palladium sur charbon actif à 10% (1,1 g) dans 100 ml de méthanol est chauffé 3 heures à reflux sous argon. Le mélange réactionnel est filtré puis concentré sous pression réduite. Le résidu obtenu est repris par 400 ml d'acétate d'éthyle, lavé par 100 ml de saumure, séchée sur sulfate de sodium, filtré puis concentré sous pression réduite. Le résidu est purifié sur une colonne de silice en éluant par un mélange dichlorométhane : méthanol (96:4). On isole, sous forme d'huile visqueuse, 6,5 g de (S)-5-amino -α-[[1,1-diméthyléthoxy)carbonyl]amino]pyridine-2-pentanoate de méthyle.
Rdt (%) = 62

4.3. Acide(*S*)-5-amino-α-[[(1,1-diméthyléthoxy) carbonyl]amino]pyridine-2-pentanoïque.

A un mélange de (*S*)-5-amino-α-[[1,1-diméthyléthoxy) carbonyl]amino]pyridin-2-pentanoate de méthyle. (6,2 g ; 17,6 mmoles) dans 45 ml de méthanol et 15 ml d'eau, on ajoute à 0°C de l'hydroxyde de lithium monohydraté (0,9 g ; 21,1 mmoles). On laisse revenir lentement à température ambiante et l'agitation est poursuivie 18 heures. Le mélange réactionnel est refroidi à 0°C, neutralisé à l'aide d'acide chlorhydrique 1N (22,0 ml ; 22,0 mmoles) puis concentré sous pression réduite. On isole, sous forme de poudre amorphe, 7, 0 g d'acide(*S*)-5-amino-α-[[(1,1-diméthyléthoxy) carbonyl]amino]pyridine-2-pentanoïque.
Rdt (%) = 100

4.4.(*S*)-[4-(5-Aminopyridin-2-yl)-1-[[4-difluoro méthylène)pipéridin-1-yl]carbonyl]butyl] carbamate de 1,1-diméthyléthyle.

On utilise le même mode opératoire qu'à l'exemple 1.4., à l'exception de la purification sur colonne de silice qui s'effectue par un gradient cyclohexane : acétate d'éthyle de 50 à 100% en acétate d'éthyle. Ainsi, à partir d'acide (S)-5-amino-α-[[(1,1-diméthyl éthoxy)carbonyl]amino] pyridine-2-pentanoique (3,14 g ; 8,0 mmoles) et de chlorhydrate de 4-(difluoro méthylène)pipéridine (1,63 g ; 9,6 mmoles), on isole, sous forme de solide blanc, 2,45 g de (*S*)-[4-(5-amino pyridin-2-yl)-1-[[4-difluorométhylène) pipéridin-1-yl]carbonyl]butyl]carbamate de 1,1-diméthyléthyle.
Rdt (%) = 72 ; F (°C) = 142.

4.5. Chlorhydrate de (S)-1-[2-amino-5-(5-amino pyridin-2-yl)-1-oxopentyl]-4-(difluorométhylène) pipéridine.

On utilise le même mode opératoire quà l'exemple 1.5. Ainsi, à partir de (*S*)-[4-(5-amino pyridin-2-yl)-1-[[4-(difluorométhylène)pipéridin-1-yl]carbonyl]butyl]carbamate de 1,1-diméthyléthyle (2,4 g ; 5,6 mmoles), on obtient, sous forme de poudre amorphe hygroscopique, 2,25 g de chlorhydrate de (S)-1-[2-amino-5-(5-aminopyridin-2-yl)-1-oxopentyl] -4-(difluorométhylène)pipéridine, utilisé tel quel à l'étape suivante.
Rdt (%) = 100

4.6. Chlorhydrate de (*S*)-*N*-[3-[[[4-(5-aminopyridin-2-yl)-1-[[4-difluorométhylène)pipéridin-1-yl] carbonyl]butyl]amino] sulfonyl]-3'-fluoro[1,1'-biphényl]-2-yl]propanamide.

**[0053]** - On utilise le même mode opératoire qu'à l'exemple 1.6., à l'exception de la purification sur colonne RP18 qui s'effectue par un gradient eau : acétonitrile de 0 à 40% en acétonitrile. Ainsi, à partir de chlorhydrate de (*S*)-1-[2-amino-5-(5-aminopyridin-2-yl)-1-oxopentyl] -4-(difluorométhylène)pipéridine (0,65 g ; 1,6 mmoles) et chlorure de [2-[bis (1-oxopropyl)amino]-3'-fluoro 1,1'-biphényl]-3-yl]sulfonyle (0,69 g ; 1, 6 mmoles) , on isole 0,69 g de chlorhydrate de (*S*)-*N*-[3-[[[4-(5-aminopyridin-2-yl) 1-[[4-(difluorométhylène)pipéridin-1-yl]carbonyl]butyl]amino] sulfonyl]-3'-fluoro[1,1'-biphényl]-2-yl]propanamide.
Rdt (%) = 65 ; F (°C) = 136-140 ;
$[\alpha]_D^{20}$ (°) = +90 (c = 0, 2 ; méthanol)

<u>Exemple 5</u> (composé n° 6 ) :

Chlorhydrate de (*S*)-*N*-[2-[[[4-(5-Aminopyrazin-2-yl)-1-[[4-(difluorométhylène)pipéridin-1-yl]carbonyl]butyl]amino] sulfonyl]-6-cyclopentylphényl]propanamide.

5.1. 5-bromopyrazin-2-amine.

A une solution de 2-aminopyrazine (15,0 g, 0,158 mole) dans 900 ml de dichlorométhane, on ajoute à 0°C par petites quantités 28,2 g de *N*-bromosuccinimide (0,158 mole). Après 3 heures, le mélange réactionnel est filtré sur fritté, lavé par du carbonate de sodium saturé (2x400 ml), de l'eau (400 ml), de la saumure (200 ml), séché sur sulfate de sodium, filtré et concentré sous pression réduite. Le résidu obtenu est purifié sur une colonne de silice éluée sous pression par un gradient cyclohexane : acétate d'éthyle (9 : 1) à (1 : 1). On obtient, sous forme de poudre jaunâtre, 18,0 g de 5-bromopyrazin-2-amine.
Rdt (%) = 66 ; F (°C) = 114.

5.2.(*S*)-5-(5-aminopyrazin-2-yl)-2-[[(1,1-diméthyl éthoxy)carbonyl]amino]pent-4-ynoate de méthyle.

On procède de la même façon qu'à l'exemple 1.2., et à partir de 7,5 g (43,0 mmoles) de 5-bromopyrazin-2-amine et de 11,71 g (51,6 mmoles) de (S)-2-[[(1,1-diméthyléthoxy) carbonyl]amino]pent-4-ynoate de méthyle, on obtient, après 7 h à température ambiante, et sous forme d'une huile visqueuse, 10,6 g de (*S*)-5-(5-aminopyrazin-2-yl)-2-[[(1,1-diméthyléthoxy)carbonyl]amino]pent-4-ynoate de méthyle.
Rdt (%) = 77

5.3. (*S*)-5-(5-Aminopyrazin-2-yl)-2-[[(1,1-diméthyléthoxy)carbonyl]amino]pentanoate de méthyle.

Un mélange de (*S*)-5-(5-aminopyrazin-2-yl)-2-[[(1,1-diméthyléthoxy) carbonyl]amino]pent-4-ynoate de méthyle (10,0 g ; 31,2 mmoles), de formiate d'ammonium (29,7 g ; 468,0 mmoles) et de palladium sur charbon actif à 10% (1,2 g) dans du méthanol (100 ml) est chauffé 3 heures à reflux. Le mélange réactionnel est filtré et concentré sous pression réduite. Le résidu obtenu est repris par de l'acétate d'éthyle (300 ml), de la saumure (2×200 ml), séché sur sulfate de sodium, filtré et concentré sous pression réduite. Le résidu obtenu est purifié sur une colonne de silice éluée sous pression par un mélange dichlorométhane : méthanol (96:4). On isole, sous forme d'une huile visqueuse, 7,9 g de (*S*)-5-(5-aminopyrazin-2-yl)-2-[[(1,1-diméthyléthoxy) carbonyl]amino]pentanoate de méthyle.
Rdt (%) = 79

5.4. Acide(*S*)-5-(5-aminopyrazin-2-yl)-2-[[(1,1-diméthyl éthoxy)carbonyl]amino]pentanoïque.

A une solution de (*S*)-5-(5-aminopyrazin-2-yl)-2-[[(1,1-diméthyléthoxy)carbonyl]amino]pentanoate de méthyle (7,75 g, 23,9 mmoles) dans du méthanol (60 ml) et de l'eau (20 ml), on ajoute à 0°C de l'hydroxyde de lithium (1,2 g, 28,6 mmoles). On laisse revenir à température ambiante et poursuivre 18 heures. Le méthanol est évaporé sous pression réduite, le résidu obtenu est refroidi à 0°C, acidifié à pH 3-4 à l'aide d'acide chlorhydrique 1N (30 ml), extrait à l'acétate d'éthyle (2x200 ml), lavé par de la saumure (50 ml), séché sur sulfate de sodium, filtré et concentré sous presion réduite. On obtient, sous forme d'une poudre blanche, et utilisé tel quel à l'étape suivante, 7,17 g de (*S*)-5-(5-aminopyrazin-2-yl) -2-[[(1,1-diméthyléthoxy)carbonyl]amino]pentanoïque.

Rdt (%) = 97 ; F (°C) = 76.

5.5. (*S*)-[4-(5-Aminopyrazin-2-yl)-1-[[4-(difluoro méthylène)pipéridin-1-yl]carbonyl]butyl]carbamate de 1,1-diméthyléthyle.

On procède de la même façon que l'exemple 1.4., et à partir de 2,32 g (7,5 mmoles) d'acide (*S*)-5-(5-aminopyrazin-2-yl) -2-[[(1,1-diméthyléthoxy)carbonyl]amino] pentanoïque et de 1,9 g (11,25 mmoles) de chlorhydrate de 4-(difluoro méthylène)pipéridine, on obtient, sous forme de poudre amorphe, 2,61 g de (*S*)-[4-(5-aminopyrazin-2-yl)-1-[[4-(difluorométhylène) pipéridin-1-yl]carbonyl]butyl]carbamate de 2,1-diméthyléthyle.
Rdt (%) = 82 ; F (°C) = 133.

5.6. Chlorhydrate de (*S*)-1-[2-amino-5-(5-aminopyrazin-2-yl)-1-oxopentyl]-4-(difluorométhylène)pipéridine (2:1).

On procède de la même façon qu'à l'exemple 1.5., et à partir de 2,6 g (6,1 mmoles) de (*S*)-[4-(5-amino pyrazin-2-yl) -1-[[4-(difluorométhylène)pipéridin-3-yl]carbonyl]butyl] carbamate de 1,1-diméthyl éthyle, on obtient, sous forme d'huile visqueuse, 2,45 g de chlorhydrate de (*S*)-1-[2-amino -5-(5-aminopyrazin-2-yl)-1- oxopentyl]-4-(difluorométhylène) pipéridine (2:1).
Rdt (%) = 100

5.7. Chlorhydrate de (S) -N,- [2- [[[4- (5-aminopyrazin-2-yl)-1-[[4-(difluorométhylène)pipéridin-1-yl]carbonyl]butyl] amino]sulfonyl]-6-cyclopentylphènyl]propanamide

On procède de la même façon qu'à l'exemple 1.6., et on obtient à partir de 1,11 g (3,0 mmoles) de chlorure de 2-[bis(1-oxopropyl)amino]-3-cyclopentylbenzènesulfonyle et de 1,21 g (3,0 mmoles) de chlorhydrate de (*S*)-1-[2-amino-5-(5-aminopyrazin-2-yl)-1-oxopentyl]-4-(difluorométhylène) pipéridine (2:1), 1,3 g de (S)-N-[2-[[[4-(5-aminopyrazin-2 -yl)-1-[[4-(difluorométhylène)pipéridin-1-yl]carbonyl]butyl] amino]sulfonyl]-6-cyclopentylphényl]propanamide.
Rdt (%) = 68 ; F (°C) = 136-140 ;
$[\alpha]_D^{20}$ (°) = +103 (c = 0,2 ; méthanol).

Exemple 6 (composé n° 71) :

Chlorhydrate de *N*-[3-[[[(1*S*)-4-(5-aminopyridin-2-yl)-1-[(4-méthylpipéridin-1-yl)carbonyl]butyl] amino]sulfonyl][1,1'-biphényl]-2-yl]acétamide.

6.1. Chlorure de 2-(diacétylamino) [1,1'-biphényle]-3-sulfonyle.

Une solution de sel de *N,N*-diéthyléthanamine de l'acide 2-amino[1,1'-biphényle]-3-sulfonique (31,2g ; 89,0 mmoles) dans de l'anhydride acétique (93,0 ml) est chauffée 4 heures à reflux. Le mélange réactionnel est concentré sous pression réduite. Le résidu obtenu est repris par du dichlorométhane (250,0 ml), refroidi à 0°C puis on ajoute du pentachlorure de phosphore (37,40 g ; 178,0 mmoles). Après 6 heures à 0°C, le mélange réactionnel est concentré sous pression réduite. Le résidu obtenu est repris par de l'éther (500 ml), lavé par de la saumure (100 ml), séché sur sulfate de sodium, filtré puis concentré. Le résidu est chromatographié sur une colonne de Florisil® en éluant avec un gradient n-hexane/éther de 0 à 60 % en éther. On isole 14,1 g de chlorure de 2-(diacétylamino)[1,1'-biphényle]-3-sulfonyle, sous la forme d'un solide blanc amorphe.
Rdt (%) = 45,0

6.2.[(*S*)-4-(5-aminopyridin-2-yl)-1-[(4-méthyl-pipéridin-1-yl)carbonyl]butyl]carbamate de 1,1-diméthyléthyle

On utilise le même mode opératoire qu'à l'exemple 1.4, à l'exception de la purification sur colonne de silice qui s'effectue par un gradient acétate d'éthyle : méthanol de 0 à 10 % en méthanol. Ainsi, à partir d'acide (S)-5-amino-α-[[(1,1-diméthyléthoxy)carbonyl]amino]pyridine-2-pentanoïque (11,0g ; 31,0 mmoles) et de 4-méthylpipéridire (5,5 ml ; 46,0 mmoles), on isole 10,3g de [(*S*)-4-(5-aminopyridin-2-yl) -1-[(4-méthylpipéridin-1-yl)carbonyl]butyl]carbamate de 1,1-diméthyléthyle.
Rdt (%) = 86.

6.3. Chlorhydrate de 1-[(2S)-2-amino-5-(5-aminopyridin-2-yl)-1-oxopentyl]-4-méthylpipéridine.

On utilise le même mode opératoire qu'à l'exemple 1.5. Ainsi, à partir de [(*S*)-4-(5-aminopyridin-2-yl)-1-[(4-méthylpipéridin-1-yl)carbonyl]butyl]carbamate de 1,1-diméthyléthyle (10,2 g ; 26,0 mmoles), on obtient, sous forme de poudre amorphe hygroscopique, 9,5 g de chlorhydrate de 1-[(2S)-2-amino-5-(5-aminopyridin-2-yl)-1-oxopentyl]-4-méthylpipéridine, utilisé tel quel à l'étape suivante.
Rdt (%) = 100

6.4. Chlorhydrate de *N*-[3-[[[(1*S*)-4-(5-aminopyridin-2-yl)-1-[(4-méthylpipéridin-1-yl)carbonyl]butyl] amino]sulfonyl] [1,1'-biphényl]-2-yl]acétamide.

On procède de la même façon qu'à l'exemple 1.6. Ainsi, à partir de chlorure de 2- (diacétylamino) [1,1'-biphényle]-3-sulfonyle (4,57g ; 13,0 mmoles) et de chlorhydrate de 1-[(2*S*)-2-amino-5-(5-aminopyridin-2-yl)-1-oxopentyl]-4-méthylpipéridine, on obtient 5,2g de chlorhydrate de *N*-[3-[[[(1*S*) -4- (5-aminopyridin-2-yl) -1- [(4-méthylpipéridin-1-yl)carbonyl]butyl] amino]sulfonyl][1,1'-biphényl]-2-yl] acétamide.
Rdt (%) = 66 ; F(°C) = 176-180 ;
$[\alpha]_D^{20}$ (°) = +184 (c = 0,2 ; méthanol)

<u>Exemple 7</u> (composé n° 52) :

Chlorhydrate de *N*-[[[3-[(1*S*)-4-(5-aminopyridin-2-yl)-1-[[4-(difluorométhylène)pipéridin-1-yl]carbonyl]butyl] amino]sulfonyl] [1,1'-biphényl]-2-yl]acétamide.

On procède de la même façon qu'à l'exemple 1.6. Ainsi, à partir de 3,52 g (10,0 mmoles) de chlorure de 2-(diacétylmino) [1,1'-biphényle]-3-sulfonyle et de 4,2 g (10,5 mmoles) de (*S*)-[4-(5-aminopyridin-2-yl)-1-[[4-difluorométhylène) pipéridin-1-yl]carbanyl]butyl]carbamate de 1,1-diméthyléthyle, on obtient 4,5 g de *N*-[[[3-[(1*S*)-4-(5-aminopyridin-2 yl)-1-[[4-(difluorométhylène)pipéridin-1-yl]carbonyl]butyl] amino]sulfonyl] [1,1'-biphényl]-2-yl]acétamide.
Rdt (%) = 71 ; F(°C) = 170-174 ;
$[\alpha]_D^{20}$ (°) = +90 (c = 0,2 ; méthanol).

<u>Exemple 8</u> (composé n° 114) :

Chlorhydrate de N-[3-[[[(1S)-4-(6-aminopyridin-3-yl)-1-[(4-méthylpipéridin-1-yl)carbonyl]butyl]amino]sulfonyl]-5-phénylpyridin-4-yl]propanamide.

8.1 (3,5-dibromopyridin-4-yl)carbamate de 1,1-diméthyléthyle.

Un mélange de pyridin-4-ylcarbamate de 1,1-diméthyléthyle (11,0 g ; 57,0 mmoles) et de N-bromosuccinimide (25,6 g ; 142,0 mmoles) dans de l'acétonitrile (50 ml) est chauffé 12 heures à 55°C. Le mélange réactionnel est concentré sous pression réduite. Le résidu obtenu est repris par de l'éther (400 ml), lavé avec une solution aqueuse saturée de bicarbonate de potassium (2 x 200 ml), séché sur sulfate de sodium, filtré puis concentré sous pression réduite. Le résidu est chromatographié sur une colonne de silice en éluant avec un gradient acétate d'éthyle / cyclohexane de 0 à 10 % en acétate d'éthyle. On isole 8,2 g de (3,5-dibromopyridin-4-yl)carbamate de 1,1-diméthyléthyle, solide blanc.
Rdt (%) = 41

8.2 (3-bromo-5-phénylpyridin-4-yl)carbamate de 1,1-diméthyléthyle.

Un mélange de (3,5-dibromopyridin-4-yl)carbamate de 1,1-diméthyléthyle(4,2 g ; 12,0 mmoles), d'acide phénylboronique (1,76 g ; 14,4 mmoles), de carbonate de sodium (3,1 g ; 29,2 mmoles) et de tétrakis (triphénylphosphine) palladium (0) (0,416 g ; 0,36 mmoles) dans du dioxane (24 ml) et de l'eau (12 ml) est chauffé sous argon 8 heures à 70°C. Le mélange réactionnel est concentré sous pression réduite. Le résidu obtenu est repris par de l'acétate d'éthyle (200 ml), lavé avec de l'eau (2 x 100 ml), de la saumure (100 ml), séché sur du sulfate de sodium, filtré puis concentré sous pression réduite. Le résidu est chromatographié sur une colonne de silice en éluant avec un gradient acétate d'éthyle / cyclohexane de 0 à 5 %. On isole 2,3 g de (3-bromo-5-phénylpyridin-4-yl)carbamate de 1,1-diméthyléthyle, solide blanc.
Rdt (%) = 55

8.3 Tributyl [(phénylméthyl)thio]stannane.

A une solution de benzèneméthanethiol (11,72 ml ; 100,0 mmoles) dans du DMF anhydre (20,0 ml), on ajoute à 20°C, sous argon, goutte à goutte du 1,8-diazabicyclo [5.4.0.]undéc-7-ène (DBU). Après 0,5 heure à 20°C, le mélange

réactionnel est refroidi à 0°C et on additionne goutte à goutte le chlorure de tributylstannane. On laisse revenir à température ambiante et l'agitation est poursuivie 5 heure. Le mélange réactionnel est repris par du pentane (400 ml), lavé avec de l'eau (3 x 300 ml), de la saumure (100 ml), séché sur sulfate de sodium, filtré puis concentré sous pression réduite. On obtient 39,0 g de tributyl[(phénylméthyl)thio] stannane, huile incolore, utilisée telle quelle à l'étape suivante.
Rdt (%) = 95

8.4 [3-phényl-5-[(phénylméthyl)thio]pyridin-4-yl]carbamate de 1,1-diméthyléthyle.

Un mélange de (3-bromo-5-phénylpyridin-4-yl)carbamate de 1,1-diméthyléthyle(2,30 g ; 6,6 mmoles), de tributyl [(phénylméthyl) thio] stannane (2,44 ml ; 7,3 mmoles) et de tétrakis(triphénylphosphine) palladium (0) (0,30 g ; 0,26 mmoles) dans du dioxane anhydre (4,0 ml) est chauffé 10 heures sous argon à 90°C. Après 4 heures et 6 heures, on ajoute du tétrakis(triphénylphosphine) palladium (0) (0,30 g et 0,15 g respectivement). Le mélange réactionnel est repris par de l'éther (100 ml), traité 0,5 heure par une solution aqueuse de fluorure de potassium à 5 % (50 ml) puis filtré sur fritté. Le filtrat est lavé par de la saumure (50 ml), séché sur sulfate de sodium, filtré puis concentré sans pression réduite. Le résidu obtenu est chromatographié sur une colonne de silice en éluant avec un gradient acétate d'éthyle /cyclohexane de 0 à 20 % en acétate d'éthyle. On isole 1,0 g de [3-phényl-5-[(phénylméthyl)thio]pyridin-4-yl] carbamate de 1,1-diméthyléthyle, solide blanc.
Rdt (%) = 45

8.5 3-phényl-5-[(phénylméthyl)thio]pyridin-4-amine.

Une solution de [3-phényl-5-[(phénylméthyl)thio]pyridin-4-yl]carbamate de 1,1-diméthyléthyle (0,9 g ; 2,7 mmoles) dans du méthanol (50 ml) est traitée 5 minutes à 0°C par un courant d'acide chlorhydrique. On laisse revenir à température ambiante et l'agitation est poursuivie 6 heures. Le mélange réactionnel est concentré sous pression réduite. Le résidu obtenu est repris par de l'acétate d'éthyle (150 ml), traité par une solution aqueuse saturée de carbonate de potassium (20 ml), séché sur sulfate de sodium, filtré puis concentré sous pression réduite. On obtient 0,8-g de-3-phényl-5-[(phénylméthyl)thio]pyridin-4-amine, huile visqueuse, utilisée telle quelle à l'étape suivante.
Rdt (%) = 100

8.6 Chlorure de 4-[bis(1-oxoproyl)amino]-5-phénylpyridine-3-sulfonyle.

Une solution de 3-phényl-5-[(phénylméthyl)thio]pyridin-4-amine (0,8 g ; 2,74 mmoles) dans de l'anhydride propionique est chauffée 6 heures à 150°C. Le mélange réactionnel est concentré sous pression réduite et utilisé tel quel à l'étape suivante. A un mélange du brut obtenu ci-dessus dans de l'acide acétique (3 ml) et de l'eau (0,2 ml), on ajoute goutte à goutte à 5°C du chlorure de sulfuryle (0,75 ml ; 9,4 mmoles). Après 0,5 heure à 5°C, le mélange réactionnel est concentré sous pression réduite. Le résidu obtenu est repris par de l'éther (150 ml), lavé avec de l'eau (50 ml), de la saumure (50 ml), séché sur sulfate de sodium, filtré puis concentré sous pression réduite. On obtient 1,1 g de chlorure de 4-[bis(1-oxoproyl)amino]-5-phénylpyridine-3-sulfonyle, huile visqueuse, utilisée telle quelle à l'étape suivante.
Rdt (%) = 100

8.7 Chlorhydrate de N-[3-[[[(1S)-4-(6-aminopyridin-3-yl)-1-[(4-méthylpipéridin-1-yl)carbonyl]butyl]amino] sulfonyl]-5-phénylpyridin-4-yl]propanamide.

A un mélange de chlorhydrate de 1-[(2S)-2-amino-5-(6-aminopyridin-3-yl)-1-oxopentyl]-4-méthylpipéridine (0,35 g ; 1,1 mmoles) et de triéthylamine (0,45 ml ; 3,3 mmoles) dans du dichlorométhane (3 ml), on ajoute goutte à goutte à 0°C une solution de chlorure de 4-[bis(1-oxoproyl)amino]-5-phénylpyridine-3-sulfonyle (0,5 g ; -1,0 mmole) dans du dichlorométhane (3 ml) . Après 6 heures à 0°C, on reprend le mélange réactionnel par de l'acétate d'éthyle (100 ml), lave avec de la saumure (50 ml), sèche sur sulfate de sodium, filtre puis on concentre sous pression réduite. Le résidu obtenu est repris par du tétrahydrofurane (20,0 ml), refroidi à 0°C puis traité 5 minutes par un courant d'ammoniac gaz. Après 4 heures à température ambiante, le mélange réactionnel est concentré sous pression réduite. Le résidu est chromatographié sur une colonne de silice en éluant avec un gradient dichlorométhane / méthanol de 0 à 5% en méthanol. On isole 0,45 g de base (rdt (%) = 77) que l'on reprend par 2 ml d'une solution d'acide chlorhydrique 0,1N dans l'isopropanol et on concentre sous pression réduite. Le résidu est purifié par chromatographie sur colonne de gel de silice RP18 en éluant par un mélange acétonitrile / eau 3:7. On isole 0,42 g de chlorhydrate de N-[3-[[[(1S)-4-(6-aminopyridin-3-yl)-1-[(4-méthylpipéridin-1-yl)carbonyl] butyl]amino]sulfonyl]-5-phénylpyridin-4-yl]propanamide.
Rdt (%) = 66 ; F (°C) = 180-184 ;
$[\alpha]_D^{20}$ (°) = +100 (c = 0,2 ; méthanol).

Exemple 9 (composé n° 118) :

Chlorhydrate de [1,1'-biphényl]-3-sulfonamide, *N*-[(1*S*)-4-(5-amino-2-pyridinyl)-1-[[4-difluorométhylène)-1-pipéridinyl] carbonyl]butyl]-2-(formylamino)-3'-méthyle.

9.1 [1,1'-biphényl]-3-sulfonamide-2-amino-*N* [(1S)-4-(5-amino-2-pyridinyl)-1-[[4-difluorométhylène)-1-pipéridinyl]carbonyl]butyl]-3'-méthyle.

A une solution agitée de 0,64 g (1,77 mmol) de dichlorhydrate de (1*S*)-5-amino-α-[[4-difluorométhylène)-1-pipéridinyl]carbonyl]-2-pyridinebutanamine dans 10 ml de dichlorométhane, on ajoute goutte à goutte 0,74 ml de de triéthylamine (5,31 mmol) et on ajoute ensuite une solution de chlorure de 2-amino-3'-méthyl-[1,1'-biphenyl]-3-sulfonyle, (0,50 g ; 1,77 mmol) dans 2 ml de dichlorométhane, goutte à goutte à 0°C. Après 16 h d'agitation à température ambiante, le milieu réactionnel est évaporé sous pression réduite. Le résidu est repris par 100 ml d'acétate d'éthyle et lavé par 25 ml d'une solution saturée de bicarbonate de sodium, puis par 25 ml d'une solution saturée de chlorure de sodium et enfin séché sur $Na_2SO_4$. Le solvant est évaporé sous pression réduite et le résidu, chromatographié sur silice dans le mélange dichlorométhane/methanol de 0 à 10 % de méthanol, donne 0,6 g de produit pur.
Rdt (%) = 60

9.2 Chlorhydrate de [1,1'-biphényl]-3-sulfonamide, *N*-[(1*S*)-4-(5-amino-2-pyridinyl)-1-[[4-difluorométhylène)-1-pipéridinyl]carbonyl]butyl]-2-(formylamino)-3'-méthyle.

Une solution de 0,55 g de [1,1'-biphényl]-3-sulfonamide-2-amino-*N* [(*1S*)-4-(5-amino-2-pyridinyl)-1-[[4-difluorométhylène)-1-pipéridinyl]carbonyl]butyl]-3'-méthyle (1 mmole) dans 3 ml d'orthoformate d'éthyle est chauffée sous agitation et sous argon à 125°C pendant 7 h. Le milieu réactionnel est ensuite versé sur une solution de 50 ml d'acide acétique et 50 ml d'eau et chauffé à 100°C pendant 1 h. Après évaporation sous pression réduite le résidu est repris dans 100 ml d'acétate d'éthyle, lavé par 50 ml d'une solution saturée de chlorure de sodium et séchée sur $Na_2SO_4$. Le solvant est évaporé sous pression réduite et le résidu repris par 10 ml d'une solution 0,1 N d'acide chlorhydrique dans l'isopropanol et évaporé sous pression réduite. Le produit est ensuite chromatographié sur silice RP18 dans le mélange acide chlorhydrique N/100/acétonitrile, de 0 à 100 % d'acétonitrile. On obtient ainsi 0,22 g du produit désiré.
Rdt (%) = 37 ; F(°C) = 168 ;
$[\alpha]_D^{20}$ (°) = +108 (c = 0,2 ; méthanol).

Exemple 10 (composé n° 123) :

Chlorhydrate de *N*-[2-[[[(1S,3Z)-4-(5-amino-2-pyridinyl)-1-[[4-difluorométhylène)-1-pipéridinyl]carbonyl]-3-butènyl] amino]sulfonyl]-6- (2-thiényl)phényl] -acétamide

10.1 (2*S*)-2-[(2,2-diméthyl-1-oxopropoxy)amino]-5-nitro-2-pyridinyl)-4-pentynoate de méthyle.

A une solution de 11,95 g (52,6 mmol) de (2*S*)-2-[(2,2-diméthyl-1-oxopropoxy)amino]-4-pentynoate de méthyle et de 10 g (63,1 mmol) de 2-chloro-5-nitropyridine dans 100 ml de dichlorométhane, on ajoute 18,3 ml (105,2 mmol) de diisopropyléthylamine puis 380 mg (2,6 mmol) de bromure de cuivre. Le milieu est dégazé par barbotage d'argon pendant 15 min. Sous argon, 740 mg (1,05 mmol) de tétrakistriphénylphosphine palladium (o) est additionné au milieu réactionnel qui est ensuite porté à reflux (température = 40°C) pendant 4 h. Le milieu noircit. On évapore le dichlorométhane puis on reprend le résidu par 500 ml d'acétate d'éthyle. La phase organique est lavée par une solution de chlorure de sodium saturée, séchée sur sulfate de sodium anhydre puis évaporée à sec. Le résidu d'évaporation est purifié sur silice par un mélange cyclohexane acétate d'éthyle (85/15). On obtient 16,6 g d'une poudre marron.
Rdt (%) = 90

10.2 (2*S*)-5-(5-amino-2-pyridinyl)-2-[(2,2)diméthyl-1-oxopropoxy)amino]-4-pentynoate de méthyle.

On porte à reflux pendant 5 h un mélange de 11,6 g (33,2 mmol) de (2*S*)-2-[(2,2-diméthyl-1-oxopropoxy) amino]-5-nitro-2-pyridinyl)-4-pentynoate de méthyle, de 6,5 g (116,2 mmol)de fer, de 100 ml d'eau, 200 ml d'éthanol et de 20 ml d'acide acétique. On évapore l'éthanol puis on filtre le milieu sur célite. Le produit est extrait au dichlorométhane. La phase organique est séchée sur sulfate de magnésium anhydre puis évaporée à sec. Le résidu d'évaporation est purifié sur silice par un mélange dichlorométhane / méthanol (97 :3). On obtient 8 g d'une huile marron.
Rdt (%) = 80

10.3 (*2S*, *4Z*)-5-(5-amino-2-pyridinyl)-2-[(2,2-diméthyl-1-oxopropoxy)amino]-4-pentènoate de méthyle.

4 g de (12,5 mmol) de (*2S,4Z*)-5-(5-amino-2-pyridinyl)-2-[(2,2-diméthyl-1-oxopropoxy)amino]-4-pentynoate de méthyle dissout dans 100 ml d'acétate d'éthyle sont placés sur l'appareillage de Parr et hydrogénés à température ambiante à une pression de 8 psi, pendant 4h. On filtre sur papier Whatman et on évapore le filtrat. Le résidu brut est purifié sur silice par un mélange cyclohexane / acétate d'éthyle (1:1). On obtient 1,8 g de poudre marron clair.
Rdt (%) = 45

10.4 Acide (*2S,4Z*)-5-(5-amino-2-pyridinyl)-2-[(2,2-diméthyl-1-oxopropoxy)amino]-4-pentènoique.

A une solution de (*2S,4Z*)-5-(5-amino-2-pyridinyl)-2-[(2,2-diméthyl-1-oxopropoxy) amino]-4-pentènoate de méthyle (1,8 g soit 5,6 mmol) dans un mélange méthanol / eau (45/15), on additionne 260 mg d'hydroxyde de lithium (6,16 mmol). On laisse une nuit sous agitation à température ambiante. On évapore le milieu à sec par distillation azéotropique du toluène. On ajoute 1,6 ml d'acide chlorhydrique 4N dans le dioxane et 30 ml de dichlorométhane dans le milieu. On évapore de nouveau à sec. On obtient quantitativement le composé attendu sous forme d'une gomme orangée.

10.5 6-(*1Z,4S*)-5-[4-(difluorométhylène)-1-piperidinyl]-4-[2,2-diméthyl-1-oxopropoxy)amino]-5-oxo-1-pentènyle-3-pyridinamine.

Sous argon, à une solution de 1,07 g (6,2 mmol) de 4-difluorométhylène-pipéridine dans un mélange dichlorométhane/diméthylformamide (8:2), on ajoute 2,82 ml (16,1 mmol) de diisopropyléthylamine. Le milieu est refroidi à 0°C. A cette température, on ajoute 2,2 g (6,2 mmol) d'acide (*2S,4Z*)-5-(5-amino-2-pyridinyl)-2-[(2,2-diméthyl-1-oxopropoxy)amino]-4-pentènoique et 2,2 g (6,93 mmol) de TBTU. Le milieu est laissé sous agitation toute la nuit remonter à température ambiante. Le milieu est évaporé à sec et repris par de l'acétate d'éthyle, lavé par une solution de carbonate de sodium saturée. La phase organique est séchée sur sulfate de sodium anhydre et évaporé à sec. Le résidu d'évaporation est purifié sur silice par un mélange cyclohexane / acétate d'éthyle / méthanol (45/45/10).
Rdt (%) = 75.

10.6 Chlorhydrate de *N*-[2-[[[(1S,3Z)-4-(5-amino-2-pyridinyl)-1-[[4-difluorométhylène)-1-pipéridinyl]carbonyl]-3-butènyl]amino]sulfonyl]-6-(2-thiényl)phényl]-acétamide

On ajoute 46 mmol (11,5 ml) d'acide chlorhydrique 4N dans le dioxane à une solution de 1,95 g (4,6 mmol) de 6-(*1Z,4S*)-5-[4-(difluorométhylène)-1-pipéridinyl]-4-[2,2-diméthyl-1-oxopropoxy) amino]-5-oxo-1-pentènyle-3-pyridinamine dans 40 ml de dichlorométhane. On laisse le milieu sous agitation une nuit à température ambiante. On évapore le milieu à sec. 351 mg (0,98 mmol) du résidu sont repris par 3 ml de dichlorométhane, on y ajoute 401 µl (2,94 mmol) de triéthylamine. Le milieu est refroidi à 0°C. A cette température, on ajoute 350 mg (0,98 mmol) de chlorure de 2-[(diacétylamino)-1,1'-biphényl]-3-sulfonyle. On laisse sous agitation revenir à température ambiante pendant 1 h. Le milieu est lavé avec une solution aqueuse de chlorure de sodium, puis évaporé à sec. Le résidu est repris par 5 ml de tétrahydrofurane. Au milieu refroidi à 0°C, on fait barboter de l'ammoniac pendant 45 min. On évapore le tétrahydrofuranne, puis on purifie sur silice par un mélange cyclohexane / acétate d'éthyle (3:7) puis sur phase inverse à l'aide d'un gradient acide chlorhydrique N/100-acétonitrile de 0 % à 100 % d'acétonitrile.
Rdt (%) = 20 ; F(°C) = 170 ;
$[\alpha]_D^{20}$ (°) = +40 (c = 0,2 ; méthanol).

<u>Exemple 11</u> (composé n° 121) :

Chlorhydrate de *N*-[3-[[[(1S,3E)-4-(5-amino-2-pyridinyl)-1-[[4-difluorométhylène)-1-pipéridinyl]carbonyl]-3-butènyl] amino]sulfonyl][1,1'-biphényl]-2-yl-acétamide

11.1. (2*S*)-2-[(triphénylméthylamino]4-pentynoate de méthyle.

A une solution de 2 g (8 ,8 mmol) de (*S*)-2-[[1,1-diméthyléthoxy)carboxy]amino]pent-4-ynoate de méthyle dans 5 ml de dichlorométhane, on ajoute 22 ml (88 mmol) d'acide chlorhydrique 4N dans le dioxane. On laisse sous agitation 2 h à température ambiante. Le milieu est évaporé à sec puis repris par 8 ml de dichlorométhane. On y ajoute 1,8 ml (13,2 mmol) de triéthylamine. Le milieu est refroidi à 0°C. A cette température, on y ajoute 2,6 g (0,65 mmol) de chlorure de trityle. On laisse sous agitation remonter à température ambiante toute la nuit. Le milieu est lavé à l'eau, séché sur sulfate de sodium anhydre puis évaporé à sec. Le résidu d'évaporation est purifié sur silice par un mélange

cyclohexane / acétate d'éthyle (9:1). On obtient 3 g d'un solide visqueux blanc.
Rdt (%) = 92.

11.2. (*2S*)-5-(tributylstannyl)-2-[(triphénylméthyl) amino]4-pentènoate de méthyle.

Dans une solution de 200 mg (0,54 mmol) de (*2S*)-2-[(triphénylméthylamino]4-pentynoate de méthyle dans 2 ml de tétrahydrofuranne anhydre, on fait barboter de l'argon pendant 10 min. On ajoute 5 % de tétrakis triphénylphosphine palladium (o) (3 mg ;. $2{,}7.10^{-3}$ mmol) . Lorsque le milieu est homogène, on refroidit à 0°C. Sous argon, à 0°C, on additionne au goutte à goutte 173 µl (0,65 mmol) d'hydrure de tributylétain. Le milieu devient jaune opaque. On laisse 2 h sous agitation remonter à température ambiante. Le milieu est évaporé à sec puis purifié sur florisil® en éluant par du cyclohexane pur. On obtient un liquide visqueux transparent.

11.3. (*2S,4E*)-5-(5-nitro-2-pyridinyl)-2-[(triphénylméthyl)amino]-4-pentènoate de méthyle.

Dans une solution de 80 mg (0,13 mmol) de (2*S*)-5-(tributylstannyl)-2-[(triphénylméthyl)amino]4-pentènoate de méthyle et de 39,5 mg (0,19 mmol) de 2-bromo-5-nitropyridine dans 1 ml de dioxane anhydre, on fait barboter de l'argon pendant 10 min. On ajoute ensuite 11,4 mg (0,0125 mmol) de tétrakis triphénylphosphine palladium (o). On porte à 110°C, sous argon, pendant 24 h. Le milieu est lavé par une solution de fluorure de potassium saturée puis extrait à l'acétate d'éthyle. La phase organique est séchée sur sulfate de sodium anhydre, puis concentrée sous pression réduite. Le résidu d'évaporation est purifié sur silice par un mélange cyclohexane / acétate d'éthyle 98:2 puis 95: 5. On obtient 20 mg d'une huile jaune.
Rdt (%) = 31.

11.4. (*2S*, *4E*)-5-(5-amino-2-pyridinyl)-2-[(triphényl-méthyl)amino]-4-pentènoate de méthyle.

On porte à 110°C pendant 40 min., un mélange de 1,2 g (2,43 mmol) de (*2S*, *4E*)-5-(5-nitro-2-pyridinyl)-2-[(triphénylméthyl) amino]-4-pentènoate de méthyle, de 477 mg (8,5 mmol) de Fer, de 3,6 ml d'eau, de 7,2 ml d'éthanol et de 720 µl d'acide acétique. Le milieu est filtré sur célite. L'éthanol est évaporé sous pression réduite. Le résidu brut est extrait par un mélange dichlorométhane / isopropanol (75:25). La phase organique est séchée sur sulfate de sodium anhydre puis concentrée sous pression réduite. Le résidu d'évaporation est purifié sur silice par un mélange cyclohexane / acétate d'éthyle (6:4). On obtient 500 mg d'une huile jaune.
Rdt (%) = 41,6.

11.5. Acide (*2S*,*4E*)-2-[(2,2-diméthyl-1-oxopropoxylamino]-5-[5-[2,2-diméthyl-1-oxopropyl)amino]-2-pyridinyl]-4-pentènoate de méthyle.

Une solution de 500 mg (1,08 mmol) de (*2S*,*4E*)-5-(5-amino-2-pyridinyl)-2-[(triphénylméthyl)amino]-4-pentènoate de méthyle dans 3,24 ml d'acide chlorhydrique et 2 ml de tétrahydrofurane est portée à reflux à 65°C pendant 1 h 30. On laisse refroidir le milieu. On additionne de la soude 1N dans le milieu jusqu'à pH basique. On y ajoute 2,35 g (10,8 mmol) de ditertiobutyl carbonate. Le milieu est laissé sous agitation à température ambiante pendant 24 h. On lave le milieu à l'éther diéthylique, puis réacidifie par de l'acide citrique et le brut est extrait par du dichlorométhane. On sèche la phase organique sur sulfate de sodium anhydre puis on évapore à sec. On obtient 300 mg d'une huile jaune.
Rdt (%) = 60.

11.6. *N*-(1*S*,3*E*)-1-[[4-difluorométhylène)-1-pipéridinyl)carbonyl]-4-[5-(2,2-diméthyl-1-oxopropyl)amino]-2-pyridinyl]-3-butènyl]2,2-diméthylpropanamide.

A une solution de 120 mg (0,73 mmol) de 4-difluorométhylène pipéridine dans 8 ml de dichlorométhane et 509 µl de diisopropyléthylamine, refroidie à 0°C, on ajoute 300 mg (0,73 mmol) d'acide (*2S,4E*)-2-[(2,2-diméthyl-1-oxopropylamino]-5-[5-[2,2-diméthyl-1-oxopropyl)-amino]-2-pyridinyl]-4-pentènoate de méthyle puis 257 mg (0,80 mmol) de TBTU. Le milieu est laissé sous agitation à 0°C pendant 2h30. On évapore le dichlorométhane. Le résidu est repris par de l'acétate d'éthyle, lavé par une solution de carbonate de sodium saturé puis à l'eau, séché sur sulfate de sodium anhydre. La phase organique est concentrée sous vide. Le résidu d'évaporation est purifié sur silice par un mélange dichlorométhane / méthanol (99 :1). On obtient 342 mg d'une huile jaune.
Rdt (%) = 89,7.

11.7. Chlorhydrate de *N*-[3-[[[(1S,3E)-4-(5-amino-2-pyridinyl)-1-[[4-difluorométhylène)-1-pipéridinyl] carbonyl]-3-butènyl]amino]sulfonyl][1,1'-biphényl]-2-yl-acétamide

A une solution de 342 mg (0,66 mmol) de *N*-(*1S,3E*)-1-[[4-difluorométhylène)-1-pipéridinyl)carbonyl]-4-[5-(2,2-diméthyl-1-oxopropyl)amino]-2-pyridinyl]-3-butènyl]2,2-diméthylpropanamide dans 4 ml de dichlorométhane, on ajoute 4,6 ml (6,6 mmol) d'acide chlorhydrique 4N dans du dioxane. Le milieu est laissé à température ambiante, sous agitation, pendant 2 h. Le milieu est évaporé à sec. Le résidu d'évaporation est repris par 3 ml de dichlorométhane. On y ajoute 315 µl (2,31 mmol) de triéthylamine. Le milieu est refroidi à 0°C. A cete température, on ajoute 232 mg (0,66 mmol) de chlorure de 2-(diacéthylamino)-[1,1'-biphényl]-3-sulfonyle. On laisse sous agitation revenir à température ambiante pendant 2h. Le milieu est lavé par une solution de chlorure de sodium saturée. La phase organique est séchée sur sulfate de sodium anhydre puis évaporée à sec. Le résidu est repris par 4 ml de tétrahydrofurane et refroidi à 0°C. On fait barboter de l'ammoniac pendant 45 min. à 0°C. On évapore à sec. On purifie le résidu d'évaporation sur silice par un mélange cyclohexane / acétate d'éthyle (2:8), puis sur phase inverse, à l'aide d'un gradient acide chlorhydrique N/100 - acétonitrile de 0 à 100 % d'acétonitrile. On obtient 150 mg du composé attendu.
Rdt (%) = 38 ; F(°C) = 175 ;
$[\alpha]_D^{20}$ (°) = +76 (c = 0,1 ; méthanol).

<u>Exemple 12</u> (composé n° 126) :

Chlorhydrate de (*S*)-*N*-[3-[[[3-(5-amino-2-pyridinyl)-1-[[4-(difluorométhylène)-1-pipéridinyl]carbonyl]propyl]amino] sulfonyl] [1,1'-biphényl]-2-yl]-acétamide.

12.1. (*S*)-4-iodo-2-[[(phénylméthoxy)carbonyl]amino]butanoate de phénylméthyle.

A une solution de 30 g (80 mmoles) de (*L*)-(*N*)-[(phénylméthoxy)carbonyl]glutamate de 1-phénylméthyle dans 800 ml de tétrachlorure de carbone sous argon, on ajoute 14 g (43 mmoles) de di(acétyloxy)iodobenzène et 10,5 g (41 mmoles) d'iode. Le mélange est porté à reflux sous irradiation UV. Après 1 h 30 min on ajoute à nouveau 14 g (43 mmoles) de di(acétyloxy)iodobenzène et 10,5 g d'iode. Après 2 h d'irradiation UV, le mélange est lavé avec une solution d'hydrogénosulfite de sodium à 10% (2 x 300 ml), puis 200 ml d'une solution saturée d'hydrogénocarbonate de sodium, puis 200 ml d'eau. Le mélange est séché sur sulfate de sodium, concentré sous pression réduite et purifié par filtration sur une colonne de gel de silice en éluant avec un mélange cyclohexane/acétate d'éthyle 95/5. On obtient, sous forme d'huile, 12,8 g de (*S*)-4-iodo-2-[[(phénylméthoxy)carbonyl] amino]-butanoate de phénylméthyle.
Rdt (%) = 35.

12.2. (*S*)-5-nitro-α-[[(phénylméthoxy)carbonyl]amino]-2-pyridine-butanoate de phénylméthyle.

Une suspension de 6 g (92 mmoles) de zinc en poudre et de 0,4 ml (4.7 mmoles) de 1,2-dibromoéthane dans 7 ml de N,N-diméthylformamide anhydre est chauffé à 60°C sous agitation et sous argon pendant 45 min. On ajoute ensuite 0,126 ml (1 mmole) de chlorure de triméthylsilyle et le mélange est placé sous forte agitation à température ambiante pendant 30 min. On ajoute alors une solution de 7 g (15,4 mmoles) de (*S*)-4-iodo-2-[[(phénylméthoxy)carbonyl]amino]butanoate de phénylméthyle dans 1 ml de N,N-diméthylformamide anhydre. Après 30 min. à température ambiante, sont ajoutés 0,28 g (0,31 mmoles) de tris(dibenzylidèneacétone)dipalladium (0), 0,38 g (1,24 mmoles) de tri-orthotolylphosphine, 3,78 g (18,5 mmoles) de 2-bromo-5-nitropyridine et 1 ml de N,N-diméthylformamide anhydre et le mélange est placé sous agitation 3 h à température ambiante. Le milieu réactionnel est repris par 200 ml d'acétate d'éthyle et 5 g de charbon actif puis filtré sur un gâteau de célite. Le gâteau est rincé avec 2x100 ml d'acétate d'éthyle et les phases organiques sont réunies, lavées avec 5 x 100 ml d'eau, séchées sur sulfate de sodium et concentrées sous pression réduite. Le résidu est purifié par filtration sur une colonne de gel de silice en éluant avec un gradient cyclohexane:acétate d'éthyle de 0 à 20 % d'acétate d'éthyle. On obtient ainsi, sous forme de mousse jaune, 5,16 g de (*S*)-5-nitro-α-[[(phénylméthoxy)carbonyl]amino]-2-pyridine-butanoate de phénylméthyle.
Rdt (%) = 74.

12.3. Acide (*S*)- α,5-diamino-2-pyridinebutanoïque

Un mélange de 5,16 g (11,5 mmoles) de (*S*)-5-nitro-α-[[(phénylméthoxy)carbonyl]amino]-2-pyridinebutanoate de phénylméthyle, 2 g de charbon actif et 0,77 g de palladium sur charbon actif dans 100 ml de méthanol et 100 ml d'eau est agité pendant 4 jours sous 50 psi d'hydrogène à température ambiante. Le milieu réactionnel est filtré sur un gâteau de célite et le gâteau est rincé avec 3 x 50 ml d'eau bouillante. Les phases aqueuses sont réunies et concentrées sous pression réduites. On obtient ainsi 2,05 g d'acide (*S*)-α,5-diamino-2-pyridinebutanoïque sous forme

de solide blanc.
Rdt (%) = 92 ; F(°C) > 260.

12.4. Acide (*S*)- α,5-bis[[(1,1-diméthyléthoxy)-carbonyl]amino] -2-pyridine butanoïque

A une solution de 0,85 g (4,36 mmoles) d'acide (*S*)- α,5-diamino-2-pyridinebutanoïque dans 50 ml d'eau et 50 ml de 1,1-diméthyléthanol, on ajoute 1,05 ml (9,19 mmoles) d'une solution aqueuse de soude à 35 % et 2 g (9,17 mmoles) de dicarbonate de bis-1,1-diméthyléthyle. La solution est agitée 2 h à température ambiante et on ajoute à nouveau 2 g (9,17 mmoles) de dicarbonate de bis- 1, 1-diméthyléthyle et 1 ml d'une solution aqueuse de soude à 35 %. Après 15 h d'agitation à température ambiante, le milieu réactionnel est dilué avec 100 ml d'une solution aqueuse de soude 1N et 50 ml d'une solution saturée de chlorure de sodium puis lavé avec 2 x 300 ml d'éther. La phase aqueuse est acidifiée avec de l'acide citrique à pH = 4-5 puis extraite avec un mélange dichlorométhane:isopropanol 75 :25 (2x100 ml). Les phases organiques sont réunies et concentrées sous pression réduite. Le résidu est repris dans 100 ml de toluène et à nouveau concentré sous pression réduite. On obtient 1,18 g d'acide (*S*)- α,5-bis[[(1,1-diméthyléthoxy) carbonyl]amino]-2-pyridinebutanoïque sous forme d'huile visqueuse.
Rdt (%) = 69

12.5. (*S*)-1-[[4-(difluorométhylène)-1-pipéridinyl]carbonyl]-3-[5-[[(1,1-diméthyléthoxy)carbony]amino]-2-pyridinyl]propyl]-carbamate de 1,1-diméthyléthyle.

A un mélange de 1,18 g (3 mmoles) d'acide (*S*) - α,5-bis[[(1,1-diméthyléthoxy)carbonyl]amino]-2-pyridinebutanoïque, de 1,15 ml (6,6 mmoles) de *N,N*-diisopropyléthylamine et de 0,56 g (3,3 mmoles) de chlorhydrate de 4-difluorométhylène-1-pipéridine dans 30 ml de dichlorométhane, on ajoute à 0°C sous argon et sous agitation 1,16 g (3,6 mmoles) de tétrafluoroborate de O-(benzotriazol-1-yl)-N,N,N',N'-tétraméthyluronium (TBTU). On laisse revenir à température ambiante et on poursuit l'agitation pendant 2 h. Le milieu réactionnel est dilué avec 50 ml de dichlorométhane et lavé avec 50 ml d'eau puis 50 ml d'une solution saturée d'hydrogénocarbonate de sodium et à nouveau avec 2x50 ml d'eau. La phase organique est séchée sur sulfate de sodium, concentrée sous pression réduite et le résidu est purifié par chromatographie sur une colonne de gel de silice en éluant avec un mélange cyclohexane:acétate d'éthyle 1:1. On obtient sous forme d'huile visqueuse, 1,62 g de (*S*)-1-[[4-(difluorométhylène)-1-pipéridinyl]carbonyl]-3-[5-[[(1,1-diméthyléthoxy)carbony]amino]-2-pyridinyl]propyl]-carbamate de 1,1-diméthyléthyle.
Rdt (%) = 100

12.6. Chlorhydrate de (*S*)-5-amino-α-[[4-(difluorométhylène)-1-pipéridinyl]carbonyl]-2-pyridinepropanamine.

Une solution de 1,62 g (3,18 mmoles) de (*S*)-1-[[4-(difluorométhylène)-1-pipéridinyl]carbonyl]-3-[5-[[(1,1-diméthyléthoxy)carbonyl]amino]-2-pyridinyl]propyl]-carbamate de 1,1-diméthyléthyle dans 50 ml de dichlorométhane est traitée pendant 5 min. à 0°C par un courant de chlorure d'hydrogène. On laisse la solution revenir à température ambiante et l'agitation est maintenue pendant 3h. Le précipité blanc obtenu est ensuite filtré et rincé avec 10 ml de dichlorométhane. On obtient ainsi 1 g de chlorhydrate de (*S*)-5-amino-α-[[4-(difluorométhylène)-1-pipéridinyl]carbonyl]-2-pyridinepropanamine (2 :1) très hygroscopique et utilisé tel quel à l'étape suivante.
Rdt (%) = 88 ; F(°C) = 70

12.7. Chlorhydrate de (*S*)-N-[3-[[[3-(5-amino-2-pyridinyl)-1-[[4-(difluorométhylène)-1-pipéridinyl]carbonyl]propyl] amino]sulfonyl] [1,1'-biphényl]-2-yl]-acétamide

A une solution de 0,7 g (1,83 mmoles) de chlorhydrate de (*S*)-5-amino-α-[[4-(difluorométhylène)1-pipéridinyl] carbonyl]-2-pyridinepropanamine (2 :1) dans 20 ml de dichlorométhane, on ajoute 0,8 ml (5,7 mmoles) de triéthylamine et on refroidit à 0°C sous agitation. On ajoute alors 0,58 g (1,64 mmoles) de chlorure de 2-(diacétylamino)-[1,1' biphényl]-3-sulfonyle par petites quantités. Après 2 h à 0°C, on ajoute 50 ml de dichlorométhane et la solution est lavée successivement avec 2x20 ml d'eau, 50 ml d'une solution saturée d'hydrogénocarbonate de sodium et 2x10 ml d'eau. La phase organique séchée sur sulfate de sodium est concentrée sous pression réduite. Le résidu obtenu est repris dans 20 ml de tétrahydrofurane puis est refroidi à 0°C et est traité pendant 5 min. par un courant d'ammoniac. On laisse le mélange réactionnel revenir à température ambiante puis, après 4 h, on le concentre sous pression réduite. Le résidu obtenu est purifié par chromatographie sur une colonne de gel de silice en éluant sous pression avec un mélange dichlorométhane:méthanol 99:1. Après concentration sous pression réduite, le résidu est ensuite repris dans 1,8 ml d'une solution 1M de chlorure d'hydrogène dans le méthanol (1,8 mmoles) est à nouveau concentré sous pression réduite puis est purifié par chromatographie sur une colonne RP18 en éluant par un gradient acétonitrile :acide chlorhydrique N/1000 de 5:95 à 100:0 en 60 min. On obtient après lyophilisation 0,61 g de chlorhydrate de (S)-N-[3-[[[3-(5-amino-2-pyridinyl)-1-[[4-(difluorométhylène)-1-pipéridinyl] carbonyl]propyl]amino]sulfonyl][1,1'-biphényl]-2-yl]-acétamide sous forme de poudre blanche.
Rdt (%) = 60 ; F(°C) = 184-186.
$[\alpha]^{20}_{D}$ (°) = +118 (c = 0,23 ; méthanol).

Légende des tableaux qui suivent :

- dans la colonne "sel", "HCl" correspond à un chlorhydrate et le rapport entre parenthèse est le rapport (acide:base),
- dans la colonne "$[\alpha]^{20}_{D}$", c = 0,2 ; méthanol sauf pour les composés n°9 et n°11 (c = 0,4 ; méthanol).

Tableau 1

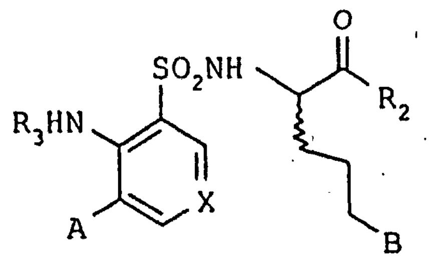

$(I_1)$

| N° | $R_3$ | X | A | $R_2$ | B | Sel | Point de fusion (°C) | $[\alpha]_D^{20}$ (°) |
|---|---|---|---|---|---|---|---|---|
| 1 | $-COCH_2CH_3$ | CH | 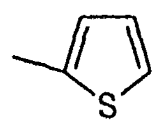 | 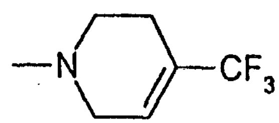 | 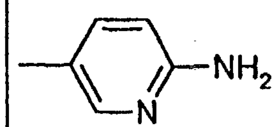 | HCl (1:1) | 155-159 | + 89 |
| 2 | $-COCH_2CH_3$ | CH | 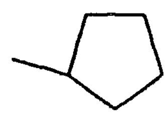 | 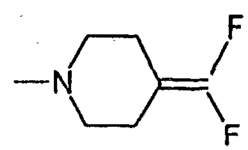 | 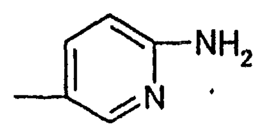 | HCl (1:1) | 128-133 | + 86 |
| 3 | $-COCH_2CH_3$ | CH | 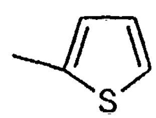 | 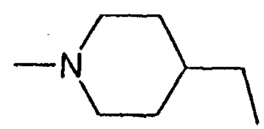 | 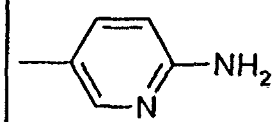 | HCl (1:1) | 144-148 | +120 |

| N° | $R_3$ | X | A | $R_2$ | B | Sel | Point de fusion (°C) | $[\alpha]_D^{20}$ (°) |
|---|---|---|---|---|---|---|---|---|
| 4 | $-COCH_2CH_3$ | CH | 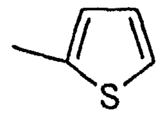 | 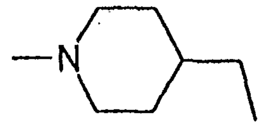 | 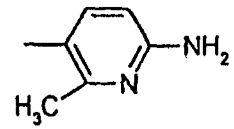 | HCl (1:1) | 145-150 | + 110 |
| 5 | $-COCH_2CH_3$ | CH | 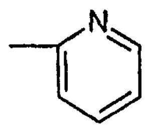 | 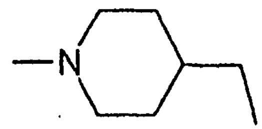 | 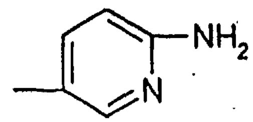 | HCl (2:1) | 145-150 | + 138 |
| 6 | $-COCH_2CH_3$ | CH | 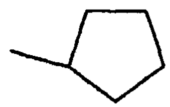 | 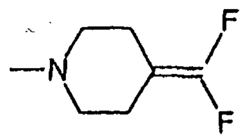 | 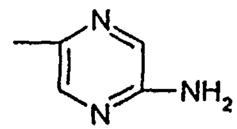 | HCl (1:1) | 136-140 | + 103 |
| 7 | $-COCH_2CH_3$ | CH | 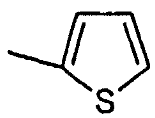 | 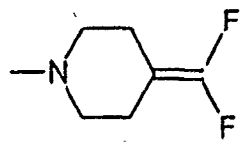 | 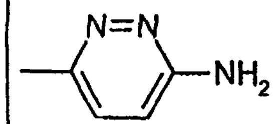 | HCl (1:1) | 140-144 | + 60 |
| 8 | $-COCH_2CH_3$ | CH | 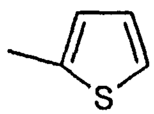 | 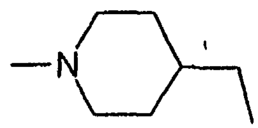 | 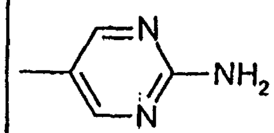 | HCl (1:1) | 122-126 | + 83 |

| N° | $R_3$ | X | A | $R_2$ | B | Sel | Point de fusion (°C) | $[\alpha]_D^{20}$ (°) |
|---|---|---|---|---|---|---|---|---|
| 9 | $-COCH_2CH_3$ | CH | 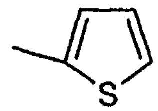 | 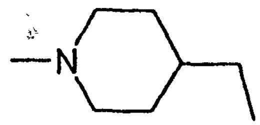 | 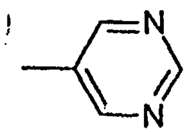 | HCl (1:1) | 112 | + 114 |
| 10 | $-COCH_2CH_3$ | CH | 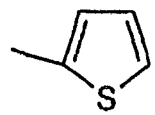 | 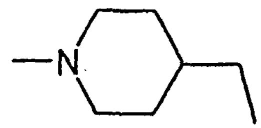 | 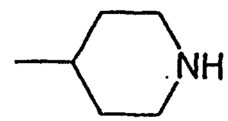 | HCl (1:1) | 134-138 | + 112 |
| 11 | $-COCH_2CH_3$ | CH | 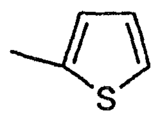 | 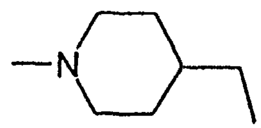 | 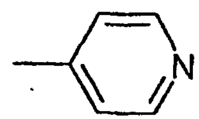 | HCl (1:1) | 116 | + 102 |
| 12 | $-COCH_2CH_3$ | CH | 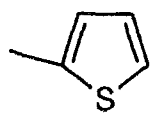 | 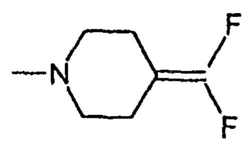 | 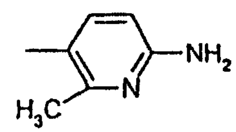 | HCl (1:1) | 144-148 | + 102 |
| 13 | $-COCH_2CH_3$ | CH | 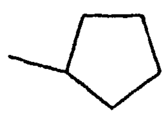 | 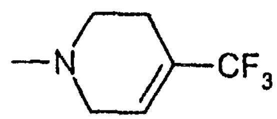 | 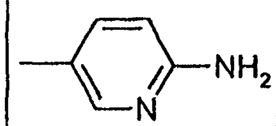 | HCl (1:1) | 158-162 | + 80 |

| N° | $R_3$ | X | A | $R_2$ | B | Sel | Point de fusion (°C) | $[\alpha]_D^{20}$ (°) |
|---|---|---|---|---|---|---|---|---|
| 14 | $-COCH_2CH_3$ | CH | 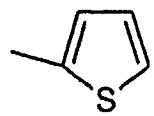 | 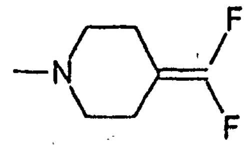 | 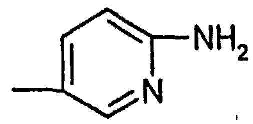 | HCl (1:1) | 135-140 | + 108 |
| 15 | $-COCH_2CH_3$ | CH | 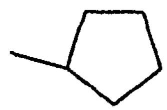 | 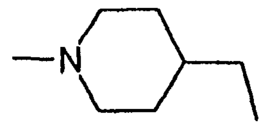 | 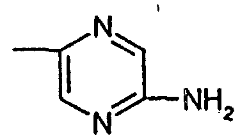 | HCl (1:1) | 142-146 | + 103 |
| 16 | $-COCH_2CH_3$ | CH | 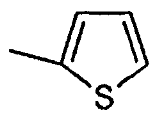 | 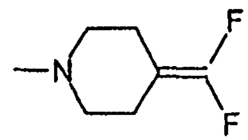 | 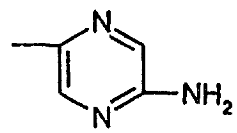 | - | 154-158 | +123 |
| 17 | $-COCH_2CH_3$ | CH | 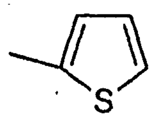 | 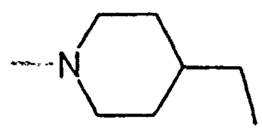 | 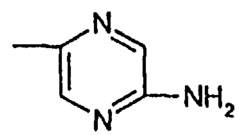 | HCl (1:1) | 146-150 | + 139 |
| 18 | $-COCH_2CH_3$ | CH | 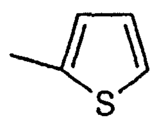 | 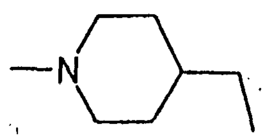 | 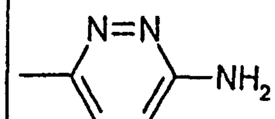 | HCl (1:1) | 143-147 | + 62 |

| N° | $R_3$ | X | A | $R_2$ | B | Sel | Point de fusion (°C) | $[\alpha]_D^{20}$ (°) |
|---|---|---|---|---|---|---|---|---|
| 19 | $-COCH_2CH_3$ | CH | 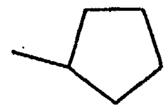 | 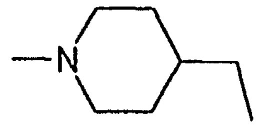 | 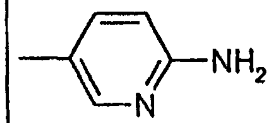 | HCl (1:1) | 132-136 | + 93 |
| 20 | $-COCH_2CH_3$ | CH | 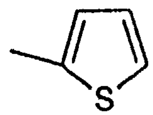 | 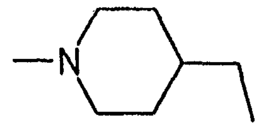 | 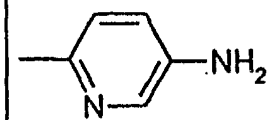 | HCl (1:1) | 142-146 | + 111 |
| 21 | $-COCH_2CH_3$ | CH | 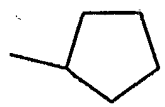 | 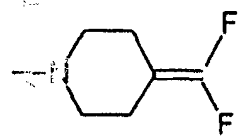 | 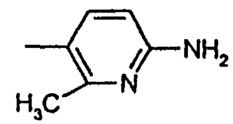 | HCl (1:1) | 145-149 | + 128 |
| 22 | $-COCH_2CH_3$ | CH | 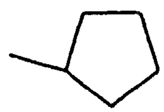 | 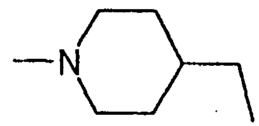 | 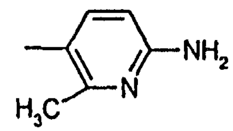 | HCl (1:1) | 140-144 | + 97 |
| 23 | $-COCH_2CH_3$ | CH | 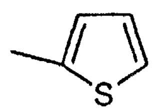 | 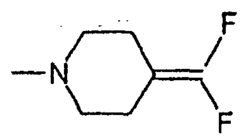 | 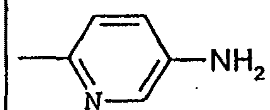 | HCl (1:1) | 152-156 | + 82 |
| 24 | $-COCH_2CH_3$ | CH | 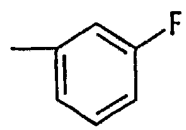 | 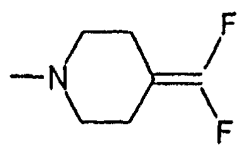 | 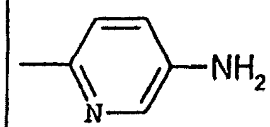 | HCl (1:1) | 136-140 | + 90 |

| N° | $R_3$ | X | A | $R_2$ | B | Sel | Point de fusion (°C) | $[\alpha]_D^{20}$ (°) |
|---|---|---|---|---|---|---|---|---|
| 25 | $-COCH_2CH_3$ | CH | 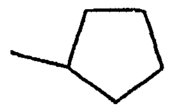 | 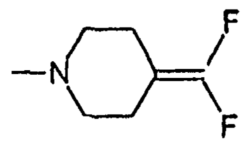 | 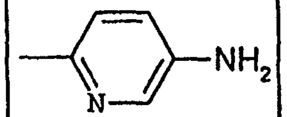 | HCl (1:1) | 146-150 | + 76 |
| 26 | $-COCH_2CH_3$ | CH | 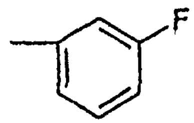 | 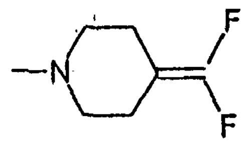 | 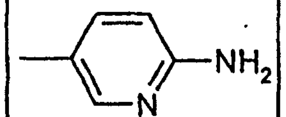 | HCl (1:1) | 146-150 | + 101 |
| 27 | $-COCH_2CH_3$ | CH | 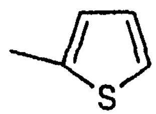 | 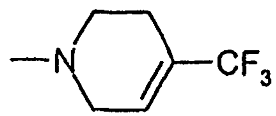 | 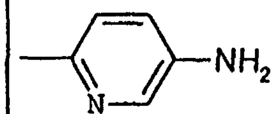 | HCl (1:1) | 142-146 | + 65 |
| 28 | $-COCH_2CH_3$ | CH | 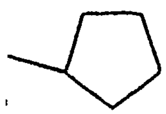 | 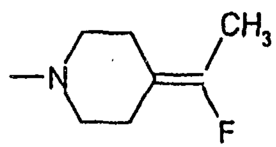 | 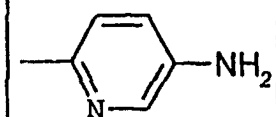 | HCl (1:1) | 150-154 | + 66 |
| 29 | $-COCH_2CH_3$ | CH | 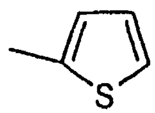 | 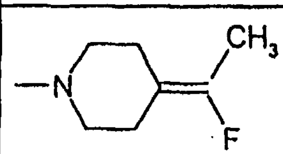 | 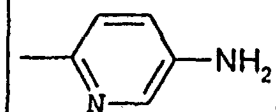 | HCl (1:1) | 160-164 | + 69 |

| N° | $R_3$ | X | A | $R_2$ | B | Sel | Point de fusion (°C) | $[\alpha]_D^{20}$ (°) |
|---|---|---|---|---|---|---|---|---|
| 30 | $-COCH_2CH_3$ | CH | 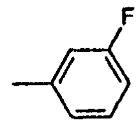 | 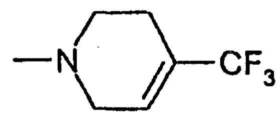 | 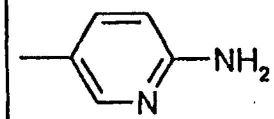 | HCl (1:1) | 150-154 | +72 |
| 31 | $-COCH_2CH_3$ | CH | 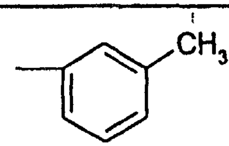 | 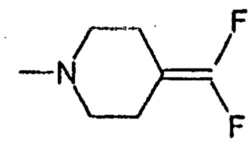 | 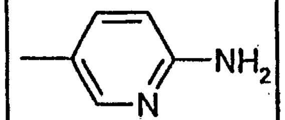 | HCl (1:1) | 149-152 | +118 |
| 32 | $-COCH_2CH_3$ | CH | 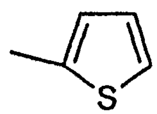 | 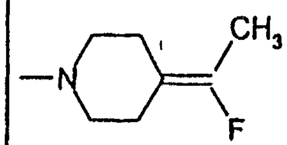 | 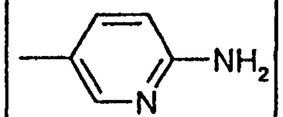 | HCl (1:1) | 146-150 | 155 |
| 33 | $-COCH_2CH_3$ | CH | 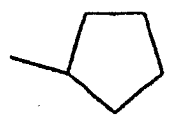 | 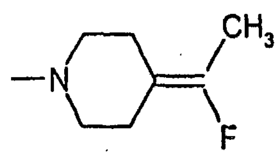 | 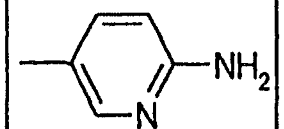 | HCl (1:1) | 143-147 | +56 |
| 34 | $-COCH_2CH_3$ | CH | 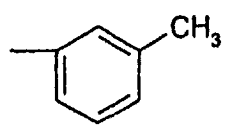 | 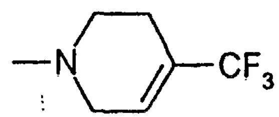 | 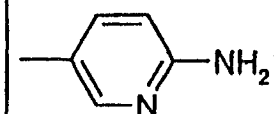 | HCl (1:1) | 144-148 | +95 |

| N° | $R_3$ | X | A | $R_2$ | B | Sel | Point de fusion (°C) | $[\alpha]_D^{20}$ (°) |
|---|---|---|---|---|---|---|---|---|
| 35 | $-COCH_2CH_3$ | CH | 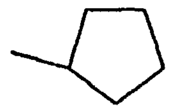 | 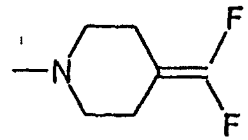 | 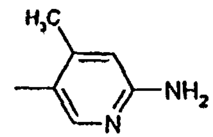 | HCl (1:1) | 146-150 | +77 |
| 36 | $-COCH_2CH_3$ | CH | 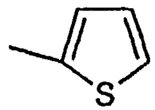 | 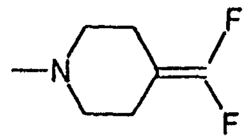 | 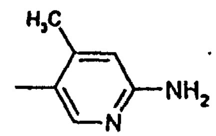 | HCl (1:1) | 147-151 | +122 |
| 37 | $-COCH_2CH_3$ | CH | 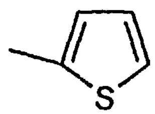 | 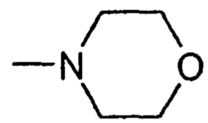 | 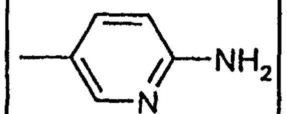 | HCl (1:1) | 159-163 | +114 |
| 38 | $-COCH_2CH_3$ | CH | 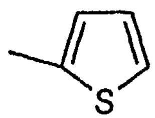 | 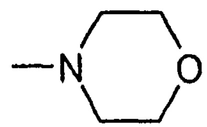 | 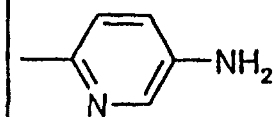 | HCl (1:1) | 150-156 | +65 |
| 39 | $-COCH_3$ | CH | 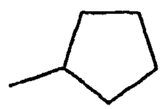 | 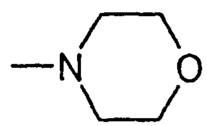 | 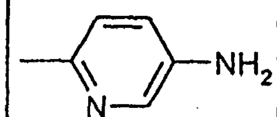 | HCl (1:1) | 188-192 | +64 |

| N° | $R_3$ | X | A | $R_2$ | B | Sel | Point de fusion (°C) | $[\alpha]_D^{20}$ (°) |
|---|---|---|---|---|---|---|---|---|
| 40 | $-COCH_2CH_3$ | CH | 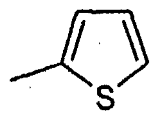 | 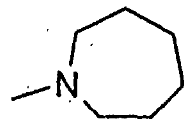 | 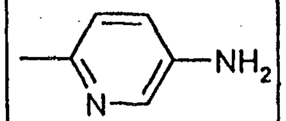 | HCl (1:1) | 153-157 | +74 |
| 41 | $-COCH_2CH_3$ | CH | 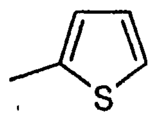 | 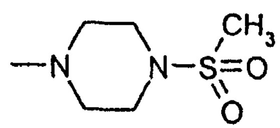 | 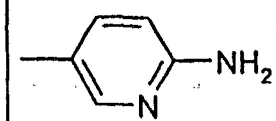 | HCl (1:1) | 166-170 | +95 |
| 42 | $-COCH_2CH_3$ | CH | 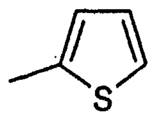 | 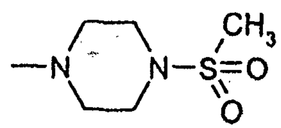 | 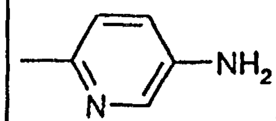 | HCl (1:1) | 172-176 | +62 |
| 43 | $-COCH_3$ | CH | 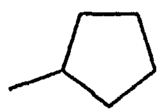 | 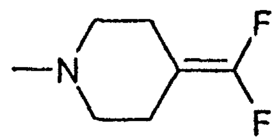 | 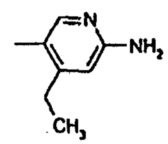 | HCl (1:1) | 138-142 | +85 |
| 44 | $-COCH_2CH_3$ | CH | 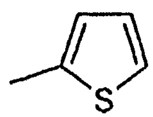 | 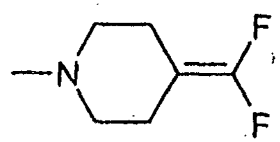 | 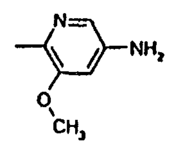 | HCl (1:1) | 157-161 | +102 |
| 45 | $-COCH_2CH_3$ | CH | 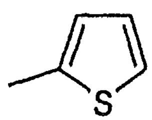 | 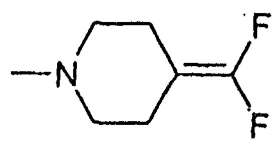 | 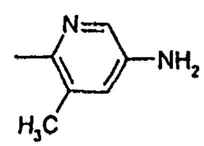 | HCl (1:1) | 156-160 | +86 |

| N° | $R_3$ | X | A | $R_2$ | B | Sel | Point de fusion (°C) | $[\alpha]_D^{20}$ (°) |
|---|---|---|---|---|---|---|---|---|
| 46 | $-COCH_2CH_3$ | CH | 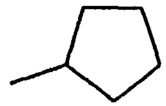 | 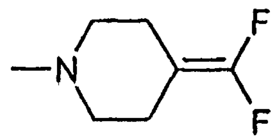 | 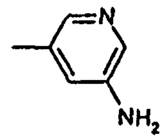 | HCl (1:1) | 163-167 | +70 |
| 47 | $-COCH_3$ | CH | 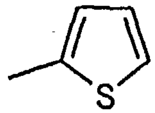 | 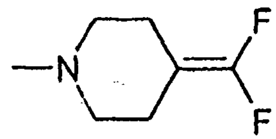 | 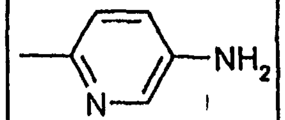 | HCl (1:1) | 152-156 | +92 |
| 48 | $-COCH_3$ | CH | 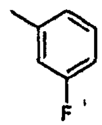 | 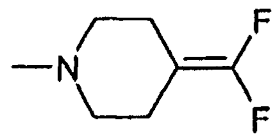 | 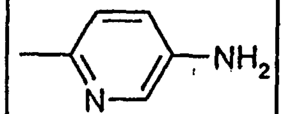 | HCl (1:1) | 154-158 | +91 |
| 49 | $-COCH_2CH_3$ | CH | 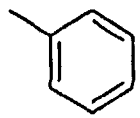 | 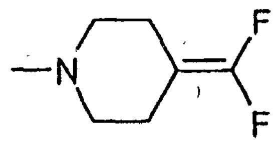 | 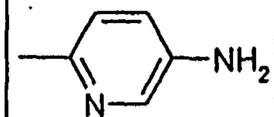 | HCl (1:1) | 154-158 | +78 |
| 50 | $-COCH_3$ | CH | 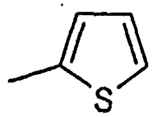 | 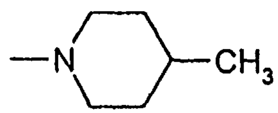 | 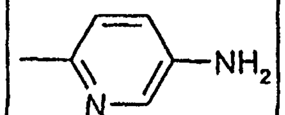 | HCl (1:1) | 156-160 | +91 |
| 51 | $-COCH_3$ | CH | 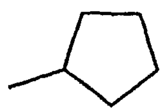 | 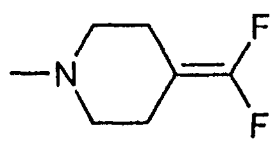 | 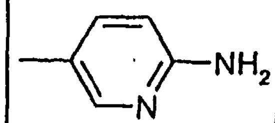 | HCl (1:1) | 148-152 | +79 |

| N° | $R_3$ | X | A | $R_2$ | B | Sel | Point de fusion (°C) | $[\alpha]_D^{20}$ (°) |
|---|---|---|---|---|---|---|---|---|
| 52 | $-COCH_3$ | CH | 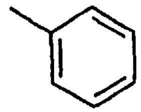 | 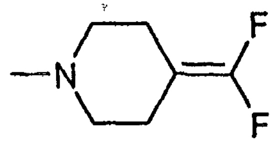 | 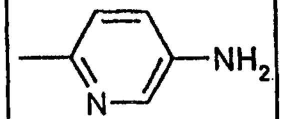 | HCl (1:1) | 170-174 | +90 |
| 53 | $-COCH_2CH_3$ | CF | 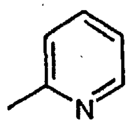 | 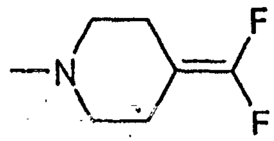 | 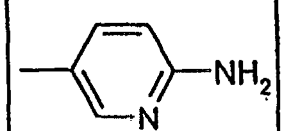 | HCl (1:1) | 150-154 | +88 |
| 54 | $-COCH_3$ | CH | 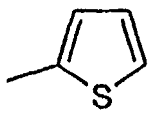 | 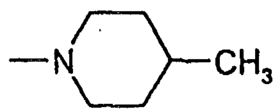 | 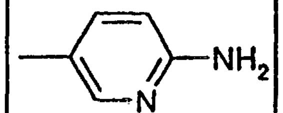 | HCl (1:1) | 170-172 | +132 |
| 55 | $-COCH_3$ | CH | 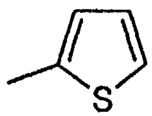 | 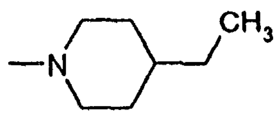 | 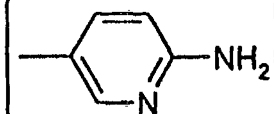 | HCl (1:1) | 166-168 | +122 |
| 56 | $-COCH_3$ | CH | 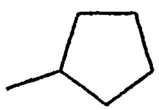 | 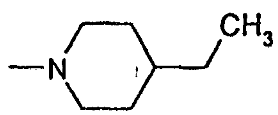 | 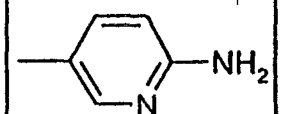 | HCl (1:1) | 160-162 | +82 |
| 57 | $-COCH_2CH_3$ | CF | 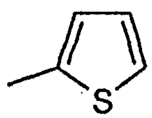 | 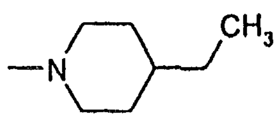 | 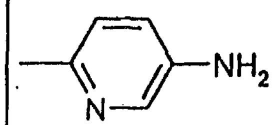 | HCl (1:1) | 150-154 | +39 |

| N° | $R_3$ | X | A | $R_2$ | B | Sel | Point de fusion (°C) | $[\alpha]_D^{20}$ (°) |
|---|---|---|---|---|---|---|---|---|
| 58 | $-COCH_2CH_3$ | CF | 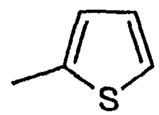 | 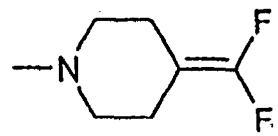 | 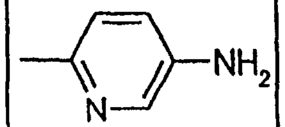 | HCl (1:1) | 148-152 | +90 |
| 59 | $-COCH_3$ | CH | 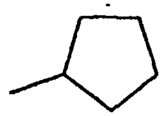 | 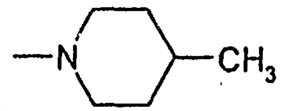 | 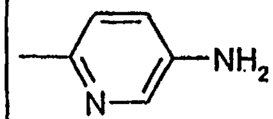 | HCl (1:1) | 148-152 | +77 |
| 60 | $-COCH_2CH_3$ | CH | 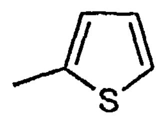 | 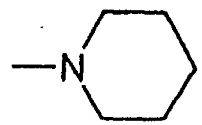 | 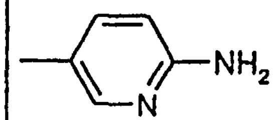 | HCl (1:1) | 154-158 | +106 |
| 61 | $-COCH_2CH_3$ | CH | 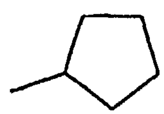 | 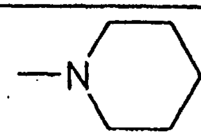 | 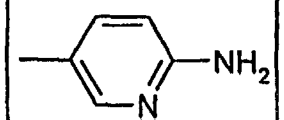 | HCl (1:1) | 144-148 | +91 |
| 62 | $-COCH_2CH_3$ | CH | 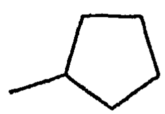 | 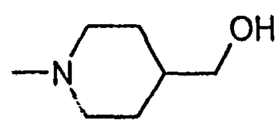 | 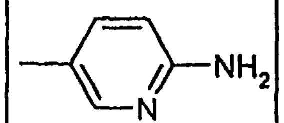 | HCl (1:1) | 158 | +78 |
| 63 | $-COCH_3$ | CH | 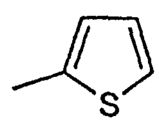 | 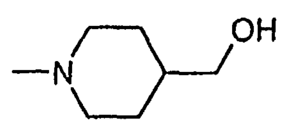 | 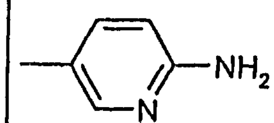 | HCl (1:1) | 162 | +110 |

| N° | $R_3$ | X | A | $R_2$ | B | Sel | Point de fusion (°C) | $[\alpha]_D^{20}$ (°) |
|---|---|---|---|---|---|---|---|---|
| 64 | $-COCH_2CH_3$ | CH | 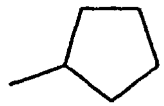 | 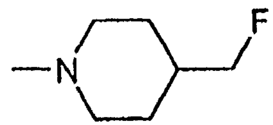 | 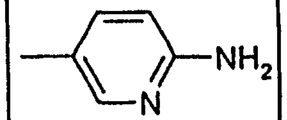 | HCl (1:1) | 148 | +86 |
| 65 | $-COCH_3$ | CH | 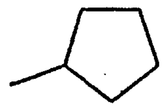 | 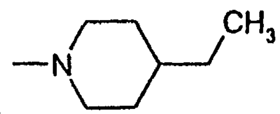 | 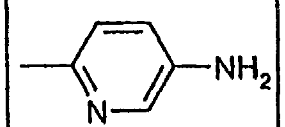 | HCl (1:1) | 148-152 | +81 |
| 66 | $-COCH_2CH_3$ | CH | 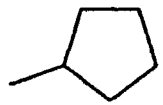 | 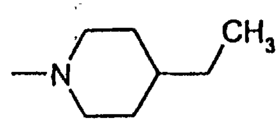 | 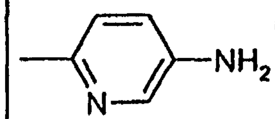 | HCl (1:1) | 146-150 | +67 |
| 67 | $-COCH_2CH_2CH_3$ | CH | 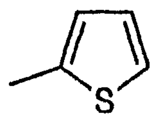 | 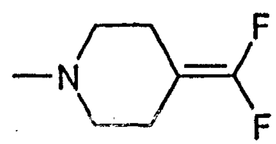 | 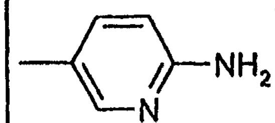 | HCl (1:1) | 140-144 | +102 |
| 68 | $-COCH_3$ | CH | 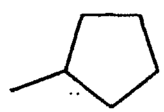 | 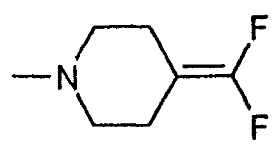 | 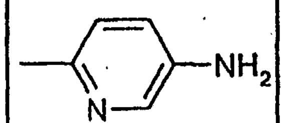 | HCl (1:1) | 150-154 | +59 |
| 69 | $-COCH_2CH_3$ | CH | 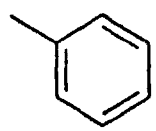 | 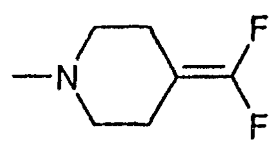 | 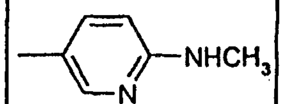 | HCl (1:1) | 154-155 | + 85 |

| N° | $R_3$ | X | A | $R_2$ | B | Sel | Point de fusion (°C) | $[\alpha]_D^{20}$ (°) |
|---|---|---|---|---|---|---|---|---|
| 70 | $-COCH_2CH_3$ | CH | 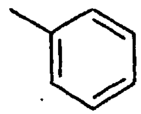 | 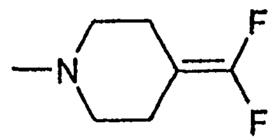 | 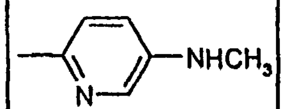 | HCl | 139-140 | + 100 |
| 71 | $-COCH_3$ | CH | 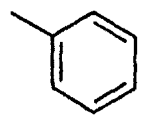 | 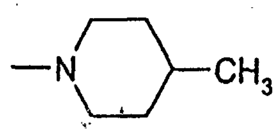 | 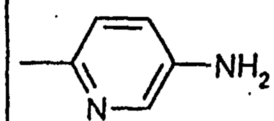 | HCl | 176-180 | + 84 |
| 72 | $-COCH_2CH_3$ | CH | 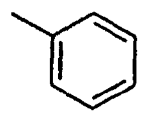 | 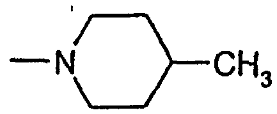 | 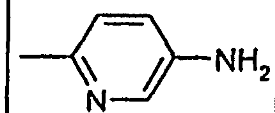 | HCl | 178-182 | + 71 |

| N° | $R_3$ | X | A | $R_2$ | B | Sel | Point de fusion (°C) | $[\alpha]_D^{20}$ (°) |
|---|---|---|---|---|---|---|---|---|
| 73 | $-COCH_2CH_3$ | CH | 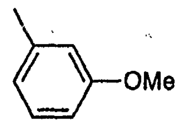 | 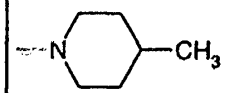 | 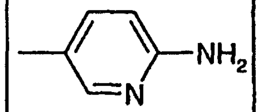 | HCl | 138-139 | +155 |
| 74 | $-COCH_3$ | CH | 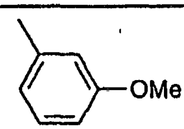 | 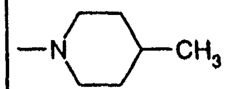 | 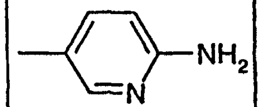 | HCl | 145-146 | +116 |
| 75 | $-COCH_2CH_3$ | CH | 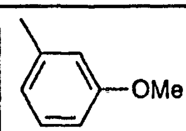 | 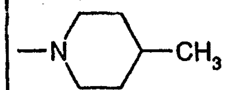 | 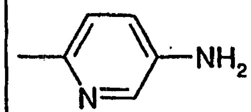 | HCl | 146-147 | +62 |
| 76 | $-COCH_3$ | CH | 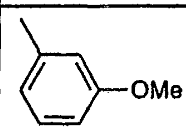 | 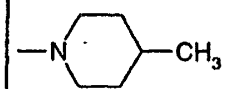 | 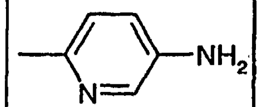 | HCl | 156-157 | +84 |
| 77 | $-COCH_3$ | CH | 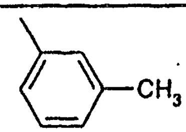 | 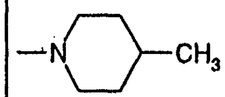 | 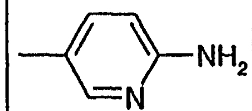 | HCl | 147-148 | +136 |
| 78 | $-COCH_3$ | CH | 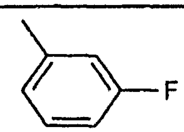 | 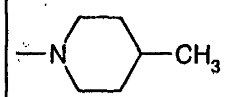 | 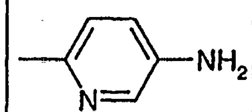 | HCl | 168 | +83 |
| 79 | $-COCH_3$ | CH | 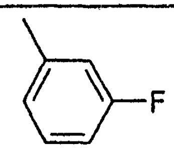 | 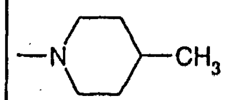 | 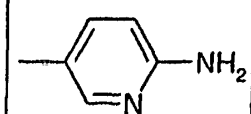 | HCl | 164 | +63 |

| N° | $R_3$ | X | A | $R_2$ | B | Sel | Point de fusion (°C) | $[\alpha]^{20}_{D}$ (°) |
|---|---|---|---|---|---|---|---|---|
| 80 | $-COCH_2CH_3$ | CH | 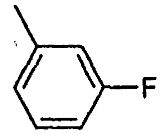 | 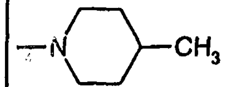 | 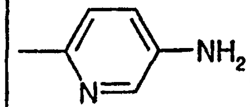 | HCl | 152 | +112 |
| 81 | $-COCH_2CH_3$ | CH | 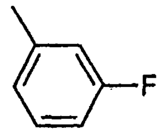 | 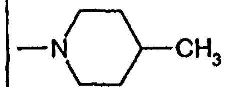 | 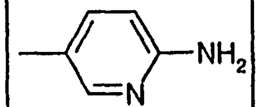 | HCl | 160 | +107 |
| 82 | $-COCH_2CH_3$ | CH | 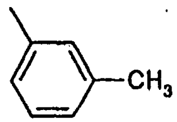 | 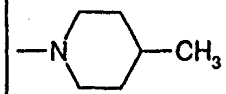 | 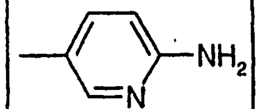 | HCl | 155-157 | +130 |
| 83 | $-COCH_3$ | CH | 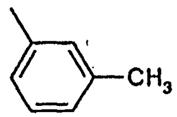 | 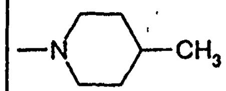 | 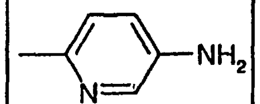 | HCl | 159-161 | +106 |
| 84 | $-COCH_2CH_3$ | CH | 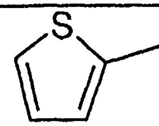 | 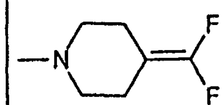 | 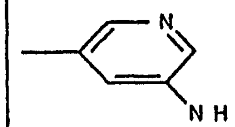 | HCl | 160-164 | +105 |
| 85 | $-COCH_2CH_3$ | CH | 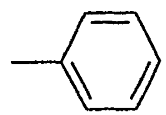 | 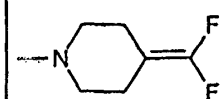 | 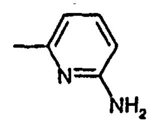 | HCl | 180-184 | +88 |
| 86 | $-COCH_2CH_3$ | CH | 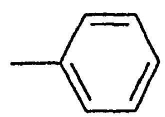 | 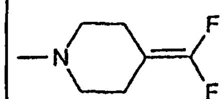 | 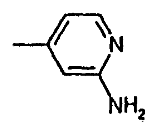 | HCl | 230-234 | +100 |

| N° | $R_3$ | X | A | $R_2$ | B | Sel | Point de fusion (°C) | $[\alpha]_D^{20}$ (°) |
|---|---|---|---|---|---|---|---|---|
| 87 | $-COCH_2CH_3$ | CH | 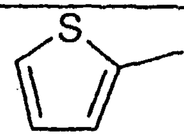 | 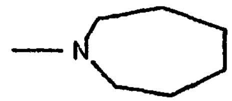 | 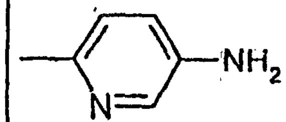 | HCl | 153-157 | +74 |
| 88 | $-COCH_2CH_3$ | CH | 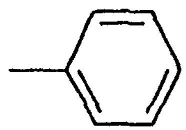 | 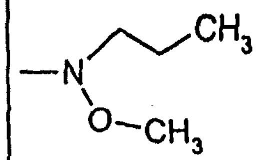 | 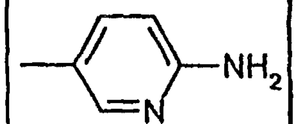 | HCl | 144-148 | +77 |
| 89 | $-COCH_2CH_3$ | CH | 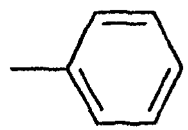 | 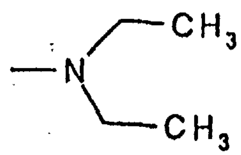 | 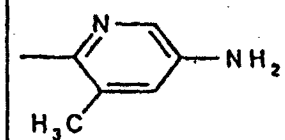 | HCl | 160-164 | +66 |
| 90 | $-COCH_2CH_3$ | CH | 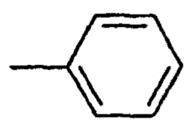 | 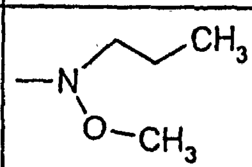 | 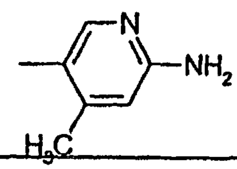 | HCl | 150-154 | +87 |

| N° | $R_3$ | X | A | $R_2$ | B | Sel | Point de fusion (°C) | $[\alpha]_D^{20}$ (°) |
|---|---|---|---|---|---|---|---|---|
| 91 | $-COCH_2CH_3$ | CBr | 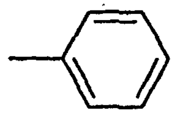 | 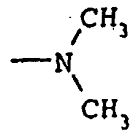 | 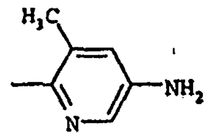 | HCl | 166-170 | +76 |
| 92 | $-COCH_2CH_3$ | CH | 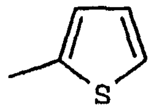 | 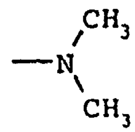 | 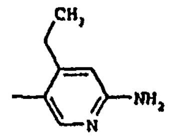 | HCl | 160-164 | +100 |
| 93 | $-COCH_2CH_3$ | CH | 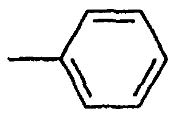 | 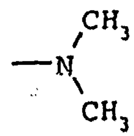 | 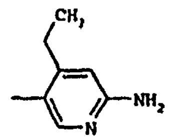 | HCl | 152-156 | +82 |
| 94 | $-COCH_3$ | CH | 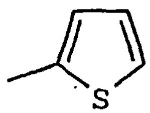 | 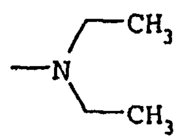 | 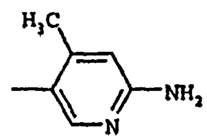 | HCl | 162-166 | +101 |
| 95 | $-COCH_3$ | CH | 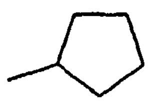 | 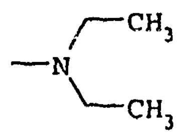 | 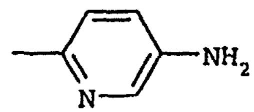 | HCl | 148-152 | +57 |
| 96 | $-COCH_3$ | CH | 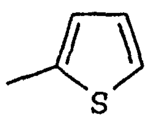 | 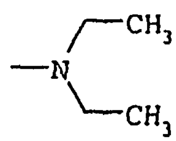 | 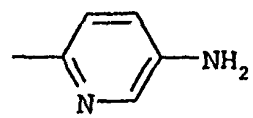 | HCl | 145-149 | +64 |

| N° | $R_3$ | X | A | $R_2$ | B | Sel | Point de fusion (°C) | $[\alpha]_D^{20}$ (°) |
|---|---|---|---|---|---|---|---|---|
| 97 | $-COCH_3$ | CBr | 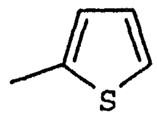 | 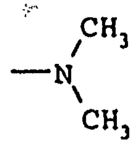 | 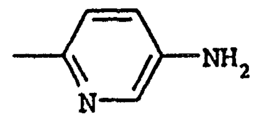 | HCl | 168-172 | +50 |
| 98 | $-COCH_3$ | CH | 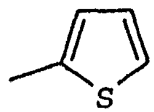 | 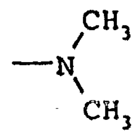 | 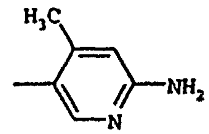 | HCl | 153-158 | +191 |
| 99 | $-COCH_2CH_3$ | CH | 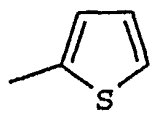 | 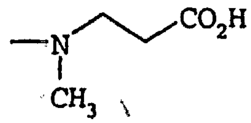 | 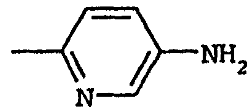 | HCl | 72-76 | +50 |
| 100 | $-COCH_2CH_3$ | CH | 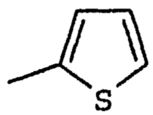 | 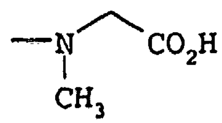 | 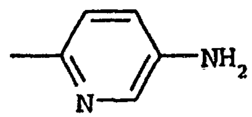 | HCl | 145-149 | +47 |
| 101 | $-COCH_2CH_3$ | CH | 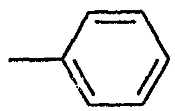 | 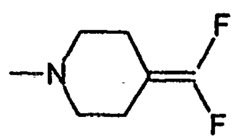 | 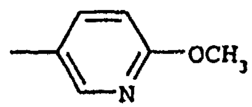 | - | 139 | +127 |
| 102 | $-COCH_2CH_3$ | CH | 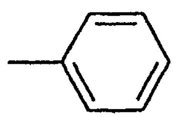 | 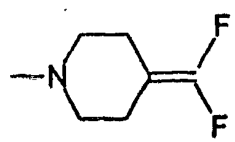 | 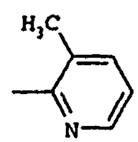 | HCl | 134 | +114 |

| N° | $R_3$ | X | A | $R_2$ | B | Sel | Point de fusion (°C) | $[\alpha]_D^{20}$ (°) |
|---|---|---|---|---|---|---|---|---|
| 103 | $-COCH_2CH_3$ | CH | 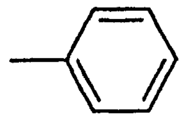 | 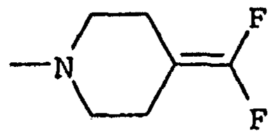 | 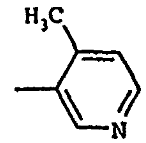 | HCl | 137 | +122 |
| 104 | $-COCH_2CH_3$ | CH | 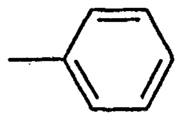 | 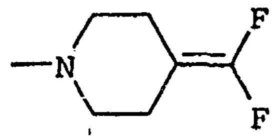 | 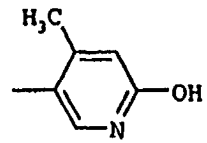 | - | 139 | +132 |
| 105 | $-COCH_2CH_3$ | CH | 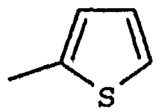 | 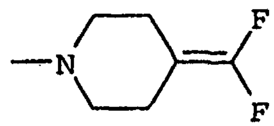 | 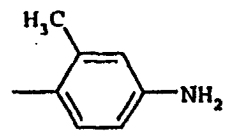 | HCl | 173-177 | +108 |
| 106 | $-COCH_2CH_3$ | CH | 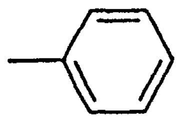 | 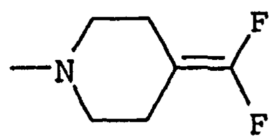 | 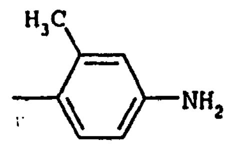 | HCl | 170-174 | +129 |
| 107 | $-COCH_2CH_3$ | CH | 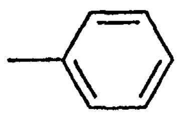 | 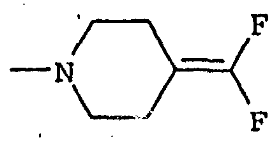 | 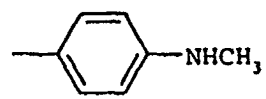 | HCl | 160-164 | +124 |
| 108 | $-COCH_2CH_3$ | CH | 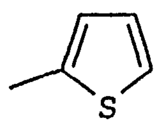 | 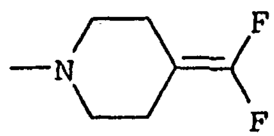 | 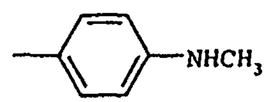 | HCl | 162-164 | +120 |

| N° | $R_3$ | X | A | $R_2$ | B | Sel | Point de fusion (°C) | $[\alpha]_D^{20}$ (°) |
|---|---|---|---|---|---|---|---|---|
| 109 | $-COCH_2CH_3$ | CH | 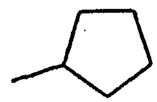 | 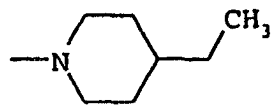 | 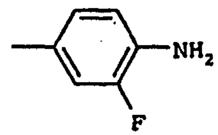 | HCl | 142-146 | +87 |
| 110 | $-COCH_2CH_3$ | CH | 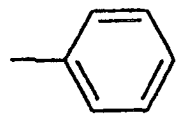 | 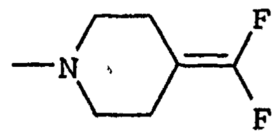 | 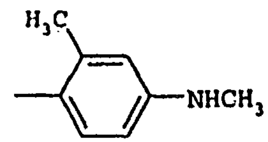 | HCl | 162-166 | +116 |
| 111 | $-COCH_2CH_3$ | CH | 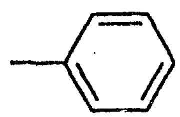 | 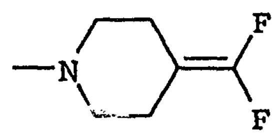 | 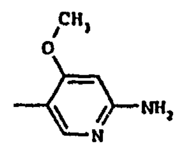 | HCl | 165-169 | +94,5 |
| 112 | $-COCH_2CH_3$ | CH | 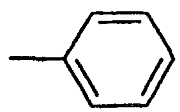 | 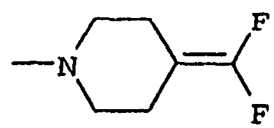 | 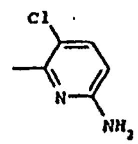 | HCl | 156-160 | +83 |
| 113 | $-COCH_2CH_3$ | CH | 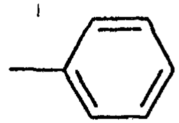 | 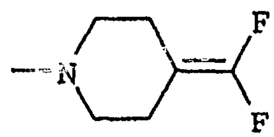 | 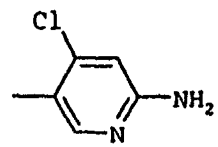 | HCl | 158-162 | +109 |
| 114 | $-COCH_2CH_3$ | N | 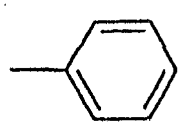 | 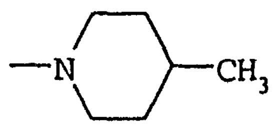 | 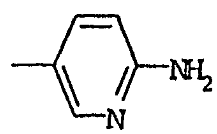 | HCl | 180-184 | +100 |

| N° | $R_3$ | X | A | $R_2$ | B | Sel | Point de fusion (°C) | $[\alpha]_D^{20}$ (°) |
|---|---|---|---|---|---|---|---|---|
| 115 | $-COCH_2CH_3$ | N | 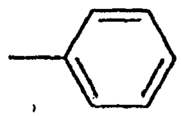 | 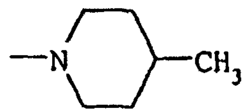 | 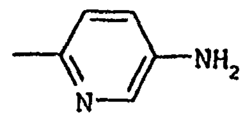 | HCl | 178-182 | +85 |
| 116 | $-COCH_2CH_3$ | N | 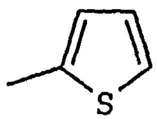 | 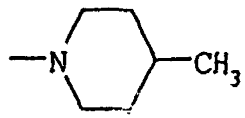 | 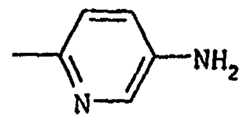 | HCl (2:1) | 190-194 | +73 |
| 117 | $-COCH_2CH_3$ | N | 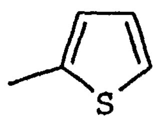 | 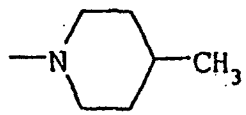 | 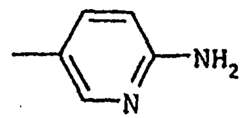 | HCl (1.5:1) | 180-184 | +91 |
| 118 | $-CHO$ | CH | 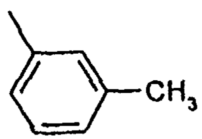 | 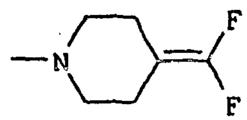 | 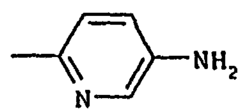 | HCl | 168 | +108 |

Tableau 2

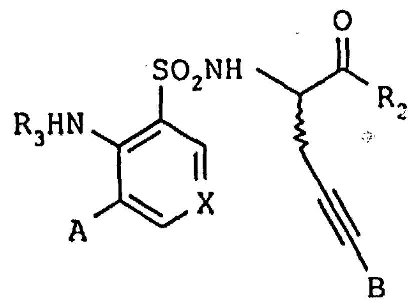

(I

| N° | $R_3$ | X | A | $R_2$ | B | Sel | Point de fusion (°C) | $[\alpha]_D^{20}$ (°) |
|---|---|---|---|---|---|---|---|---|
| 119 | $-COCH_3$ | CH | 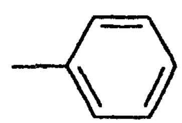 | 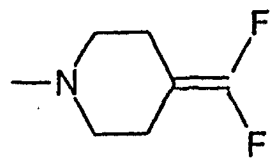 | 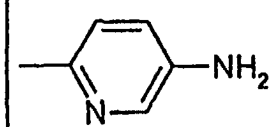 | HCl | 166 | +52 |

Tableau 3

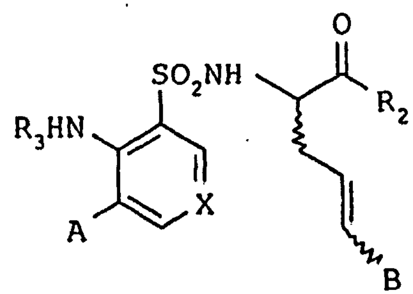

$(I_3)$

avec $R_3$ = $-COCH_3$ et X = CH

| N° | configuration de la double liaison | A | $R_2$ | B | Sel | Point de fusion (°C) | $[\alpha]_D^{20}$ (°) |
|---|---|---|---|---|---|---|---|
| 120 | cis | 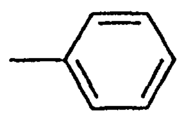 | 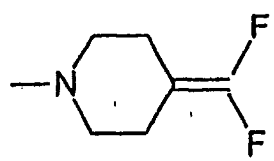 | 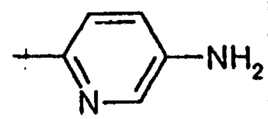 | HCl | 161 | +68 |
| 121 | trans | 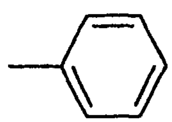 | 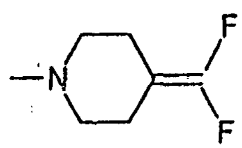 | 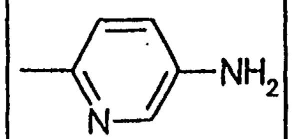 | HCl | 175 | +76 |

| N° | configuration de la double liaison | A | $R_2$ | B | Sel | Point de fusion (°C) | $[\alpha]_D^{20}$ (°) |
|---|---|---|---|---|---|---|---|
| 122 | cis | 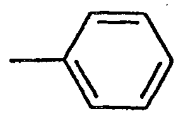 | 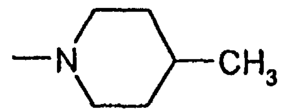 | 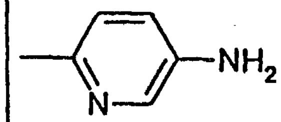 | HCl | 119 | +134 |
| 123 | cis | 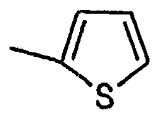 | 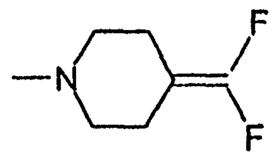 | 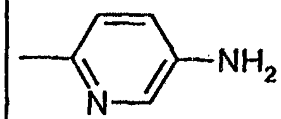 | HCl | 170 | +40 |
| 124 | trans | 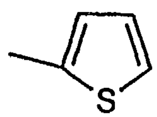 | 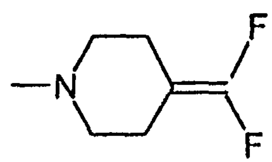 | 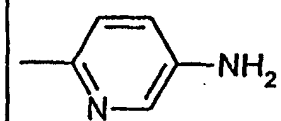 | HCl | 173.5 | +87 |
| 125 | trans | 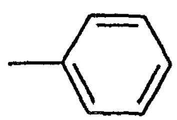 | 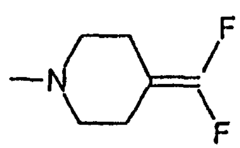 | 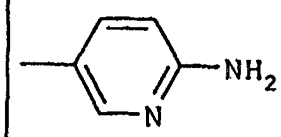 | - | - | - |

Tableau 4

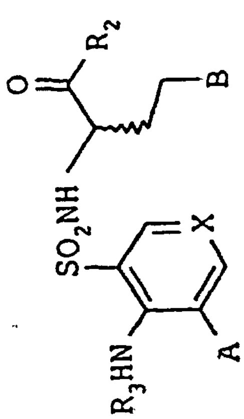

$(I_4)$

| N° | $R_3$ | X | A | $R_2$ | B | Sel | Point de fusion (°C) | $[\alpha]_D^{20}$ (°) |
|---|---|---|---|---|---|---|---|---|
| 126 | $-COCH_3$ | CH | 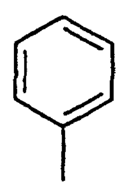 | 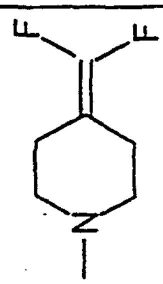 | 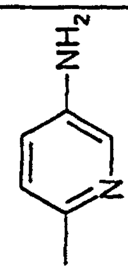 | HCl | 184-6 | +118 (c = 0,23) |

Les composés de l'invention ont fait l'objet d'études pharmacologiques qui ont mis en évidence leurs propriétés antithrombotiques et anticoagulantes, et leur intérêt comme substances à activité thérapeutique.

1. <u>Détermination des constantes d'inhibition (Ki) vis à vis de la thrombine *(in vitro)*.</u>

Sur une microplaque de 96 puits, on dépose dans chaque puits 25 µl d'une solution de composé à tester (on étudie 7 concentrations), 50 µl d'une solution de substrat chromogène (on étudie-2 concentrations ; S2238 Chromogénix™) en solution dans du tampon Tris à pH 7,5 (Tris 50 mM, NaCl 100 mM et BSA 0,1 %) et finalement 25 µl d'une solution de thrombine à 0,24 U/ml. On suit la libération de 4-nitroaniline à 405 nm à l'aide d'un lecteur de plaques.
On détermine le $K_i$ par la méthode de Dixon.
Les composés de l'invention inhibent la thrombine humaine et leur $K_i$ est compris entre 0.001 et 100µM.

2. <u>Activité anticoagulante en plasma de rat (*ex-vivo*).</u>

On traite des rats mâles CD pesant 200 à 250 g avec le composé à tester ou avec son véhicule par voie orale. Ensuite on anesthésie les animaux au Nembutal™ (60 mg/kg ; 0,1 ml/kg), on prélève le sang sur du citrate trisodique à 3,8% (1 vol/9 vol de sang) au niveau du sinus rétro-orbital et on prépare le plasma par centrifugation à 3000 g pendant 15 minutes à la température ambiante. On incube alors à 37 °C, 200 µl de plasma avec 200 µl d'une solution de thrombine, la concentration finale en thrombine étant de 0,75 unités NIH/ml et on note le temps de coagulation.
L'effet anticoagulant est exprimé en pourcentage d'augmentation du temps de thrombine sur les plasmas prélevés 30 et 90 minutes après administration de 20 mg/kg p.o., il est compris entre 100 et 2000%.

3. <u>Activité antithrombotique chez le rat dans un modèle de thrombose mixte artério-veineuse *(in vivo)*.</u>

La formation d'un thrombus chez le rat est obtenue par la mise en place d'un shunt entre la veine jugulaire gauche et l'artère carotide droite ; shunt dans lequel est inséré un fil de coton imprégné de thromboplastine (Facteur Tissulaire ou FT). Le composé est administré par voie orale à plusieurs doses 30 ou 60 minutes avant le montage du shunt. Cinq minutes après le montage du shunt, le thrombus formé au contact du fil + FT est prélevé et rapidement pesé. L'activité antithrombotique est évaluée par réduction du poids frais de thrombus (mg) chez les animaux traités avec le composé, comparativement aux animaux témoins traités avec son véhicule.
L'activité antithrombotique est exprimée par une $DA_{50}$, dose diminuant de 50 % le poids du thrombus frais-dépendant. Celle-ci est comprise entre 0,1 et 50 mg/kg.

4. <u>Perméabilité membranaire *in vitro*.</u>

Les composés de l'invention sont évalués dans un modèle de perméabilité membranaire sur une lignée cellulaire Caco-2 provenant d'un adénocarcinome humain. Cette lignée cellulaire constitue un modèle de choix pour l'étude d'absorption des xénobiotiques [P. Artusson, Thérapeutics Drug Carrier System (1991), 8(4), pp 305-330]. Le passage des composés de l'invention est exprimé en fonction de la quantité de produit ayant franchi la barrière cellulaire pendant 2 h. Les valeurs sont comprises entre 0 et 50 %.
Les composés de l'invention peuvent être utiles dans toutes les indications cliniques liées à la thrombose ou dans celles où des complications thrombotiques pourraient intervenir.
A cet effet ils peuvent être présentés sous toutes formes appropriées à l'administration orale, parentérale ou intraveineuse, telles que comprimés, dragées, gélules, capsules, suspensions ou solutions buvables ou injectables, etc. en association avec des excipients convenables. Toutes ces formes sont dosées pour permettre une administration de 1 à 1000 mg par jour et par patient, en une ou plusieurs doses.

## Revendications

**1.** Composés de formule [I]:

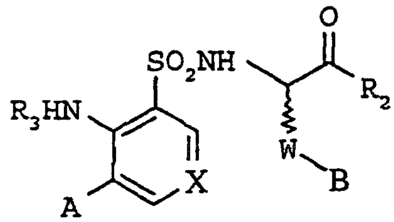

(I)

dans laquelle :

**X** représente soit un groupe $=CR_4-$, soit un atome d'azote,

**W** représente un groupe $-(CH_2)_2-$, $-(CH_2)_3-$, $-CH_2-C\equiv C-$ (liaison triple) ou $-CH_2-CH=CH-$ (liaison double en configuration cis ou trans),

$R_2$ représente

- soit un groupe pipéridinyle éventuellement substitué
  - par un ou deux groupes choisis parmi les groupes hydroxy, $(C_1-C_4)$ alkyle, hydroxy $(C_1-C_4)$ alkyle, $(C_1-C_4)$alcoxy$(C_1-C_4)$alkyle, $(C_1-C_4)$alcoxy, $(C_1-C_4)$alkylthio, monofluorométhyle, difluorométhyle, trifluorométhyle, $(C_3-C_6)$cycloalkyle,
  - par un groupe =CYZ [**Y** et **Z** étant choisis indépendamment l'un de l'autre parmi les atomes d'hydrogène, d'halogène et les groupes $(C_1-C_4)$ alkyle (éventuellement substitués par 1 à 3 atomes d'halogène)]
  - par un groupe

    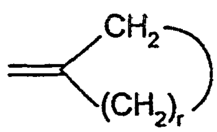

    (r = 1 à 3), ou
  - par un groupe spiro[$(C_3-C_6)$cycloalkane],
- soit un groupe 1,2,3,6-tétrahydropyridinyle éventuellement substitué par un groupe $(C_1-C_4)$alkyle (ce groupe $(C_1-C_4)$alkyle étant éventuellement substitué par 1 à 3 atomes d'halogène) ou un groupe $(C_3-C_6)$cycloalkyle,
- soit un groupe hexahydro-1*H*-azépinyle éventuellement substitué en position 4 par un groupe trifluorométhyle ou difluorométhylène,
- soit un groupe heptahydroazocin-1-yle,
- soit un groupe octahydro-1*H*-azonin-1-yle,
- soit un groupe

  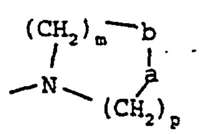

  (a-b étant un groupe -CONR'-, m = 1 à 2, p = 1 à 2 et R' est un atome d'hydrogène ou un groupe $(C_1-C_4)$ alkyle),
- soit un groupe

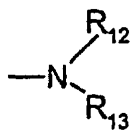

où
soit $\mathbf{R_{12}}$ est un groupe ($C_1$-$C_4$) alkyle, un groupe carboxy ($C_1$-$C_4$) alkyle ou un groupe ($C_1$-$C_4$) alcoxycarbonyl ($C_1$-$C_4$) alkyle et $R_{13}$ est un groupe ($C_1$-$C_4$) alcoxy ou ($C_1$-$C_4$) alkyle,
soit $\mathbf{R_{12}}$ est un groupe ($C_1$-$C_4$) alkyle ou $-CH_2CF_3$ et $\mathbf{R_{13}}$ est un groupe

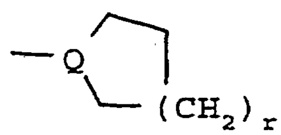

(Q étant un atome de carbone ou d'azote et r = 1 à 3),

- soit un groupe pipérazinyle éventuellement substitué par un groupe ($C_1$-$C_4$)alkyle ou un groupe ($C_1$-$C_4$) alkylsulfonyle,
- soit un groupe morpholinyle,

$R_4$ représente

- soit un atome d'halogène,
- soit un atome d'hydrogène,

$R_3$ représente

- soit un groupe ($C_1$-$C_5$)alkyle,
- soit un groupe $-COR_1$, où $\mathbf{R_1}$ représente soit un atome d'hydrogène, soit un groupe ($C_1$-$C_4$)alkyle, $-(CH_2)_nOCH_3$, $-CH_2O(C_2H_4O)_nCH_3$, $-(CH_2)_nCF_3$ ou $-(CH_2)_nOH$ (n = 1 à 4),
- soit un groupe $-SO_2R_5$,
- soit un groupe $-CONHR_5$,
- soit un groupe $-SO_2N(R_5)_2$, où $R_5$ est un groupe ($C_1$-$C_4$) alkyle,

A représente

- soit un groupe phényle éventuellement substitué par 1 à 3 substituants choisis parmi
  - un atome d'halogène et
  - les groupes ($C_1$-$C_4$)alkyle, ($C_1$-$C_4$)alcoxy, trifluorométhyle, trifluorométhoxy, $-CH_2OR_{10}$, $-CH_2OCOR_{10}$, $-CH_2OCONR_{10}R_{11}$, $-COOR_{10}$, $-CONR_{10}R_{11}$, nitro, $-NR_{10}R_{11}$, $-NHCOR_{10}$, et $-NH(CH_2)_qOR_{10}$, où $R_{10}$ et $R_{11}$ sont chacun indépendamment l'un de l'autre un atome d'hydrogène ou un groupe ($C_1$-$C_4$)alkyle et q est compris entre 0 et 6,
- soit un hétérocycle choisi parmi les groupes pyridyle, thiényle, furyle, pyrimidinyle et thiazolyle, les dits groupes pouvant être substitués comme le groupe phényle ci-dessus,
- soit un groupe ($C_5$-$C_8$) cycloalkyle et

B représente

- soit un groupe pyridyle éventuellement substitué par 1 ou 2 substituants choisis parmi un groupe ($C_1$-$C_4$) alkyle, hydroxy ou ($C_1$-$C_4$) alcoxy,
- soit un groupe aminopyrazinyle,
- soit un groupe aminopyridazinyle,
- soit un groupe pyrimidinyle éventuellement substitué par un groupe amino,

- soit un groupe pipéridinyle,
- soit un groupe aminopyridinyle éventuellement substitué sur la pyridine par un groupe $(C_1-C_4)$alkyle, $(C_1-C_4)$alcoxy ou un atome d'halogène, le groupe amino pouvant éventuellement aussi être substitué par un groupe $(C_1-C_4)$ alkyle,
- soit un groupe aminophényle, le groupe amino pouvant être éventuellement substitué par un groupe $(C_1-C_4)$alkyle et le groupe phényle pouvant être substitué par un groupe $(C_1-C_4)$ alkyle ou un atome d'halogène,

sous forme de racémates ou d'énantiomères purs ou de mélanges d'énantiomères, ou encore sous forme d'acides ou de bases libres ou de sels d'addition à des acides pharmaceutiquement acceptables.

**2.** Composés selon la revendication 1, **caractérisés en ce que** :

**X, W, $R_4$, A** et **B** sont tels que défini dans la revendication 1,

**$R_2$** représente

- soit un groupe pipéridinyle éventuellement substitué
  - par un ou deux groupes choisis parmi les groupes hydroxy, $(C_1-C_4)$ alkyle, hydroxy $(C_1-C_4)$ alkyle, $(C_1-C_4)$alcoxy$(C_1-C_4)$ alkyle, $(C_1-C_4)$ alcoxy, $(C_1-C_4)$ alkylthio, monofluorométhyle, difluorométhyle, trifluorométhyle, $(C_3-C_6)$ cycloalkyle,
  - par un groupe =CYZ [Y et Z étant choisis indépendamment l'un de l'autre parmi les atomes d'hydrogène, d'halogène et les groupes $(C_1-C_4)$alkyle (éventuellement substitués par 1 à 3 atomes d'halogène)],
- soit un groupe 1,2,3,6-tétrahydropyridinyle éventuellement substitué par un groupe $(C_1-C_4)$ alkyle (ce groupe $(C_1-C_4)$alkyle étant éventuellement substitué par 1 à 3 atomes d'halogène) ou un groupe $(C_3-C_6)$ cycloalkyle,
- soit un groupe hexahydro-1*H*-azépinyle éventuellement substitué en position 4 par un groupe trifluorométhyle ou difluorométhylène,
- soit un groupe

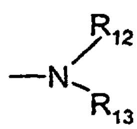

  où **$R_{12}$** est un groupe $(C_1-C_4)$ alkyle, un groupe carboxy $(C_1-C_4)$ alkyle ou un groupe $(C_1-C_4)$ alcaxycarbonyl $(C_1-C_4)$ alkyle et **$R_{13}$** est un groupe $(C_1-C_4)$ alcoxy ou $(C_1-C_4)$ alkyle,
- soit un groupe pipérazinyle éventuellement substitué par un groupe $(C_1-C_4)$alkyle ou un groupe $(C_1-C_4)$ alkylsulfonyle,
- soit un groupe morpholinyle,

**$R_3$** représente

- soit un groupe $(C_1-C_5)$alkyle,
- soit un groupe $-COR_1$, où $R_1$ représente soit un atome d'hydrogène, soit un groupe $(C_1-C_4)$alkyle, $-(CH_2)_nOCH_3$, $-CH_2O(C_2H_4O)_nCH_3$, $-(CH_2)_nCF_3$ ou $-(CH_2)_nOH$ (n = 1 à 4),

sous forme de racémates ou d'énantiomères purs ou de mélanges d'énantiomères, ou encore sous forme d'acides ou de bases libres ou de sels d'addition à des acides pharmaceutiquement acceptables.

**3.** Composés selon l'une des revendications 1 ou 2, **caractérisés en ce que** :

**X, R,** et **B** sont tels que défini dans la renvendication 1,

**W** représente un groupe $-(CH_2)_3-$ ou $-CH_2-CH=CH-$ (double liaison en configuration cis ou trans),

**$R_2$** représente

- soit un groupe pipéridinyle éventuellement substitué
  - par un ou deux groupes choisis parmi les groupes hydroxy, $(C_1-C_4)$ alkyle, hydroxy $(C_1-C_4)$ alkyle, $(C_1-C_4)$alcoxy$(C_1-C_4)$alkyle, $(C_1-C_4)$ alcoxy, $(C_1-C_4)$alkylthio, monofluorométhyle, difluorométhyle et trifluorométhyle,
  - par un groupe =CYZ [Y et Z étant choisis indépendamment l'un de l'autre parmi les atomes d'hydrogène, d'halogène et les groupes $(C_1-C_4)$ alkyle (éventuellement substitués par 1 à 3 atomes d'halogène)]
- soit un groupe 1,2,3,6-tétrahydropyridinyle éventuellement substitué par un groupe $(C_1-C_4)$alkyle (ce groupe $(C_1-C_4)$alkyle étant éventuellement substitué par un à 3 atomes d'halogène),
- soit un groupe hexahydro-1*H*-azépinyle,
- soit un groupe pipérazinyle éventuellement substitué par un groupe $(C_1-C_4)$alkylsulfonyle,
- soit un groupe morpholinyle,

**$R_3$** représente un groupe $-COR_1$, où **$R_1$** est un groupe $(C_1-C_4)$alkyle, $-(CH_2)_nOCH_3$ ou $-(CH_2)_nCF_3$ (n = 1 à 4),

A représente

- soit un groupe phényle éventuellement substitué par 1 à 3 substituants choisis parmi
  - un atome d'halogène et
  - les groupes $(C_1-C_4)$ alkyle et $(C_1-C_4)$ alcoxy,
- soit un hétérocycle choisi parmi les groupes pyridyle ou thiényle,
- soit un groupe $(C_5-C_8)$cycloalkyle,

sous forme de racémates ou d'énantiomères purs ou de mélanges d'énantiomères, ou encore sous forme d'acides ou de bases libres ou de sels d'addition à des acides pharmaceutiquement acceptables.

**4.** Composés selon l'une quelconque des revendications 1 à 3, **caractérisés en ce qu'**ils sont choisis parmi:

- le *N*-[2-[[[(1*S*)-4-(5-amino-3-méthylpyridin-2-yl)-1-[[4-(difluorométhylène)pipéridin-1-yl]carbonyl]butyl]amino] sulfonyl]-6-thién-2-ylphényl]propanamide,
- le *N*-[2-[[[(1*S*)-4-(6-amino-4-éthylpyridin-3-yl)-1-[[4-(difluorométhylène)pipéridin-1-yl]carbonyl]butyl]amino] sulfonyl]-6-cyclopentylphényl]acétamide,
- le *N*-[3-[[[(1*S*)-4-(5-aminopyridin-2-yl)-1-[[4-(difluorométhylène)pipéridin-1-yl]carbonyl]butyl]amino] sulfonyl]-3'-fluoro[1,1'-biphényl]-2-yl]propanamide,
- le *N*-[3-[[[(1*S*)-4-(5-aminopyridin-2-yl)-1-[[4-(difluorométhylène)pipéridin-1-yl]carbonyl]butyl]amino] sulfonyl]-3'-fluoro[1,1'-biphényl]-2-yl]acétamide,
- le *N*-[2-[[[(1*S*)-4-(5-aminopyridin-2-yl)-1-[(4-éthylpipéridin-1-yl)carbonyl]butyl]amino]sulfonyl]-6-thién-2-ylphényl]propanamide,
- le *N*-[2-[[[(1*S*)-4-(6-aminopyridin-3-yl)-1-pipéridin-1-ylcarbonyl)butyl]amino]sulfonyl]-6-cyclopentylphényl] propanamide,
- le *N*-[2-[[[(1*S*)-4-(5-aminopyridin-2-yl)-1-[[4-(difluorométhylène)pipéridin-1-yl]carbonyl]butyl]amino] sulfonyl]-6-cyclopentylphényl]propanamide,
- le *N*-[3-[[[(1*S*)-4-(5-aminopyridin-2-yl)-1-[[4-(difluorométhylène)pipéridin-1-yl]carbonyl]butyl]amino] sulfonyl] [1,1'-biphényl]-2-yl]acétamide,
- le *N*-[2-[[[(1*S*)-4-(6-aminopyridin-3-yl)-1-[[4-(difluorométhylène)pipéridin-1-yl]carbonyl]butyl]amino] sulfonyl]-6-thién-2-ylphényl]acétamide,
- le *N*-[2-[[[(1*S*)-4-(6-amino-4-méthylpyridin-3-yl)-1-[[4-(difluorométhylène)pipéridin-1-yl]carbonyl]butyl]amino] sulfonyl]-6-thién-2-ylphényl]propanamide,
- le *N*-[2-[[[(1*S*)-4-(6-aminopyridin-3-yl)-1-[[4-(difluorométhylène)pipéridin-1-yl]carbonyl]butyl]amino] sulfonyl]-6-cyclopentylphényl]acétamide,

- le *N*-[2-[[[(1*S*)-4-(6-aminopyridin-3-yl)-1-[[4-(trifluorométhyl)-1,2,3,6-tétrahydropyridin-1-yl]carbonyl] butyl] amino]sulfonyl]-6-thién-2-ylphênyl]propanamide,
- le *N*-[2-[[[(1*S*)-4-(6-aminopyridin-3-yl)-1-[[4-(difluorométhylène)pipéridin-1-yl]carbonyl]butyl]amino] sulfonyl]-6-cyclopentylphényl]propanamide,
- le N-[3-[[[(1S)-4-(5-aminopyridin-2-yl)-1-[[4-(difluorométhylène)pipéridin-1-yl]carbonyl]butyl]amino] sulfonyl] [1,1'-biphényl]-2-yl]propanamide,
- le *N*-[2-[[[(1*S*)-4-(6-amino-4-méthylpyridin-3-yl)-1-[[4-difluorométhylène)pipéridin-1-yl]carbonyl]butyl]amino] sulfonyl]-6-cyclopentylphényl]propanamide,
- le *N*-[3-[[[(1*S*)-4-(6-aminopyridin-3-yl)-1-[[4-(difluorométhylène)pipéridin-1-yl]carbonyl]butyl]amino] sulfonyl]-3'-fluoro[1,1'-biphényl]-2-yl]propanamide,
- le *N*-[2-[[[(1S)-4-(5-aminopyridin-2-yl)-1-[(4-méthylpipéridin-1-yl)carbonyl]butyl]amino]sulfonyl]-6-thién-2-ylphényl]acétamide,
- le *N*-[2-[[[(1*S*)-4-(6-aminopyridin-3-yl)-1-[(4-éthylpipéridin-1-yl)carbonyl]butyl]amino]sulfonyl]-6-thién-2-ylphényl]propanamide,
- le *N*-[2-[[[(1*S*)-4-(6-aminopyridin-3-yl)-1-[(4-méthylpipéridin-1-yl)carbonyl]butyl]amino]sulfonyl]-6-cyclopentyl-phényl]acétamide,
- le *N*-[3-[[[(1*S*)-4-(5-aminopyridin-2-yl)-1-[(4-méthylpipéridin-1-yl)carbonyl]butyl]amino]sulfonyl]-3'-méthyl [1,1'-biphényl]-2-yl]acétamide,
- le *N*-[3-[[[(1*S*)-4-(6-amino-4-méthoxypyridin-3-yl-1-[[4-(difluorométhylène)pipéridin-1-yl]carbonyl]butyl]amino] sulfonyl][1,1'-biphényl]-2-yl]propanamide,
- le *N*-[3-[[[(1*S*)-4-(5-aminopyridin-2-yl)-1-[(4-méthylpipéridin-1-yl)carbonyl]butyl]amino]sulfonyl]-3'-méthyl [1,1'-biphényl]-2-yl]propanamide,
- le *N*-[3-[[[(1*S*)-4-(5-aminopyridin-2-yl)-1-[(4-méthylpipéridin-1-yl)carbonyl]butyl]amino]sulfonyl]-3'-fluoro[1,1'-biphényl]-2-yl]acétamide,
- le *N*-[3-[[[(1*S*)-4-(5-aminopyridin-2-yl)-1-[(4-méthylpipéridin-1-yl)carbonyl]butyl]amino]sulfonyl]-3'-méthoxy [1,1'-biphényl]-2-yl]propanamide,
- le *N*-[(1*S*)-4-(5-amino-2-pyridinyl)-1-[[4-(difluorométhylène)-1-pipéridinyl]carbonyl]butyl]-2-(formylamino)-3'-méthyl-[1,1'-biphényl]-3-sulfonamide,
- le *N*-[3-[[[(1*S*,3*Z*)-4-(5-amino-2-pyridinyl)-1-[[4-(difluorométhylène)-1-pipéridinyl]carbonyl]-3-butènyl]amino] sulfonyl][1,1'-biphényl]-2-yl]-acétamide,
- le *N*-[3-[[[(1*S*,3*Z*)-4-(5-amino-2-pyridinyl)-1-[(4-méthyl-1-pipéridinyl)carbonyl]-3-butènyl]amino]sulfonyl] [1,1'-biphényl]-2-yl]-acétamide,

sous forme de racémates ou d'énantiomères purs ou de mélanges d'énantiomères, ou encore sous forme d'acides ou de bases libres ou de sels d'addition à des acides pharmaceutiquement acceptables.

**5.** Composés selon l'une quelconque des revendications 1 à 4, **caractérisés en ce que** la configuration préférentielle de la partie acide aminé centrale

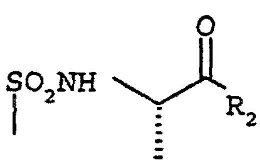

est [*S*].

**6.** Procédé de préparation des composés de formule [I] selon la revendication 1, dans laquelle X représente un groupe =$CR_4$-, où $R_4$ est tel que défini dans la revendication 1, procédé **caractérisé en ce que** l'on fait réagir, dans une étape (i), un composé de formule [V]

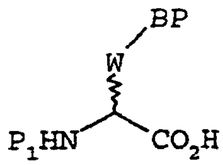

[V]

dans laquelle $P_1$ est un groupe protecteur d'une fonction amine, B et W sont tels que défini dans la revendication 1 et P est soit un groupe protecteur, soit un atome d'hydrogène, avec un composé de formule [VI]

$R_2H$ [VI]

dans laquelle $R_2$ est tel que défini dans la revendication 1, pour obtenir un composé de formule [IV]

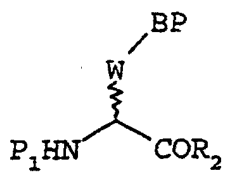

[IV]

qui est ensuite traité au chlorure d'hydrogène dans une étape (ii), pour obtenir un composé de formule [III]

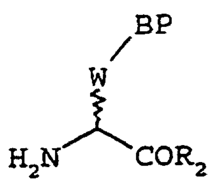 , x HCl

[III]

qui, dans une étape (iii), est condensé avec un composé de formule [II]

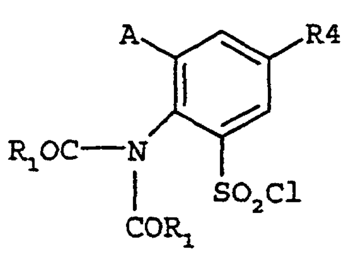

[II]

dans laquelle $R_1$, $R_4$ et A sont tels que défini dans la revendication 1, suivie, dans une étape (iv), d'une hydrogénolyse si l'on désire obtenir un composé de formule [I] où $R_4$ est un atome d'hydrogène.

**7.** Procédé de préparation des composés de formule [I] selon la revendication 1, dans laquelle X représente un groupe $=CR_4$-, où $R_4$ est tel que défini dans la revendication 1, procédé **caractérisé en ce que** l'on fait réagir, dans une étape (i), un composé de formule [III] tel qu'obtenu à l'étape (ii) du procédé selon la revendication 6 et où P représente un atome d'hydrogène

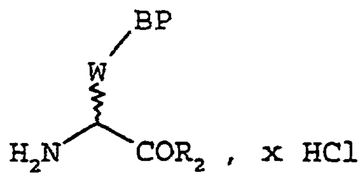

[III]

avec un composé de formule [IIa]

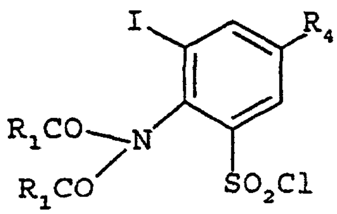

[IIa]

dans laquelle $R_4$ et $R_1$ sont tels que définis dans la revendication 1, pour obtenir un composé de formule [Ia]

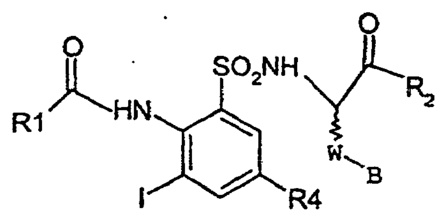

[Ia]

que l'on condense avec un composé de formule [VII]

$$A\text{-}Sn(R_5)_3 \quad [VII]$$

dans laquelle A est tel que défini dans la revendication 1 et $R_5$ est un groupe ($C_1$-$C_4$) alkyle, suivie, dans une étape (iii), d'une hydrogénolyse, si l'on désire obtenir le composé de formule [I] où $R_4$ est un atome d'hydrogène.

**8.** Procédé de préparation des composés de formule [I] selon la revendication 1, dans laquelle X représente un atome d'azote, procédé **caractérisé en ce qu'**il comprend une étape de condensation d'un composé de formule [III], telle que définie dans la revendication 6 ou dans la revendication 7, avec un composé de formule [XV]

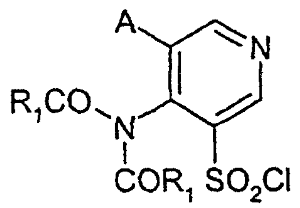

[XV]

9. Composés de formule [III],

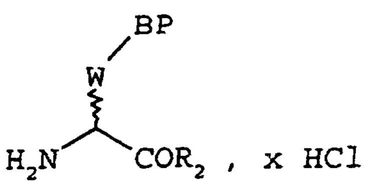

[III]

dans laquelle B et $R_2$ sont tels que définis dans la revendication 1 et P est soit un groupe protecteur de la fonction amine, soit un atome d'hydrogène, utiles comme intermédiaires de synthèse des composés de formule [I] selon la revendication 1.

10. Composés de formule [XV],

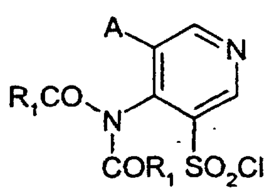

[XV]

dans laquelle A et $R_1$ sont tels que définis dans la revendication 1, utiles comme intermédiaires de synthèse des composés de formule [I] selon la revendication 1.

11. Médicament, **caractérisé en ce qu'**il contient au moins un composé selon l'une quelconque des revendications 1 à 5.

12. Composition pharmaceutique, **caractérisée en ce qu'**elle contient au moins un composé selon l'une quelconque des revendications 1 à 5, ainsi qu'au moins un excipient pharmaceutiquement acceptable.

**Patentansprüche**

1. Verbindungen der Formel [I]:

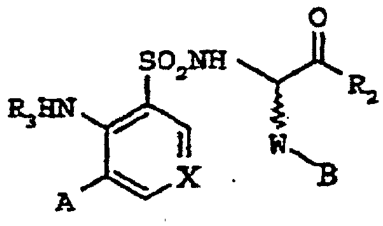

(I)

in der:

**X** entweder eine Gruppe $=CR_4$- oder ein Stickstoffatom bedeutet,
**W** eine Gruppe $-(CH_2)_2-$, $-(CH_2)_3-$, $-CH_2-C{\equiv}C-$ (Dreifachbindung) oder $-CH_2-CH=CH-$ (Doppelbindung in cis- oder trans-Konfiguration), darstellt.
**$R_2$**

- entweder eine Piperidinylgruppe, die gegebenenfalls
  - durch eine oder zwei Gruppen ausgewählt aus Hydroxygruppen, ($C_1$-$C_4$)-Alkylgruppen, Hydroxy-($C_1$-$C_4$)-alkylgruppen, ($C_1$-$C_4$)-Alkoxy-($C_1$-$C_4$)-alkylgruppen, ($C_1$-$C_4$)-Alkoxygruppen, ($C_1$-$C_4$)-Alkylthiogruppen, Monofluormethylgruppen, Difluormethylgruppen, Trifluormethylgruppen oder ($C_3$-$C_6$)-Cycloalkylgruppen,
  - durch eine Gruppe =CYZ [wobei Y und Z unabhängig voneinander aus Wasserstoffatomen, Halogenatomen und (gegebenenfalls durch 1 bis 3 Halogenatome substituierten) ($C_1$-$C_4$)-Alkylgruppen ausgewählt sind],
  - durch eine Gruppe

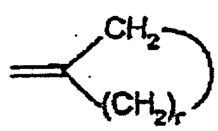

(r = 1 bis 3) oder
  - durch eine Spiro[($C_3$-$C_6$)-cycloalkan]-gruppe substituiert ist,

- oder eine 1,2,3,6-Tetrahydropyridinyl-gruppe, die gegebenenfalls durch eine ($C_1$-$C_4$)-Alkylgruppe (wobei diese ($C_1$-$C_4$)-Alkylgruppe gegebenenfalls durch 1 bis 3 Halogenatome substituiert ist) oder eine ($C_3$-$C_6$)-Cycloalkylgruppe substituiert ist,
- oder eine Hexahydro-1*H*-azepinylgruppe, die gegebenenfalls in der 4-Stellung durch eine Trifluormethylgruppe oder eine Difluormethylengruppe substituiert ist,
- oder eine Heptahydroazocin-1-yl-gruppe,
- oder eine Octahydro-1*H*-azonin-1-yl-gruppe,
- oder eine Gruppe

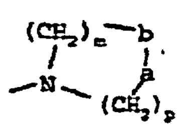

(wobei a-b eine Gruppe -CONR'-, m = 1 bis 2, p = 1 bis 2 und R' ein Wasserstof atom oder eine ($C_1$-$C_4$)-Alkylgruppe bedeuten),
- oder eine Gruppe

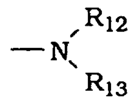

worin
entweder $R_{12}$ eine ($C_1$-$C_4$)-Alkylgruppe, eine Carboxy-($C_1$-$C_4$)-alkylgruppe oder eine ($C_1$-$C_4$)-Alkoxycarbonyl-($C_1$-$C_4$)-alkylgruppe und $R_{13}$ eine ($C_1$-$C_4$)-Alkoxy- oder ($C_1$-$C_4$)-Alkylgruppe bedeuten,
oder $R_{12}$ eine ($C_1$-$C_4$)-Alkylgruppe oder eine Gruppe $-CH_2CF_3$ und $R_{13}$ eine Gruppe

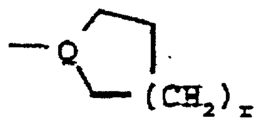

(worin Q ein Kohlenstoff- oder Stickstoffatom darstellt und r = 1 bis 3 bedeutet) bedeuten,

- oder eine Piperazinylgruppe, die gegebenenfalls durch eine ($C_1$-$C_4$)-Alkylgruppe oder eine ($C_1$-$C_4$)-Alkylsulfonylgruppe substituiert ist,
- oder eine Morpholinylgruppe bedeutet,

**$R_4$**

- entweder ein Halogenatom
- oder ein Wasserstoffatom bedeutet,

**$R_3$**

- entweder eine ($C_1$-$C_5$)-Alkylgruppe
- oder eine Gruppe $-COR_1$, worin $R_1$ entweder ein Wasserstoffatom oder eine ($C_1$-$C_4$)-Alkylgruppe oder eine Gruppe $-(CH_2)_nOCH_3$, $-CH_2O(C_2H_4O)_nCH_3$, $-(CH_2)_nCF_3$ oder $-(CH_2)_nOH$ (n = 1 bis 4) darstellt,
- oder eine Gruppe $-SO_2R_5$,
- oder eine Gruppe $-CONHR_5$,
- oder eine Gruppe $-SO_2N(R_5)_2$, worin $R_5$ eine ($C_1$-$C_4$)-Alkylgruppe darstellt, bedeutet,

**A**

- entweder eine Phenylgruppe, die gegebenenfalls substituiert ist durch 1 bis 3 Substituenten ausgewählt aus
  - einem Halogenatom und
  - ($C_1$-$C_4$)-Alkylgruppen, ($C_1$-$C_4$)-Alkoxygruppen, Trifluormethylgruppen, Trifluormethoxygruppen, $-CH_2OR_{10}$, $-CH_2OCOR_{10}$, $-CH_2OCONR_{10}R_{11}$, $-COOR_{10}$, $-CONR_{10}R_{11}$, Nitrogruppen, $-NR_{10}R_{11}$, $-NHCOR_{10}$ und $-NH(CH_2)_qOR_{10}$, worin $R_{10}$ und $R_{11}$ unabhängig voneinander ein Wasserstoffatom oder eine ($C_1$-$C_4$)-Alkylgruppe darstellen und q einen Wert zwischen 0 und 6 aufweist,
- oder einen Heterocyclus ausgewählt aus Pyridyl-, Thienyl-, Furyl-, Pyrimidinyl- und Thiazolyl-gruppen, wobei diese Gruppen wie die oben angegebene Phenylgruppe substituiert sein können,
- oder eine ($C_5$-$C_8$)-Cycloalkylgruppe bedeutet und

**B**

- entweder eine Pyridylgruppe, die gegebenenfalls durch 1 oder 2 Substituenten ausgewählt aus ($C_1$-$C_4$)-Alkylgruppen, der Hydroxygruppe oder ($C_1$-$C_4$)-Alkoxygruppen substituiert ist,
- oder eine Aminopyrazinylgruppe,
- oder eine Aminopyridazinylgruppe,
- oder eine gegebenenfalls durch eine Aminogruppe substituierte Pyrimidinylgruppe,

- oder eine Piperidinylgruppe.
- oder eine Aminopyridinylgruppe, die gegebenenfalls am Pyridin durch eine ($C_1$-$C_4$)-Alkylgruppe, ($C_1$-$C_4$)-Alkoxygruppe oder ein Halogenatom substituiert ist, wobei die Aminogruppe gegebenenfalls auch durch eine ($C_1$-$C_4$)-Alkylgruppe substituiert sein kann,
- oder eine Aminophenylgruppe, wobei die Aminogruppe gegebenenfalls durch eine ($C_1$-$C_4$)-Alkylgruppe substituiert sein kann und die Phenylgruppe durch eine ($C_1$-$C_4$)-Alkylgruppe oder ein Halogenatom substituiert sein kann,

in Form der Racemate oder der reinen Enantiomeren oder von Enantiomerenmischungen oder auch in Form von freien Säuren oder Basen oder von Additionssalzen mit pharmazeutisch annehmbaren Säuren.

2. Verbindungen nach Anspruch 1, **dadurch gekennzeichnet, daß**:

**X, W, $R_4$, A** und **B** die in Anspruch 1 angegebenen Bedeutungen besitzen,
**$R_2$**

- entweder eine Piperidinylgruppe, die gegebenenfalls substituiert ist
  - durch eine oder zwei Gruppen ausgewählt aus Hydroxygruppen, ($C_1$-$C_4$)-Alkylgruppen, Hydroxy-($C_1$-$C_4$)-alkylgruppen, ($C_1$-$C_4$)-Alkoxy-($C_1$-$C_4$)-alkylgruppen, ($C_1$-$C_4$)-Alkoxygruppen, ($C_1$-$C_4$)-Alkylthiogruppen, Monofluormethylgruppen, Difluormethylgruppen, Trifluormethylgruppen und ($C_3$-$C_6$)-Cycloalkylgruppen,
  - durch eine Gruppe =CYZ [worin Y und Z unabhängig voneinander ausgewählt sind aus Wasserstoffatomen, Halogenatomen und (gegebenenfalls durch 1 bis 3 Halogenatome substituierten) ($C_1$-$C_4$)-Alkylgruppen),
- oder eine 1,2,3,6-Tetrahydropyridinyl-gruppe, die gegebenenfalls durch eine ($C_1$-$C_4$)-Alkylgruppe (wobei diese ($C_1$-$C_4$)-Alkylgruppe gegebenenfalls durch 1 bis 3 Halogenatome substituiert ist) oder eine ($C_3$-$C_6$)-Cycloalkylgruppe substituiert ist,
- oder eine Hexahydro-1*H*-azepinylgruppe, die gegebenenfalls in der 4-Stellung durch eine Trifluormethylgruppe oder eine Difluormethylengruppe substituiert ist.
- oder eine Gruppe

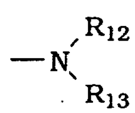

worin $R_{12}$ eine ($C_1$-$C_4$)-Alkylgruppe, eine Carboxy-($C_1$-$C_4$)-alkylgruppe oder eine ($C_1$-$C_4$)-Alkoxycarbonyl-($C_1$-$C_4$)-alkylgruppe und $R_{13}$ eine ($C_1$-$C_4$)-Alkoxy- oder ($C_1$-$C_4$)-Alkylgruppe bedeuten,
- oder eine Piperazinylgruppe, die gegebenenfalls durch eine ($C_1$-$C_4$)-Alkylgruppe oder eine ($C_1$-$C_4$)-Alkylsulfonylgruppe substituiert ist,
- oder eine Morpholinylgruppe bedeutet,

$R_3$

- entweder eine ($C_1$-$C_5$)-Alkylgruppe
- oder eine Gruppe -$COR_1$, worin $R_1$ entweder ein Wasserstoffatom oder eine ($C_1$-$C_4$)-Alkylgruppe oder eine Gruppe $-(CH_2)_nOCH_3$, $-CH_2O(C_2H_4O)_nCH_3$, $-(CH_2)_nCF_3$ oder $-(CH_2)_nOH$ (n = 1 bis 4) darstellt, bedeutet,

in Form der Racemate oder der reinen Enantiomeren oder von Enantiomerenmischungen oder auch in Form der freien Säuren oder Basen oder von Additionssalzen mit pharmazeutisch annehmbaren Säuren.

3. Verbindungen nach einem der Ansprüche 1 oder 2, **dadurch gekennzeichnet, daß**:

**X, $R_4$** und **B** die in Anspruch 1 angegebenen Bedeutungen besitzen,
**W** eine Gruppe $-(CH_2)_3-$ oder $-CH_2-CH=CH-$ (Doppelbindung in der cis- oder trans-Konfiguration) darstellt,

**$R_2$**

- entweder eine Piperidinylgruppe, die gegebenenfalls substituiert ist
  - durch eine oder zwei Gruppen ausgewählt aus Hydroxygruppen, $(C_1-C_4)$-Alkylgruppen, Hydroxy-$(C_1-C_4)$-alkylgruppen, $(C_1-C_4)$-Alkoxy-$(C_1-C_4)$-alkylgruppen, $(C_1-C_4)$-Alkoxygruppen, $(C_1-C_4)$-Alkylthiogruppen, Monofluormethylgruppen, Difluormethylgruppen und Trifluormethylgruppen,
  - durch eine Gruppe =CYZ [worin Y und Z unabhängig voneinander ausgewählt sind aus Wasserstoffatomen, Halogenatomen und (gegebenenfalls durch 1 bis 3 Halogenatome substituierten) $(C_1-C_4)$-Alkylgruppen],
- oder eine 1,2,3,6-Tetrahydropyridinyl-gruppe, die gegebenenfalls durch eine $(C_1-C_4)$-Alkylgruppe (wobei diese $(C_1-C_4)$-Alkylgruppe gegebenenfalls durch 1 bis 3 Halogenatome substituiert ist) substituiert ist,
- oder eine Hexahydro-1*H*-azepinylgruppe,
- oder eine Piperazinylgruppe, die gegebenenfalls durch eine $(C_1-C_4)$-Alkylsulfonylgruppe substituiert ist,
- oder eine Morpholinylgruppe bedeutet,

**$R_3$** eine Gruppe $-COR_1$ darstellt, worin $R_1$ eine $(C_1-C_4)$-Alkylgruppe, oder eine Gruppe $-(CH_2)_nOCH_3$ oder $-(CH_2)_nCF_3$ (n = 1 bis 4) bedeutet,
**A**

- entweder eine Phenylgruppe, die gegebenenfalls durch 1 bis 3 Substituenten ausgewählt aus
  - einem Halogenatom und
  - $(C_1-C_4)$-Alkylgruppen und $(C_1-C_4)$-Alkoxygruppen substituiert ist,
- oder einen Heterocyclus ausgewählt aus Pyridyl- und Thienylgruppen,
- oder eine $(C_5-C_8)$-Cycloalkylgruppe bedeutet,

in Form der Racemate oder der reinen Enantiomeren oder von Enantiomerenmischungen, oder aber auch in Form der freien Säuren oder Basen oder von Additionssalzen mit pharmazeutisch annehmbaren Säuren.

**4.** Verbindungen nach einem der Ansprüche 1 bis 3, **dadurch gekennzeichnet, daß** sie ausgewählt sind aus:

- *N*-[2-[[[(1*S*)-4-(5-Amino-3-methylpyridin-2-yl)-1-[[4-(difluormethylen)-piperidin-1-yl]-carbonyl]-butyl]-amino]-sulfonyl]-6-thien-2-ylphenyl]-propanamid,
- *N*-[2-[[[(1*S*)-4-(6-Amino-4-ethylpyridin-3-yl)-1-[[4-(difluormethylen)-piperidin-1-yl]-carbonyl]-butyl]-amino]-sulfonyl]-6-cyclopentylphenyl]-acetamid,
- *N*-[3-[[[(1*S*)-4-(5-Aminopyridin-2-yl)-1-[[4-(difluormethylen)-piperidin-1-yl]-carbonyl]-butyl]-amino]-sulfonyl]-3'-fluor-[1,1'-biphenyl]-2-yl]-propanamid,
- *N*-[3-[[[(1*S*)-4-(5-Aminopyridin-2-yl)-1-[[4-(difluormethylen)-piperidin-1-yl]-carbonyl]-butyl]-amino]-sulfonyl]-3'-fluor-[1,1'-biphenyl]-2-yl]-acetamid,
- *N*-[2-[[[(1*S*)-4-(5-Aminopyridin-2-yl)-1-[(4-ethylpiperidin-1-yl)-carbonyl]-butyl]-amino]-sulfonyl]-6-thien-2-ylphenyl]-propanamid,
- *N*-[2-[[[(1*S*)-4-(6-Aminopyridin-3-yl)-1-piperidin-1-ylcarbonyl)-butyl]-amino]-sulfonyl]-6-cyclopentylphenyl]-propanamid,
- N-[2-[[[(1*S*)-4-(5-Aminopyridin-2-yl)-1-[[4-(difluormethylen)-piperidin-1-yl]-carbonyl]-butyl]-amino]-sulfonyl]-6-cyclopentylphenyl]-propanamid,
- *N*-[3-[[[(1*S*)-4-(5-Aminopyridin-2-yl)-1-[[4-(difluormethylen)-piperidin-1-yl]-carbonyl]-butyl]-amino]-sulfonyl]-[1,1'-biphenyl]-2-yl]-acetamid,
- *N*-[2-[[[(1*S*)-4-(6-Aminopyridin-3-yl)-1-[[4-(difluormethylen)-piperidin-1-yl]-carbonyl]-butyl]-amino]-sulfonyl]-6-thien-2-ylphenyl]-acetamid,
- *N*-[2-[[[(1*S*)-4-(6-Amino4-methylpyridin-3-yl)-1-[[4-(difluormethylen)-piperidin-1-yl]-carbonyl]-butyl]-amino]-sulfonyl]-6-thien-2-ylphenyl]-propanamid,
- *N*-[2-[[[(1*S*)-4-(6-Aminopyridin-3-yl)-1-[[4-(difluormethylen)-piperidin-1-yl]-carbonyl]-butyl]-amino]-sulfonyl]-6-cyclopentylphenyl]-acetamid,
- *N*-[2-[[[(1*S*)-4-(6-Aminopyridin-3-yl)-1-[[4-(trifluormethyl)-1,2,3,6-tetrahydropyridin-1-yl]-carbonyl]-butyl]-amino]-sulfonyl]-6-thien-2-ylphenyl]-propanamid,

- *N*-[2-[[[(1*S*)-4-(6-Aminopyridin-3-yl)-1-[[4-(difluormethylen)-piperidin-1-yl]-carbonyl]-butyl]-amino]-sulfonyl]-6-cyclopentylphenyl]-propanamid,
- *N*-[3-[[[(1*S*)-4-(5-Aminopyridin-2-yl)-1-[[4-(difluormethylen)-piperidin-1-yl]-carbonyl]-butyl]-amino]-sulfonyl]-[1,1'-biphenyl]-2-yl]-propanamid,
- *N*-[2-[[[(1*S*)-4-(6-Amino-4-methylpyridin-3-yl)-1-[[4-(difluormethylen)-piperidin-1-yl]-carbonyl]-butyl]-amino]-sulfonyl]-6-cyclopentylphenyl]-propanamid,
- *N*-[3-[[[(1*S*)-4-(6-Aminopyridin-3-yl)-1-[[4-(difluormethylen)-piperidin-1-yl]-carbonyl]-butyl]-amino]-sulfonyl]-3'-fluor-[1,1'-biphenyl]-2-yl]-propanamid,
- *N*-[2-[[[(1*S*)-4-(5-Aminopyridin-2-yl)-1-[4-methylpiperidin-1-yl)-carbonyl]-butyl]-amino]-sulfonyl]-6-thien-2-ylphenyl]-acetamid,
- *N*-[2-[[[(1*S*)-4-(6-Aminopyridin-3-yl)-1-[(4-ethylpiperidin-1-yl)-carbonyl]-butyl]-amino]-sulfonyl]-6-thien-2-ylphenyl]-propanamid,
- *N*-[2-[[[(1*S*)-4-(6-Aminopyridin-3-yl)-1-[(4-methylpiperidin-1-yl)-carbonyl]-butyl]-amino]-sulfonyl]-6-cyclopentylphenyl]-acetamid,
- *N*-[3-[[[(1*S*)-4-(5-Aminopyridin-2-yl)-1-[(4-methylpiperidin-1-yl)-carbonyl]-butyl]-amino]-sulfonyl]-3'-methyl-[1,1'-biphenyl-2-yl]-acetamid,
- *N*-[3-[[[(1*S*)-4-(6-Amino-4-methoxypyridin-3-yl)-1-[[4-(difluormethylen)-piperidin-1-yl]-carbonyl]-butyl]-amino]-sulfonyl]-[1,1'-biphenyl]-2-yl]-propanamid,
- *N*-[3-[[[(1*S*)-4-(5-Aminopyridin-2-yl)-1-[(4-methylpiperidin-1-yl)-carbonyl]-butyl]-amino]-sulfonyl]-3'-methyl-[1,1'-biphenyl]-2-yl]-propanamid,
- *N*-[3-[[[(1*S*)-4-(5-Aminopyridin-2-yl)-1-[(4-methylpiperidin-1-yl)-carbonyl]-butyl]-amino]-sulfonyl]-3'-fluor-[1,1'-biphenyl]-2-yl]-acetamid,
- *N*-[3-[[[(1*S*)-4-(5-Aminopyridin-2-yl)-1-[(4-methylpiperidin-1-yl)-carbonyl]-butyl]-amino]-sulfonyl]-3'-methoxy-[1,1'-biphenyl]-2-yl]-propanamid,
- *N*-[(1*S*)-4-(5-Amino-2-pyridinyl)-1-[[4-(difluormethylen)-1-piperidinyl]-carbonyl]-butyl]-2-(formylamino)-3'-methyl-[1,1'-biphenyl]-3-sulfonamid,
- *N*-[3-[[[(1*S*,3*Z*)-4-(5-Amino-2-pyridinyl)-1-[[4-(difluormethylen)-1-piperidinyl]-carbonyl]-3-butenyl]-amino]-sulfonyl]-[1,1'-biphenyl]-2-yl]-acetamid,
- *N*-[3-[[[(1*S*,3*Z*)-4-(5-Amino-2-pyridinyl)-1-[(4-methyl-1--piperidinyl]-carbonyl]-3-butenyl]-amino]-sulfonyl]-[1,1'-biphenyl]-2-yl]-acetamid,

in Form der Racemate oder der reinen Enantiomeren oder von Enantiomerenmischungen oder aber auch in Form der freien Säuren oder Basen oder von Additionssalzen mit pharmazeutisch annehmbaren Säuren.

5. Verbindungen nach einem der Ansprüche 1 bis 4, **dadurch gekennzeichnet, daß** die bevorzugte Konfiguration des zentralen Aminosäurerestes

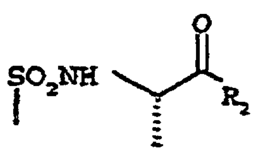

die [*S*]-Konfiguration ist.

6. Verfahren zur Herstellung der Verbindungen der Formel [I] nach Anspruch 1, worin X eine Gruppe $=CR_4-$ bedeutet, worin $R_4$ die in Anspruch 1 dangegebenen Bedeutungen besitzt, welches Verfahren **dadurch gekennzeichnet ist, daß** man in einer Stufe (i) eine Verbindung der Formel [V]

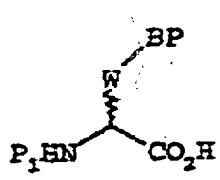

[V]

in der $P_1$ eine Schutzgruppe einer Aminofunktion darstellt, B und W die in Anspruch 1 angegebenen Bedeutungen besitzen und P entweder eine Schutzgruppe oder ein Wasserstoffatom bedeutet, mit einer Verbindung der Formel [VI]:

$$R_2H \qquad [VI]$$

in der $R_2$ die in Anspruch 1 angegebenen Bedeutungen besitzt, umsetzt, zur Bildung einer Verbindung der Formel [IV]

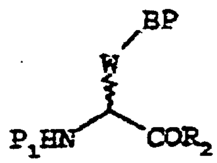

[IV]

welche anschließend in einer Stufe (ii) mit Chlorwasserstoff behandelt wird zur Bildung einer Verbindung der Formel [III]

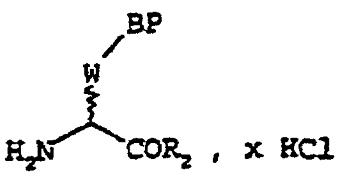

[III]

welche in einer Stufe (iii) mit einer Verbindung der Formel [II]

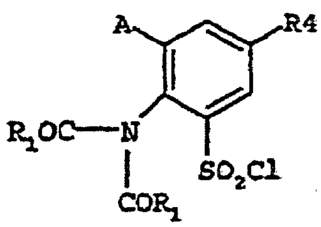

[II]

in der $R_1$, $R_4$ und A die in Anspruch 1 angegebenen Bedeutungen besitzen, kondensiert wird, gefolgt in einer Stufe (iv) von einer Hydrogenolyse, wenn man eine Verbindung der Formel [I], in der $R_4$ ein Wasserstoffatom bedeutet, herstellen will.

**7.** Verfahren zur Herstellung der Verbindungen der Formel [I] nach Anspruch 1, worin X eine Gruppe $=CR_4-$ darstellt, worin $R_4$ die in Anspruch 1 angegebenen Bedeutungen besitzt, welches Verfahren **dadurch gekennzeichnet ist, daß** man in einer Stufe (i) eine Verbindung der Formel [III], wie man sie in der Stufe (ii) des Verfahrens nach Anspruch 6 erhalten hat, worin P ein Wasserstoffatom bedeutet

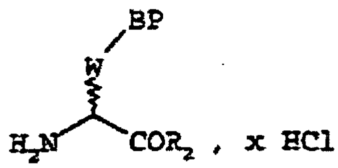

[III]

mit einer Verbindung der Formel [IIa]

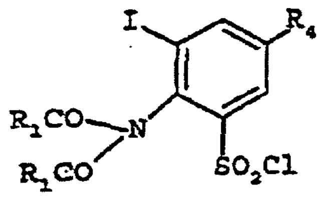

[IIa]

in der $R_4$ und $R_1$ die in Anspruch 1 angegebenen Bedeutungen besitzen, umsetzt zur Bildung einer Verbindung der Formel [Ia]

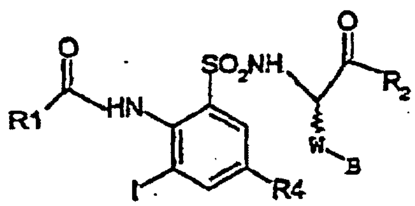

[Ia]

welche man mit einer Verbindung der Formel [VII]

$$A - Sn(R_5)_3 \qquad [VII]$$

in der A die in Anspruch 1 angegebenen Bedeutungen besitzt und $R_5$ eine ($C_1$-$C_4$)-Alkylgruppe darstellt, kondensiert, gefolgt in einer Stufe (iii) von einer Hydrogenolyse, wenn man die Verbindung der Formel [I], worin $R_4$ ein Wassestoffatom bedeutet, herzustellen wünscht.

8. Verfahren zur Herstellung der Verbindungen der Formel [I] nach Anspruch 1, worin X ein Stickstoffatom darstellt, welches Verfahren **dadurch gekennzeichnet ist, daß** es eine Stufe der Kondensation einer Verbindung der Formel [III], wie sie in Anspruch 6 oder in Anspruch 7 definiert worden ist, mit einer Verbindung der Formel [XV]

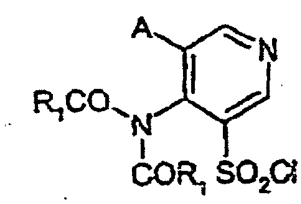

[XV]

umfaßt.

**9.** Verbindungen der Formel [III]

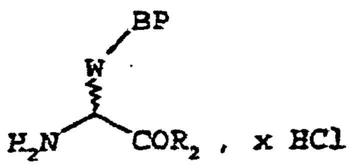

[III]

in der B und $R_2$ die in Anspruch 1 angegebenen Bedeutungen besitzen und P entweder eine Schutzgruppe für die Aminofunktion oder ein Wasserstoffatom bedeutet, als Zwischenprodukte der Synthese der Verbindungen der Formel [I] nach Anspruch 1.

**10.** Verbindungen der Formel [XV]

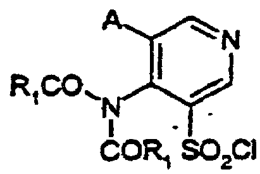

[XV]

in der A und $R_1$ die in Anspruch 1 angegebenen Bedeutungen besitzen, als Zwischenprodukte für die Synthese der Verbindungen der Formel [I] nach Anspruch 1.

**11.** Arzneimittel, **dadurch gekennzeichnet, daß** es mindestens eine Verbindung nach einem der Ansprüche 1 bis 5 enthält.

**12.** Pharmazeutische Zubereitung, **dadurch gekennzeichnet, daß** sie mindestens eine Verbindung nach einem der Ansprüche 1 bis 5 sowie mindestens ein pharmazeutisch annehmbares Trägermaterial enthält.

**Claims**

**1.** Compounds of formula [I]:

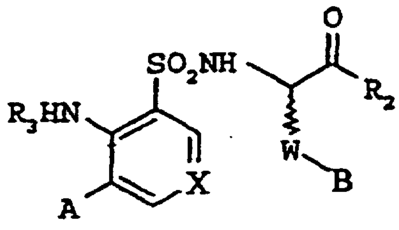

(I)

in which:

X represents either a group $=CR_4-$ or a nitrogen atom,
W represents a $-(CH_2)_2-$, $-(CH_2)_3-$, $-CH_2-C{\equiv}C-$ (triple bond) or $-CH_2-CH{=}CH-$ (double bond in cis or trans configuration) group,
$R_2$ represents

- either a piperidyl group which is optionally substituted:

- with one or two groups chosen from hydroxyl, $(C_1-C_4)$alkyl, hydroxy$(C_1-C_4)$ alkyl, $(C_1-C_4)$ alkoxy-$(C_1-C_4)$alkyl, $(C_1-C_4)$alkoxy, $(C_1-C_4)$alkylthio, monofluoromethyl, difluoromethyl, trifluoromethyl and $(C_3-C_6)$cycloalkyl groups,
- with a group =CYZ [Y and Z being chosen, independently of each other, from hydrogen atoms, halogen atoms and $(C_1-C_4)$alkyl groups (optionally substituted with 1 to 3 halogen atoms)],
- with a group:

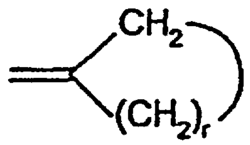

(r = 1 to 3) or
- with a spiro[$(C_3-C_6)$cycloalkane] group,

- or a 1,2,3,6-tetrahydropyridyl group optionally substituted with a $(C_1-C_4)$alkyl group (this $(C_1-C_4)$alkyl group being optionally substituted with 1 to 3 halogen atoms) or a $(C_3-C_6)$ cycloalkyl group,
- or a hexahydro-1H-azepinyl group optionally substituted in position 4 with a trifluoromethyl or difluoromethylene group,
- or a heptahydroazocin-1-yl group,
- or an octahydro-1H-azonin-1-yl group,
- or a group

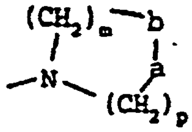

(a-b being a group -CONR'-, m = 1 to 2, p = 1 to 2 and R' is a hydrogen atom or a $(C_1-C_4)$alkyl group),
- or a group

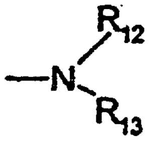

in which
either $R_{12}$ is a $(C_1-C_4)$alkyl group, a carboxy$(C_1-C_4)$alkyl group or a $(C_1-C_4)$alkoxycarbonyl$(C_1-C_4)$alkyl group and $R_{13}$ is a $(C_1-C_4)$alkoxy or $(C_1-C_4)$alkyl group,
or $R_{12}$ is a $(C_1-C_4)$alkyl or $-CH_2CF_3$ group and $R_{13}$ is a group

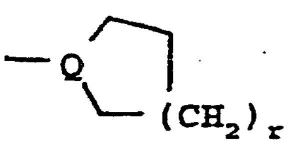

(Q being a carbon or nitrogen atom and r = 1 to 3),
- or a piperazinyl group optionally substituted with a $(C_1-C_4)$alkyl group or a $(C_1-C_4)$alkylsulphonyl group,
- or a morpholinyl group,

$R_4$ represents

- either a halogen atom,
- or a hydrogen atom,

$R_3$ represents

- either a $(C_1-C_5)$alkyl group,
- or a group $-COR_1$, in which $R_1$ is either a hydrogen atom or a group $(C_1-C_4)$alkyl, $-(CH_2)_nOCH_3$, $-CH_2O(C_2H_4O)_nCH_3$, $-(CH_2)_nCF_3$ or $-(CH_2)_nOH$ (n = 1 to 4),
- or a group $-SO_2R_5$,
- or a group $-CONHR_5$,
- or a group $-SO_2N(R_5)_2$, in which $R_5$ is a $(C_1-C_4)$alkyl group,

A represents

- either a phenyl group optionally substituted with 1 to 3 substituents chosen from
  - a halogen atom and
  - groups $(C_1-C_4)$alkyl, $(C_1-C_4)$alkoxy, trifluoromethyl, trifluoromethoxy, $-CH_2OR_{10}$, $-CH_2OCOR_{10}$, $-CH_2OCONR_{10}R_{11}$, $-COOR_{10}$, $-CONR_{10}R_{11}$, nitro, $-NR_{10}R_{11}$, $-NHCOR_{10}$ and $-NH(CH_2)_qOR_{10}$, in which $R_{10}$ and $R_{11}$ are, independently of each other, a hydrogen atom or a $(C_1-C_4)$alkyl group and q is between 0 and 6,
- or a heterocycle chosen from pyridyl, thienyl, furyl, pyrimidinyl and thiazolyl groups, the said groups possibly being substituted like the phenyl group above,
- or a $(C_5-C_8)$cycloalkyl group, and

B represents

- either a pyridyl group optionally substituted with 1 or 2 substituents chosen from a $(C_1-C_4)$alkyl group, a hydroxyl group and a $(C_1-C_4)$alkoxy group,
- or an aminopyrazinyl group,
- or an aminopyridazinyl group,
- or a pyrimidinyl group optionally substituted with an amino group,
- or a piperidyl group,
- or an aminopyridyl group optionally substituted on the pyridine with a $(C_1-C_4)$alkyl or $(C_1-C_4)$alkoxy group or a halogen atom, the amino group possibly also being substituted with a $(C_1-C_4)$alkyl group,
- or an aminophenyl group, the amino group possibly being substituted with a $(C_1-C_4)$alkyl group and the phenyl group possibly being substituted with a $(C_1-C_4)$alkyl group or a halogen atom,

in the form of racemates or pure enantiomers or mixtures of enantiomers, or alternatively in the form of acids or free bases or addition salts with pharmaceutically acceptable acids.

**2.** Compounds according to Claim 1, **characterized in that**:

**X, W, $R_4$, A** and **B** are as defined in Claim 1,
$R_2$ represents

- either a piperidyl group which is optionally substituted:
  - with one or two groups chosen from hydroxyl, $(C_1-C_4)$alkyl, hydroxy$(C_1-C_4)$alkyl, $(C_1-C_4)$alkoxy$(C_1-C_4)$alkyl, $(C_1-C_4)$alkoxy, $(C_1-C_4)$alkylthio, monofluoromethyl, difluoromethyl, trifluoromethyl and $(C_3-C_6)$cycloalkyl groups,
  - with a group =CYZ [Y and Z being chosen, independently of each other, from hydrogen atoms, halogen atoms and $(C_1-C_4)$ alkyl groups (optionally substituted with 1 to 3 halogen atoms)],
- or a 1,2,3,6-tetrahydropyridyl group optionally substituted with a $(C_1-C_4)$alkyl group (this $(C_1-C_4)$alkyl group being optionally substituted with 1 to 3 halogen atoms) or a $(C_3-C_6)$cycloalkyl group,
- or a hexahydro-1H-azepinyl group optionally substituted in position 4 with a trifluoromethyl or difluoromethvlene group,

- or a group

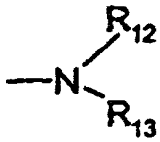

in which

$R_{12}$ is a $(C_1-C_4)$alkyl group, a carboxy$(C_1-C_4)$alkyl group or a $(C_1-C_4)$alkoxycarbonyl$(C_1-C_4)$alkyl group and $R_{13}$ is a $(C_1-C_4)$alkoxy or $(C_1-C_4)$alkyl group,

- or a piperazinyl group optionally substituted with a $(C_1-C_4)$alkyl group or a $(C_1-C_4)$alkylsulphonyl group,
- or a morpholinyl group,

$R_3$ represents

- either a $(C_1-C_5)$ alkyl group,
- or a group $-COR_1$, in which $R_1$ represents either a hydrogen atom or a group $(C_1-C_4)$ alkyl, $-(CH_2)_nOCH_3$, $-CH_2O(C_2H_4O)_nCH_3$, $-(CH_2)_nCF_3$ or $-(CH_2)_nOH$ (n = 1 to 4),

in the form of racemates or pure enantiomers or mixtures of enantiomers, or alternatively in the form of acids or free bases or addition salts with pharmaceutically acceptable acids.

**3.** Compounds according to either of Claims 1 and 2, **characterized in that**:

**X**, $\mathbf{R_4}$ and **B** are as defined in Claim 1,
W represents a $-(CH_2)_3-$ or $-CH_2-CH=CH-$ (double bond in cis or trans configuration) group,
$R_2$ represents

- either a piperidyl group which is optionally substituted:
  - with one or two groups chosen from hydroxyl, $(C_1-C_4)$alkyl, hydroxy$(C_1-C_4)$alkyl, $(C_1-C_4)$alkoxy $(C_1-C_4)$ alkyl, $(C_1-C_4)$alkoxy, $(C_1-C_4)$alkylthio, monofluoromethyl, difluoromethyl and trifluoromethyl groups,
  - with a group =CYZ [Y and Z being chosen, independently of each other, from hydrogen atoms, halogen atoms and $(C_1-C_4)$ alkyl groups (optionally substituted with 1 to 3 halogen atoms)],
- or a 1,2,3,6-tetrahydropyridyl group optionally substituted with a $(C_1-C_4)$alkyl group (this $(C_1-C_4)$alkyl group being optionally substituted with 1 to 3 halogen atoms),
- or a hexahydro-1H-azepinyl group,
- or a piperazinyl group optionally substituted with a $(C_1-C_4)$alkylsulphonyl group,
- or a morpholinyl group,

$R_3$ represents a group $-COR_1$, in which $R_1$ is a group $(C_1-C_4)$alkyl, $-(CH_2)_nOCH_3$ or $-(CH_2)_nCF_3$ (n = 1 to 4),
A represents

- either a phenyl group optionally substituted with 1 to 3 substituents chosen from
  - a halogen atom and
  - $(C_1-C_4)$alkyl and $(C_1-C_4)$alkoxy groups,
- or a heterocycle chosen from pyridyl and thienyl groups,
- or a $(C_5-C_8)$cycloalkyl group,

in the form of racemates or pure enantiomers or mixtures of enantiomers, or alternatively in the form of acids or free bases or addition salts with pharmaceutically acceptable acids.

4. Compounds according to any one of Claims 1 to 3, **characterized in that** they are chosen from:

- N-[2-[[[(1S)-4-(5-amino-3-methylpyrid-2-yl)-1-[[4-(difluoromethylene)piperid-1-yl]carbonyl]butyl]amino]sulphonyl]-6-thien-2-ylphenyl]propanamide,
- N-[2-[[[(1S)-4-(6-amino-4-ethylpyrid-3-yl)-1-[[4-(difluoromethylene)piperid-1-yl]carbonyl]butyl]amino]sulphonyl]-6-cyclopentylphenyl]acetamide,
- N-[3-[[[(1S)-4-(5-aminopyrid-2-yl)-1-[[4-(difluoromethylene)piperid-1-yl]carbonyl]butyl]amino]sulphonyl]-3'-fluoro[1,1'-diphenyl]-2-yl]propanamide,
- N-[3-[[[(1S)-4-(5-aminopyrid-2-yl)-1-[[4-(difluoromethylene)piperid-1-yl]carbonyl]butyl]amino]sulphonyl]-3'-fluoro[1,1'-diphenyl]-2-yl]acetamide,
- N-[2-[[[(1S)-4-(5-aminopyrid-2-yl)-1-[(4-ethylpiperid-1-yl)carbonyl]butyl]amino]sulphonyl]-6-thien-2-ylphenyl]propanamide,
- N-[2-[[[(1S)-4-(6-aminopyrid-3-yl)-1-piperid-1-yl-carbonyl)butyl]amino]sulphonyl]-6-cyclopentylphenyl]propanamide,
- N-[2-[[[(1S)-4-(5-aminopyrid-2-yl)-1-[[4-(difluoromethylene)piperid-1-yl]carbonyl]butyl]amino]sulphonyl]-6-cyclopentylphenyl]propanamide,
- N-[3-[[[(1S)-4-(5-aminopyrid-2-yl)-2-[[4-(difluoromethylene)piperid-1-yl]carbonyl]butyl]amino]sulphonyl][1,1'-diphenyl]-2-yl]acetamide,
- N-[2-[[[(1S)-4-(6-aminopyrid-3-yl)-1-[[4-(difluoromethylene)piperid-1-yl]carbonyl]butyl]amino]sulphonyl]-6-thien-2-ylphenyl]acetamide,
- N-[2-[[[(1S)-4-(6-amino-4-methylpyrid-3-yl)-1-[[4-(difluoromethylene)piperid-1-yl]carbonyl]butyl]amino]sulphonyl]-6-thien-2-ylphenyl]propanamide,
- N-[2-[[[(1S)-4-(6-aminopyrid-3-yl)-1-[[4-(difluoromethylene)piperid-1-yl]carbonyl]butyl]amino]sulphonyl]-6-cyclopentylphenyl]acetamide,
- N-[2-[[[(1S)-4-(aminopyrid-3-yl)-1-[[4-(trifluoromethyl)-1,2,3,6-tetrahydropyrid-1-yl]carbonyl]butyl]amino]sulphonyl]-6-thien-2-ylphenyl]propanamide,
- N-[2-[[[(1S)-4-(6-aminopyrid-3-yl)-1-[[4-(difluoromethylene)piperid-1-yl]carbonyl]butyl]amino]sulphonyl]-6-cyclopentylphenyl]propanamide,
- N-[3-[[[(1*S*)-4-(5-aminopyrid-2-yl)-1-[[4-(difluoromethylene)piperid-1-yl]carbonyl]butyl]amino]sulphonyl][1,1'-diphenyl]-2-yl]propanamide,
- N-[2-[[[(1S)-4-(6-amino-4-methylpyrid-3-yl)-1-[[4-difluoromethylene)piperid-1-yl]carbonyl]butyl]amino]sulphonyl]-6-cyclopentylphenyl]propanamide,
- N-[3-[[[(1S)-4-(6-aminopyrid-3-yl)-1-[[4-(difluoromethylene)piperid-1-yl]carbonyl]butyl]amino]sulphonyl]-3'-fluoro[1,1'-diphenyl]-2-yl]propanamide,
- N-[2-[[[(1S)-4-(5-aminopyrid-2-yl)-1-[(4-methylpiperid-1-yl)carbonyl]butyl]amino]sulphonyl]-6-thien-2-ylphenyl]acetamide,
- N-2-[[[(1S)-4-(6-aminopyrid-3-yl)-1-[(4-ethylpiperid-1-yl)carbonyl]butyl]amino]sulphonyl]-6-thien-2-ylphenyl]propanamide,
- N-[2-[[[(1S)-4-(6-aminopyrid-3-yl)-1-[(4-methylpiperid-1-yl)carbonyl]butyl]amino]sulphonyl]-6-cyclopentylphenyl]acetamide,
- N-[3-[[[(1S)-4-(5-aminopyrid-2-yl)-1-[(4-methylpiperid-1-yl)carbonyl]butyl]amino]sulphonyl]-3'-methyl[1,1'-diphenyl]-2-yl]acetamide,
- N-[3-[[[(1S)-4-(6-amino-4-methoxypyrid-3-yl-1-[[4-(difluoromethylene)piperid-1-yl]carbonyl]butyl]amino]sulphonyl][1,1'-diphenyl]-2-yl]propanamide,
- N-[3-[[[(1S)-4-(5-aminopyrid-2-yl)-1-[(4-methylpiperid-1-yl)carbonyl]butyl]amino]sulphonyl]-3'-methyl[1,1'-diphenyl]-2-yl]propanamide,
- N-[3-[[[(1S)-4-(5-aminopyrid-2-yl)-1-[(4-methylpiperid-1-yl)carbonyl]butyl)amino]sulphonyl]-3'-fluoro[1,1'-diphenyl]-2-yl]acetamide,
- N-[3-[[[(1S)-4-(5-aminopyrid-2-yl)-1-[(4-methylpiperid-1-yl)carbonyl]butyl]amino]sulphonyl]-3'-methoxy[1,1'-diphenyl]-2-yl]propanamide,
- N-[(1S)-4-(5-amino-2-pyridyl)-1-[[4-(difluoromethylene)-1-piperidyl]carbonyl]butyl]-2-(formylamino)-3'-methyl,[1,1'-diphenyl]-3-sulphonamide,
- N-[3-[[[(1S,3Z)-4-(5-amino-2-pyridyl)-1-[[4-(difluoromethylene)-1-piperidyl]carbonyl]-3-butenyl]amino]sulphonyl][1,1'-diphenyl]-2-yl]-acetamide,
- N-[3-[[[(1S,3Z)-4-(5-amino-2-pyridyl)-1-[(4-methyl-1-piperidyl)carbonyl]-3-butenyl]amino]sulphonyl][1,1'-diphenyl]-2-yl]acetamide.

in the form of racemates or pure enantiomers or mixtures of enantiomers, or alternatively in the form of acids

or free bases or addition salts with pharmaceutically acceptable acids.

**5.** Compounds according to any one of Claims 1 to 4, **characterized in that** the preferred configuration of the central amino acid portion

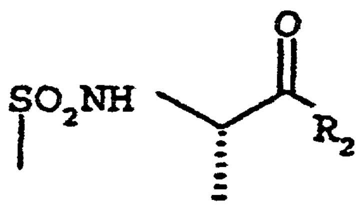

is [S].

**6.** Process for preparing the compounds of formula [I] according to Claim 1, in which X represents a group $=CR_4-$, in which $R_4$ is as defined in Claim 1, this process being **characterized in that** a compound of formula [V]

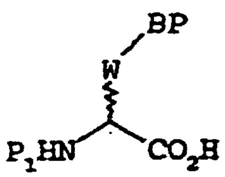

[V]

in which $P_1$ is a protecting group for an amine function, B and W are as defined in Claim 1 and P is either a protecting group or a hydrogen atom, is reacted, in a step (i), with a compound of formula [VI]

$R_2H$ [VI]

in which $R_2$ is as defined in Claim 1, to give a compound of formula [IV]

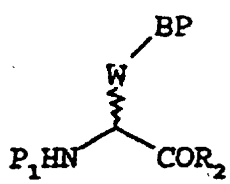

[IV]

which is then treated with hydrogen chloride in a step (ii), to give a compound of formula [III]

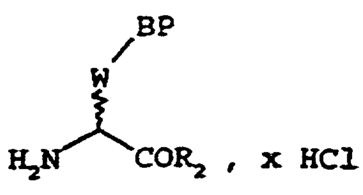

[III]

which, in a step (iii), is condensed with a compound of formula [II]

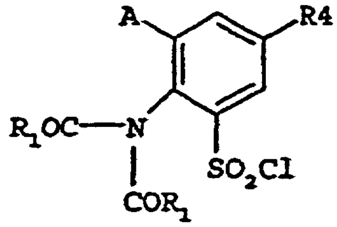

[II]

in which $R_1$, $R_4$ and A are as defined in Claim 1, followed, in a step (iv), by a hydrogenolysis when it is desired to obtain a compound of formula [I] in which $R_4$ is a hydrogen atom.

**7.** Process for preparing compounds of formula [I] according to Claim 1, in which X represents a group $=CR_4-$, in which $R_4$ is as defined in Claim I, this process being **characterized in that** a compound of formula [III] as obtained in step (ii) of the process according to Claim 6 and in which P represents a hydrogen atom

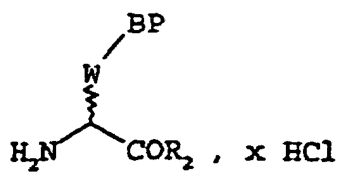

[III]

is reacted, in a step (i), with a compound of formula [IIa]

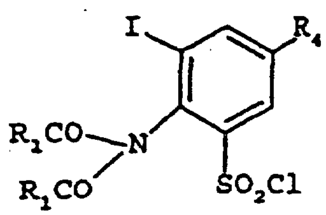

[IIa]

in which $R_4$ and $R_1$ are as defined in Claim 1, to give a compound of formula [Ia]

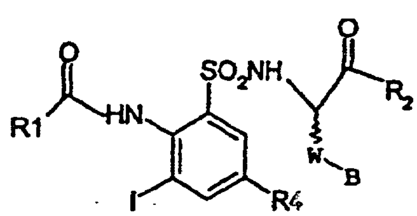

[Ia]

which is coupled with a compound of formula [VII]

$$A\text{-}Sn(R_5)_3 \qquad [VII]$$

in which A is as defined in Claim 1 and $R_5$ is a $(C_1\text{-}C_4)$alkyl group, followed, in a step (iii), by a hydrogenolysis, when it is desired to obtain the compound of formula [I] in which $R_4$ is a hydrogen atom.

**8.** Process for preparing compounds of formula [I] according to Claim 1, in which X represents a nitrogen atom, this process being **characterized in that** it comprises a step of coupling a compound of formula [III], as defined in Claim 6 or in Claim 7, with a compound of formula [XV]

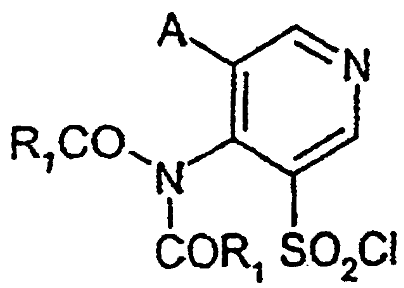

[XV]

**9.** Compounds of formula [III],

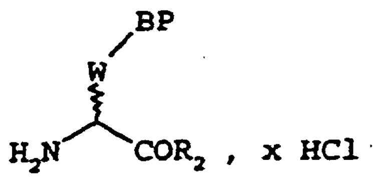

[III]

in which B and $R_2$ are as defined in Claim 1 and P is either a protecting group for the amine function or a hydrogen atom, which are useful as intermediates in the synthesis of the compounds of formula [I] according to Claim 1.

**10.** Compounds of formula [XV],

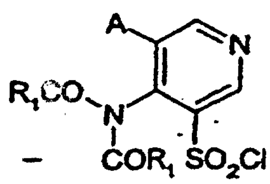

[XV]

in which A and R, are as defined in Claim 1, which are useful as intermediates in the synthesis of the compounds of formula [I] according to Claim 1.

**11.** Medicinal product, **characterized in that** it contains at least one compound according to any one of Claims 1 to 5.

**12.** Pharmaceutical composition, **characterized in that** it contains at least one compound according to any one of Claims 1 to 5, as well as at least one pharmaceutically acceptable excipient.